# EUROPEAN PATENT SPECIFICATION

(11) **EP 2 480 232 B1**
(45) Date of publication and mention of the grant of the patent: **02.10.2013**
(21) Application number: 10747364.7
(22) Date of filing: 18.08.2010
(51) Int. Cl.: A61K 31/454, C07D 401/06, A61P 11/00, A61P 29/00, A61P 19/02, A61P 1/00

(54) **DISUBSTITUTED ]4-(5-AMINOMETHYL-PHENYL)-PIPERIDIN-1-YL]-1H-INDOL-3-YL]-METHANONES**
DISUBSTITUIERTE ]4-(5-AMINOMETHYL-PHENYL)-PIPERIDIN-1-YL]-1H-INDOL-3-YL]-METHANONE
[4-(5-AMINOMÉTHYL-PHÉNYL)-PIPÉRIDIN-1-YL]-1H-INDOL-3-YL]-MÉTHANONES DISUBSTITUÉES

(30) Priority: 20.08.2009 US 235482 P; 15.01.2010 FR 1050258
(43) Date of publication of application: 01.08.2012
(73) Proprietor: SANOFI, 75008 Paris (FR)
(72) Inventor: CHOI-SLEDESKI, Yong-Mi, Bridgewater, New Jersey 08807 (US); GARDNER, Charles, J., Flemington, New Jersey 08822 (US); LIANG, Guyan, Bridgewater, New Jersey 08807 (US); POLI, Gregory, B., Bridgewater, New Jersey 08807 (US); SHUM, Patrick, Wai-Kwok, Bridgewater, New Jersey 08807 (US); STOKLOSA, Gregory, T., Bridgewater, New Jersey 08807 (US); ZHAO, Zhicheng, Bridgewater, New Jersey 08807 (US)
(74) Representative: Tepfenhárt, Dóra Andrea
(86) International application number: PCT/US2010/045828
(87) International publication number: WO 2011/022449

(56) References cited:
- WO-A2-2010/022196
- US-A1- 2003 187 020
- HOPKINS C R ET AL: "Design, synthesis, and biological activity of potent and selective inhibitors of mast cell tryptase" BIOORGANIC & MEDICINAL CHEMISTRY LETTERS, PERGAMON, ELSEVIER SCIENCE, GB LNKD- DOI:10.1016/J.BMCL.2005.04.002, vol. 15, no. 11, 2 June 2005 (2005-06-02), pages 2734-2737, XP025313674 ISSN: 0960-894X [retrieved on 2005-06-02] cited in the application
- PATANI G A ET AL: "BIOISOSTERISM: A RATIONAL APPROACH IN DRUG DESIGN" CHEMICAL REVIEWS, ACS,WASHINGTON, DC, US LNKD- DOI:10.1021/CR950066Q, vol. 96, no. 8, 1 January 1996 (1996-01-01), pages 3147-3176, XP000652176 ISSN: 0009-2665
- LEVELL J ET AL: "Structure based design of 4-(3-aminomethylphenyl)piperidinyl-1-amide s: novel, potent, selective, and orally bioavailable inhibitors of betaII tryptase" BIOORGANIC & MEDICINAL CHEMISTRY, PERGAMON, GB LNKD- DOI:10.1016/J.BMC.2005.02.014, vol. 13, no. 8, 15 April 2005 (2005-04-15) , pages 2859-2872, XP004802824 ISSN: 0968-0896 cited in the application

## Description

### FIELD OF THE INVENTION

This invention is directed to disubstituted [4-(5-aminomethyl-pheny)-piperidin-1-yl]1H-indol-3-yl-]-methanone compounds, their preparation, a pharmaceutical composition comprising these compounds, their use, and intermediates thereof.

### BACKGROUND OF THE INVENTION

Mast cell mediated inflammatory conditions, in particular asthma, are a growing public health concern. Asthma is frequently characterized by progressive development of hyper-responsiveness of the trachea and bronchi to both immunospecific allergens and generalized chemical or physical stimuli, which lead to the onset of chronic inflammation. Leukocytes containing IgE receptors, notably mast cells and basophils, are present in the epithelium and underlying smooth muscle tissues of bronchi. These leukocytes initially become activated by the binding of specific inhaled antigens to the IgE receptors and then release a number of chemical mediators. For example, degranulation of mast cells leads to the release of proteoglycans, peroxidase, arylsulfatase B, chymase, and tryptase, which results in bronchiole constriction.

Tryptase is stored in the mast cell secretory granules and is the major protease of human mast cells. Tryptase has been implicated in a variety of biological processes, including degradation of vasodilatory and bronchodilatory neuropeptides (Caughey, et al., J. Pharmacol. Exp. Ther., 1988, 244, pages 133-137; Franconi, et al., J. Pharmacol. Exp. Ther., 1988, 248, pages 947-951; and Tam, et al., Am. J. Respir. Cell Mol. Biol., 1990, 3, pages 27-32) and modulation of bronchial responsiveness to histamine (Sekizawa, et al., J. Clin. Invest., 1989, 83, pages 175-179).

As a result, tryptase inhibitors may be useful as anti-inflammatory agents (K Rice, P.A. Sprengler, Current Opinion in Drug Discovery and Development, 1999, 2(5), pages 463-474) particularly in the treatment of chronic asthma (M.Q. Zhang, H. Timmerman, Mediators Inflamm., 1997, 112, pages 311-317), and may also be useful in treating or preventing allergic rhinitis (S. J. Wilson et al, Clin. Exp. Allergy, 1998, 28, pages 220-227), inflammatory bowel disease (S.C. Bischoff et al, Histopathology, 1996, 28, pages 1-13), psoriasis (A. Naukkarinen et al, Arch. Dermatol. Res., 1993, 285, pages 341-346), conjunctivitis (A.A.lrani et al, J. Allergy Clin. Immunol., 1990, 86, pages 34-40), atopic dermatitis (A. Jarvikallio et al, Br. J. Dermatol., 1997, 136, pages 871-877), rheumatoid arthritis (L.C Tetlow et al, Ann. Rheum. Dis., 1998, 54, pages 549-555), osteoarthritis (M.G. Buckley et al, J. Pathol., 1998, 186, pages 67-74), gouty arthritis, rheumatoid spondylitis, and diseases of joint cartilage destruction.

In addition, tryptase has been shown to be a potent mitogen for fibroblasts, suggesting its involvement in the pulmonary fibrosis in asthma and interstitial lung diseases (Ruoss et al., J. Clin. Invest., 1991, 88, pages 493-499).

Therefore, tryptase inhibitors may be useful in treating or preventing fibrotic conditions (J.A. Cairns and A.F. Walls, J. Clin. Invest., 1997, 99, pages 1313-1321) for example, fibrosis, sceleroderma, pulmonary fibrosis, liver cirrhosis, myocardial fibrosis, neurofibromas and hypertrophic scars.

Additionally, tryptase inhibitors may be useful in treating or preventing myocardial infarction, stroke, angina and other consequences of atherosclerotic plaque rupture (M. Jeziorska et al, J. Pathol., 1997, 182, pages 115-122).

Tryptase has also been discovered to activate prostromelysin that in turn activates collagenase, thereby initiating the destruction of cartilage and periodontal connective tissue, respectively.

Therefore, tryptase inhibitors could be useful in the treatment or prevention of arthritis, periodontal disease, diabetic retinopathy, and tumour growth (W.J. Beil et al, Exp. Hematol., (1998) 26, pages 158-169). Also, tryptase inhibitors may be useful in the treatment of anaphylaxis (L.B. Schwarz et al, J. Clin. Invest., 1995, 96, pages 2702-2710), multiple sclerosis (M. Steinhoff et al, Nat. Med. (N. Y.), 2000, 6(2), pages 151-158), peptic ulcers and syncytial viral infections.

Substituted arylmethylamines, represented as by a compound of formula (A), their preparation, 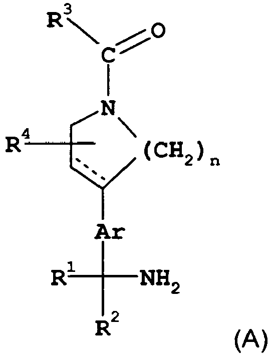 pharmaceutical compositions containing these compounds, and their pharmaceutical use in the treatment of disease states capable of being modulated by the inhibition of tryptase are reported in US Patent 6977263. Specifically disclosed in US Patent 6977263, are compounds of the following formulae 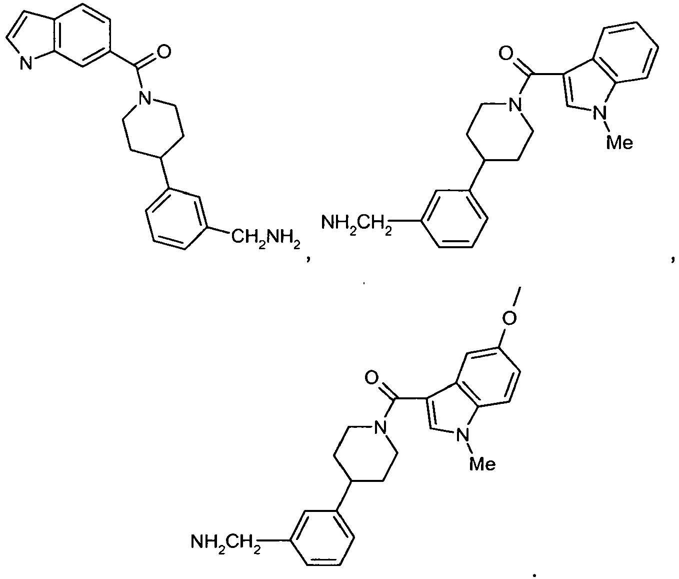 and
US Patent 6977263, however, does not disclose any of the aforesaid [(aminomethyl-phenyl)-piperidin-1-yl]-[indolyl]-methanone species wherein the position para to the aminomethyl group on the phenyl moiety thereof is also substituted with a fluoro group. Furthermore, US Patent 6977263, only discloses one [(aminomethyl-phenyl)-piperidin-1-yl]-[indolyl]-methanone compound wherein an aromatic carbon in the indole moiety thereof, other than the one bonded to the carbonyl, is substituted; more specifically solely wherein the 5-position of the indole is substituted by methoxy.

Bioorg. Med. Chem. Lett. 15, 2734 (2005) discloses three types of [(aminomethyl-phenyl)-piperidin-1-yl]-[1H-indoly-3-yl]-methanones as tryptase inhibitors. One type of the inhibitors is directed to a compound of formula B wherein none of the aromatic carbons in the indole moiety 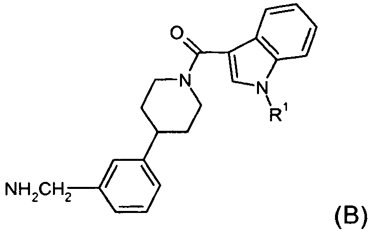 thereof, other than the one bonded to the carbonyl, is substituted, whereas the indole nitrogen is substituted by R¹ as hydrogen, methyl, ethyl, isopropyl, propyl, isobutyl, butyl, hexyl, 2-methoxyethyl, cyclohexylmethyl, cyclopropylmethyl, 3-pyridyl, 2-thiazole, acetyl, thiophene-2-carbonyl, benzenesulfonyl, or methanesulfonyl. The second type of the inhibitors is directed to a compound of formula C wherein the indole nitrogen is substituted only by hydrogen and a single aromatic 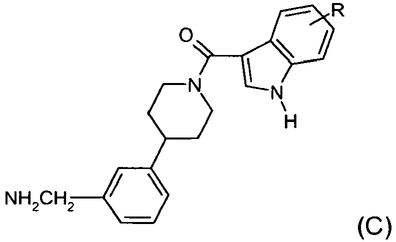 carbon in the indole moiety thereof, other than the one bonded to the carbonyl, is substituted by R as methyl in the 4-, 5-, 6-, or 7-position, or fluoro in the 7-position. The third type of the inhibitors is directed to a compound of formula D wherein a single aromatic carbon in the indole moiety thereof, 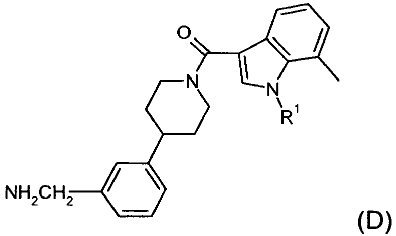 other than the one bonded to the carbonyl, is substituted by methyl in the 7-position, and the indole nitrogen is substituted by R¹ as methyl, ethyl, propyl, butyl, or 2-methoxyethyl. Bioorg. Med. Chem. Lett. 15, 2734 (2005) also discloses that substitution on an aromatic carbon in the indole in the 5- or 7-position were tolerated while substitution in the 4- or 6-position gave less active compounds.
No disclosure exists in US Patent 6977263 or Bioorg. Med. Chem. Lett. 15, 2734 (2005) of an indole containing tryptase inhibitors wherein: (1) the position para to the aminomethyl group on the phenyl moiety thereof is also substituted with a fluoro group; (2) the indole nitrogen is substituted by 2-methoxyethyl; or (3) two or more aromatic carbons in the indole moiety thereof, other than the one bonded to a carbonyl, are substituted, and that has particularly valuable pharmaceutical properties as a tryptase inhibitor. Such a compound should readily have utility in treating a patient suffering from conditions that can be ameliorated by the administration of an inhibitor of tryptase, e.g., mast cell mediated inflammatory conditions, inflammation, and diseases or disorders related to the degradation of vasodilatory and bronchodilatory neuropeptides, and have diminished liability for semicarbazide-sensitive amine oxidase (SSAO) metabolism.

### SUMMARY OF THE INVENTION

The present invention extends to the compound of formula I: 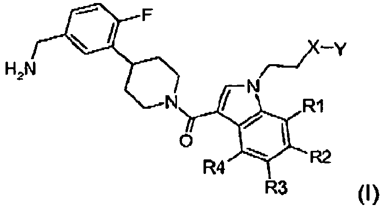 or a prodrug, pharmaceutically acceptable salt, or solvate of said compound.

In a particular embodiment, the present invention includes the compound of formula I wherein:
wherein each of substituents R₁, R₂, R₃ and R₄ is independenently for each instance, selected from the group consisting of hydrogen, methyl, fluoro, chloro, trifluoromethyl, methoxy, and trifluoromethoxy such that exactly two of the subtituents are hydrogen;
X is chosen from the group consisting of a bond, CH₂ and O;
Y is chosen from the group consisting of CH₃ and CF₃;
provided that when R₁ is F, R₂ and R₃ are H, R₄ is trifluoromethoxy, and X is O, Y can not be CH₃;
or a prodrug, Pharmaceutical acceptable salt, or solvate thereof.

Furthermore, the present invention is directed to a pharmaceutical composition comprising a pharmaceutically effective amount of the compound of formula I, and a pharmaceutically acceptable carrier.

Furthermore, the present invention is directed to the use of a compound of formula I as an inhibitor of tryptase, comprising introducing the compound into a composition comprising a tryptase inhibitor receptor. In addition, the present invention is directed to the use of a compound of formula I for treating a patient suffering from, or subject to, a physiological condition in need of amelioration with an inhibitor of tryptase comprising administering to the patient a therapeutically effective amount of the compound of Claim 1

The present invention is directed also to the preparation of a compound of formula I, and intermediates useful therein.

Aspects, features and advantages of the present invention will be better understood from the following detailed description, which is given by way of illustration only, and is not limitative of the present invention.

### DETAILED DESCRIPTION

The present invention relates to novel compounds of the formula I 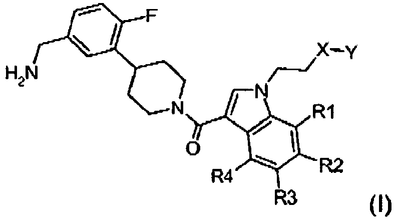 wherein each of substituents R₁, R₂, R₃ and R₄ is independenently for each instance, selected from the group consisting of hydrogen, methyl, fluoro, chloro, trifluoromethyl, methoxy, and trifluoromethoxy such that exactly two of the subtituents are hydrogen;
X is chosen from the group consisting of a bond, CH₂ and O;
Y is chosen from the group consisting of CH₃ and CF₃;
provided that when R₁ is F, R₂ and R₃ are H, R₄ is trifluoromethoxy, and X is O, Y can not be CH₃;
or a pharmaceutically acceptable salt, or solvate thereof.
Specific embodiments of the present invention include the following compounds:
[4-(5-Aminomethyl-2-fluoro-phenyl)-piperidin-1-yl]-[5-chloro-1-(2-methoxy-ethyl)-7-methyl-1 H-indol-3-yl]-methanone;
[4-(5-Aminomethyl-2-fluoro-phenyl)-piperidin-1-yl]-[6-fluoro-1-(2-methoxy-ethyl)-7-methyl-1 H-indol-3-yl]-methanone;
[4-(5-Aminomethyl-2-fluoro-phenyl)-piperidin-1-yl]-[5-fluoro-1-(2-methoxy-ethyl)-7-methyl-1 H-indol-3-yl]-methanone;
[4-(5-Aminomethyl-4-fluoro-phenyl)-piperidin-1-yl]-[5-fluoro-1-(2-methoxy-ethyl)-7-methyl-1 H-indol-3-yl]-methanone;
[4-(5-Aminomethyl-2-fluoro-phenyl)-piperidin-1-yl]-[4-fluoro-1-(2-methoxy-ethyl)-7-trifluoromethoxy-1 H-indol-3-yl]-methanone;
[4-(5-Aminomethyl-2-fluoro-phenyl)-piperidin-1-yl]-[4-chloro-1-(2-methoxy-ethyl)-7-methyl-1 H-indol-3-yl]-methanone;
[4-(5-Aminomethyl-2-fluoro-phenyl)-piperidin-1-yl]-(1-butyl-7-fluoro-4-trifluoromethoxy-1 H-indol-3-yl)-methanone; hydrochloride;
[4-(5-Aminomethyl-2-fluoro-phenyl)-piperidin-1-yl]-[4,7-dichloro-1-(2-methoxy-ethyl)-1 H-indol-3-yl]-methanone hydrochloride;
[4-(5-Aminomethyl-2-fluoro-phenyl)-piperidin-1-yl]-[7-chloro-4-fluoro-1-(2-methoxyethyl)-1H-indol-3-yl]-methanone hydrochloride;
[4-(5-Aminomethyl-2-fluoro-phenyl)-piperidin-1-yl]-[7-chloro-1-(2-methoxy-ethyl)-4-trifluoromethoxy-1 H-indol-3-yl]-methanone hydrochloride;
[4-(5-Aminomethyl-2-fluoro-phenyl)-piperidin-1-yl]-(1-propyl-7-fluoro-4-trifluoromethoxy-1H-indol-3-yl)-methanone; hydrochloride;
[4-(5-Aminomethyl-2-fluoro-phenyl)-piperidin-1-yl]-[4-chloro-1-(2-methoxy-ethyl)-7-trifluoromethoxy-1 H-indol-3-yl]-methanone hydrochloride;
[4-(5-Aminomethyl-2-fluoro-phenyl)-piperidin-1-yl]-[7-fluoro-1-(2-methoxy-ethyl)-4-trifluoromethyl-1 H-indol-3-yl]-methanone hydrochloride;
[4-(5-Aminomethyl-2-fluoro-phenyl)-piperidin-1-yl]-[7-methoxy-1-(2-methoxy-ethyl)-4-trifluoromethyl-1 H-indol-3-yl]-methanone hydrochloride;
[4-(5-Aminomethyl-2-fluoro-phenyl)-piperidin-1-yl]-[7-fluoro-1-(2-methoxy-ethyl)-4-methyl-1 H-indol-3-yl]-methanone hydrochloride;
[4-(5-Aminomethyl-2-fluoro-phenyl)-piperid in-1-yl]-[4,6-dichloro-1-(2-methoxy-ethyl)-1 H-indol-3-yl]-methanone hydrochloride;
[4-(5-Aminomethyl-2-fluoro-phenyl)-piperidin-1-yl]-[4,7-difluoro-1-(2-methoxy-ethyl)-1 H-indol-3-yl]-methanone;
[4-(5-Aminomethyl-2-fluoro-phenyl)-piperidin-1-yl]-[4,6-difluoro-1-(2-methoxy-ethyl)-1 H-indol-3-yl]-methanone hydrochloride;
[4-(5-Aminomethyl-2-fluoro-phenyl)-piperidin-1-yl]-[4,5-difluoro-1-(2-methoxy-ethyl)-1 H-indol-3-yl]-methanone hydrochloride;
[4-(5-Aminomethyl-2-fluoro-phenyl)-piperidin-1-yl]-[5,6-dimethoxy-1-(2-methoxy-ethyl)-1H-indol-3-yl]-methanone hydrochloride;
[4-(5-Aminomethyl-2-fluoro-phenyl)-piperidin-1-yl]-[7-fluoro-4-trifluoromethoxy-1-(2-trifluoromethoxy-ethyl)-1H-indol-3-yl]-methanone;
[4-(5-Aminomethyl-2-fluoro-phenyl)-piperidin-1-yl]-[4-fluoro-7-methyl-1-(2-trifluoromethoxy-ethyl)-1 H-indol-3-yl]-methanone;

### List of Abbreviations

As used above, and throughout the description of the invention, the following abbreviations, unless otherwise indicated, shall be understood to have the following meanings:
- NH₄Cl: ammonium chloride
- *n*-BuOAc: *n*-butyl acetate
- *sec*-BuLi: *sec*-butyl lithium
- *t*-Bu: *tert*-butyl
- *t*-BuOH: *tert*-butanol
- Cul: copper iodide
- DCM: dichloromethane, CH₂Cl₂ or methylene chloride
- DMF: dimethylformamide
- DMSO: dimethylsulfoxide
- EDCI: 1-ethyl-3-(3-dimethylaminopropyl) carbodiimide HCl
- eq: equivalent(s)
- Et: ethyl
- Et₂O: diethyl ether
- EtOH: ethanol
- EtOAc: ethyl acetate
- EtOC(O)Cl: ethyl chloroformate
- HCl: hydrochloric acid
- HPLC: high performance liquid chromatography
- LCMS: liquid chromatography-mass spectrometry
- LC/MS: liquid chromatography-mass spectrometry
- MgSO₄: magnesium sulfate
- Me: methyl
- MeOH: methanol
- MS: mass spectroscopy
- N₂: nitrogen
- NaHCO₃: sodium bicarbonate
- Na₂CO₃: sodium carbonate
- NaH: sodium hydride
- NaOH: sodium hydroxide
- Na₂SO₄: sodium sulfate
- NMR: nuclear magnetic resonance
- PdCl₂dppf: 1,1'-bis(diphenylphosphino) ferrocene palladium (II) dichloride
- Pt/C: platimun on carbon
- K₂CO₃: potassium carbonate
- KOH: potassium hydroxide
- ¹H: proton
- LC: liquid chromatography
- Na₂SO₄: sodium sulfate
- Raney Ni: Raney nickle
- rt: room temperature
- SiO₂: silica
- TFAA: trifluoroacetic anhydride
- THF: tetrahydrofuran
- TLC: thin layer chromatography
- TEA: triethylamine
- TMS-acetylene: trimethylsilyl-acetylene

### Definitions

As used above, and throughout the instant specification and appending claims, the following terms, unless otherwise indicated, shall be understood to have the following meanings:

As used herein, the term "compound of the present invention", and equivalent expressions, are meant to embrace the compound of formula I, as hereinbefore described, which expression includes the prodrug, the pharmaceutically acceptable salt and the solvate, e.g., hydrate. Similarly, reference to intermediates, whether or not they themselves are claimed, is meant to embrace the salts, and solvates, where the context so permits. For the sake of clarity, particular instances when the context so permits are sometimes indicated in the text, but these instances are purely illustrative and they are not intended to exclude other instances when the context so permits.

As used herein, the term "treatment" or "treating" includes prophylactic therapy as well as treatment of an established condition.

"Patient" means a human or other mammal.

"Effective amount" is meant to describe an amount of a compound effective in producing the desired therapeutic effect.

"Prodrug" means a compound that is suitable for administration to a patient without undue toxicity, irritation, allergic response, and the like, and is convertible in vivo by metabolic means (e.g. by hydrolysis) to the compound of the present invention. A thorough discussion of prodrugs is provided in T. Higuchi and V. Stella, Pro-drugs as Novel Delivery Systems, Vol. 14 of the A. C. S. Symposium Series, and in Edward B. Roche, ed., Bioreversible Carriers in Drug Design, American Pharmaceutical Association and Pergamon Press, 1987, both of which are incorporated herein by reference.

"Pharmaceutically acceptable salt" means any salt of these active ingredients with an acid that does not give rise to unwanted toxic or side effects. These acids are well known to pharmacy experts. Non-limiting examples of suitable salts are the following: chloride; bromide; iodide; aspartate, particularly acid aspartate; benzoate, particularly acid benzoate; citrate, particularly acid citrate; tartrate; phosphate, particularly acid phosphate; fumarate, particularly acid fumarate; glycerophosphate; glucose phosphate; lactate; maleate, particularly acid maleate; orotate; oxalate, particularly acid oxalate; sulphate, particularly acid sulphate; trichloroacetate; trifluoroacetate; besylate; tosylate and methanesulphonate. A list of FDA-approved pharmacologically acceptable salts is given in Philip L. Gould, "Salt Selection for Basic Drugs" 33 Int'l J. Pharm. 201, 202, 214-216 (1986); with further information in Stephen M. Berge et al., "pharmaceutical Salts", Journal of Pharmaceutical Sciences Vol. 66, No. 1, January 1977, pages 1-19; and methods for making such salts being known in the art from Handbook of Pharmaceutical Salts, P. Heinrich Stahl, Camille G. Wermuth (Eds.), IUPAC Wiley-VCH , 2002.

### Particular or Preferred Embodiments

In addition, the present invention is directed to the use of the compound of formula I for treating a patient suffering from a physiological condition that can be ameliorated by administering to the patient a therapeutically effective amount of the compound of formula I. Particular embodiments of physiological conditions that can be treated with the compound of the present invention include, but certainly are not limited to inflammatory diseases, e.g., joint inflammation, arthritis, rheumatoid arthritis, rheumatoid spondylitis, gouty arthritis, traumatic arthritis, rubella arthritis, psoriatic arthritis, and other chronic inflammatory joint diseases and asthma and other inflammatory respiratory conditions. Other embodiments of physiological conditions that can be treated by the present invention include physiological conditions such as chronic obstructive pulmonary disease (COPD), COPD exacerbations, joint cartilage destruction, ocular conjunctivitis, vernal conjunctivitis, inflammatory bowel disease, asthma, allergic rhinitis, interstitial lung diseases, fibrosis, sceleroderma, pulmonary fibrosis, liver cirrhosis, myocardial fibrosis, neurofibromas, hypertrophic scars, various dermatological conditions, for example, atopic dermatitis and psoriasis, myocardial infarction, stroke, angina and other consequences of atherosclerotic plaque rupture, as well as periodontal disease, diabetic retinopathy, tumour growth, anaphylaxis, multiple sclerosis, peptic ulcers, and syncytial viral infections.

In a particular embodiment, the present invention is directed to the use of a compound of formula I for treating a patient suffering from asthma and other inflammatory respiratory conditions, comprising administering to the patient a physiologically effective amount of the compound.

In another particular embodiment, the present invention is directed to the use of a compound of formula I for treating a patient suffering from COPD, comprising administering to the patient a physiologically effective amount of the compound.

In another particular embodiment, the present invention is directed to the use of a compound of formula I for treating a patient suffering from COPD exacerbations, comprising administering to the patient a physiologically effective amount of the compound.

In another particular embodiment, the present invention is directed to the use of a compound of formula I for treating a patient suffering from allergic rhinitis, comprising administering to the patient a physiologically effective amount of the compound.

In another particular embodiment, the present invention is directed to the use of a compound of formula I for treating a patient suffering from joint inflammation, comprising administering to the patient a physiologically effective amount of the compound.

In another particular embodiment, the present invention is directed to the use of a compound of formula I for treating a patient suffering from inflammatory bowel disease, comprising administering to the patient a physiologically effective amount of the compound.

In addition, the present invention extends to a pharmaceutical composition comprising the compound of formula I, a second compound selected from the group consisting of a beta adrenergic agonist, an anticholinergic, an anti-inflammatory corticosteroid, and an anti-inflammatory agent, and a pharmaceutically acceptable carrier thereof. In such a composition the compound of formula I and the second compound are present in amounts such that provide a therapeutically efficacious activity, i.e., additive or synergistic effect. Particular inflammatory diseases or disorders that can be treated with such a pharmaceutical composition include, but are not limited to, asthma.

Moreover, the present invention is directed to a method for treating a patient suffering from an inflammatory disorder, comprising administering to the patient the compound of formula I and a second compound selected from the group consisting of a beta adrenergic agonist, an anticholinergic, an anti-inflammatory corticosteroid, and an anti-inflammatory agent. In such a method, the compound of formula I and the second compound are present in amounts such that provide a therapeutically efficacious activity, i.e., additive or synergistic effect. In such a method of the present invention, the compound of the present invention can be administered to the patient before a second compound, a second compound can be administered to the patient before a compound of the present invention, or a compound of the present invention and a second compound can be administered concurrently. Particular examples of adrenergic agonists, anticholinergics, anti-inflammatory corticosteroids, and anti-inflammatory agents having application according to the method are described *infra.*

### Pharmaceutical Compositions

As explained above, the compound of the present invention exhibits useful pharmacological activity and accordingly may be incorporated into a pharmaceutical composition and used in the treatment of patients suffering from certain medical disorders. The present invention thus provides, according to a further aspect, pharmaceutical compositions comprising the compound of the invention, and a pharmaceutically acceptable carrier thereof. As used herein, the term "pharmaceutically acceptable" preferably means approved by a regulatory agency of a government, in particular the Federal government or a state government, or listed in the U.S. Pharmacopoeia or another generally recognized pharmacopoeia for use in animals, and more particularly in humans. Suitable pharmaceutical carriers are described in "Remington's Pharmaceutical Sciences" by E.W. Martin.

Pharmaceutical compositions according to the present invention can be prepared according to the customary methods, using one or more pharmaceutically acceptable adjuvants or excipients. The adjuvants comprise, inter alia, diluents, fillers, binders, disintegrants, glidants, lubricants, surfactants, sterile aqueous media and the various non-toxic organic solvents. The compositions may be presented in the form of tablets, capsules, pills, sustained release formulations, granules, powders, aqueous solutions or suspensions, injectable solutions, elixirs or syrups, and can contain one or more agents chosen from the group comprising sweeteners, flavorings, colorings, or stabilizers in order to obtain pharmaceutically acceptable preparations. The choice of vehicle and the content of active substance in the vehicle are generally determined in accordance with the solubility and chemical properties of the active compound, the particular mode of administration and the provisions to be observed in pharmaceutical practice. For example, excipients such as lactose, microcrystalline cellulose, pregelatinized starch, unmodified starch, silicified microcrystalline cellulose, mannitol, sorbitol, xylitol, dextrates, fructose, sodium citrate, calcium carbonate, dicalcium phosphate dihydrate, anhydrous dicalcium phosphate, calcium sulfate, along with binders such as polyvinylpyrollidone, hydroxypropylmethyl cellulose, ethyl cellulose, hydroxyethyl cellulose, methyl cellulose, sodium carboxymethyl cellulose, pregelatinized starch, starch, polyethylene glycols, polyethylene oxide, polycarbophils, gelatin and acacia and disintegrating agents such as sodium croscarmellose, sodium starch glycolate, crospovidone, starch, microcrystalline cellulose, alginic acids and certain complex silicates combined with lubricants such as magnesium stearate, calcium stearate, stearic acid, hydrogenated vegetable oil, mineral oil, polyethylene glycols, glyceryl esters of fatty acids, sodium lauryl sulfate and glidants such as silicon dioxide, talc, starch, along with some suitable wetting agent such as sodium lauryl sulfate, sorbitan esters, polyoxyethylene fatty acid esters, poloxamer, polyoxyethylene ether, sodium docusate, polyethoxylated castor oil, and benzalkonium chloride may be used for preparing tablets. To prepare a capsule, it is advantageous to use fillers such as lactose, microcrystalline cellulose, pregelatinized starch, unmodified starch, silicified microcrystalline cellulose alone or as a mixture of two or more fillers, with and without binders as described above along with suitable wetting agent (s), disintegrants, glidants, lubricants, etc. as listed above. When aqueous suspensions are used they can contain emulsifying agents or agents which facilitate suspension. Diluents such as sucrose, ethanol, polyethylene glycol, propylene glycol, glycerol and chloroform or mixtures thereof may also be used. Such pharmaceutically acceptable carriers can also be sterile water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include mannitol, human serum albumin (HSA), starch, glucose, lactose, sucrose, gelatin, malt, rice, flour, chalk, silica gel, magnesium carbonate, magnesium stearate, sodium stearate, glycerol monostearate, talc, sodium chloride, dried skim milk, glycerol, propylene, glycol, water, ethanol and the like. These compositions can take the form of solutions, suspensions, tablets, pills, capsules, powders, sustained-release formulations and the like.

Naturally, a pharmaceutical composition of the present invention will contain a therapeutically effective amount of the active compound together with a suitable amount of carrier so as to provide the form for proper administration to the patient. While intravenous injection is a very effective form of administration, other modes can be employed, such as by injection, or by oral, nasal or parenteral administration, which are discussed *infra.*

### Methods of Treatment

The compound of formula I posesses tryptase inhibition activity according to tests described in the literature and described hereinafter, and which test results are believed to correlate to pharmacological activity in humans and other mammals. Thus, in a further embodiment, the present invention is directed to the use of formula I or a composition comprising it for treating a patient suffering from, or subject to, a condition that can be ameliorated by the administration of an inhibitor of tryptase. For example, the compound of formula I is useful for treating an inflammatory disease, for example, joint inflammation, including arthritis, rheumatoid arthritis and other arthritic condition such as rheumatoid spondylitis, gouty arthritis, traumatic arthritis, rubella arthritis, psoriatic arthritis, osteoarthritis or other chronic inflammatory joint disease, or diseases of joint cartilage destruction, ocular conjunctivitis, vernal conjunctivitis, inflammatory bowel disease, asthma, allergic rhinitis, interstitial lung diseases, fibrosis, sceleroderma, pulmonary fibrosis, liver cirrhosis, myocardial fibrosis, neurofibromas, hypertrophic scars, various dermatological conditions, for example, atopic dermatitis and psoriasis, myocardial infarction, stroke, angina or other consequences of atherosclerotic plaque rupture, as well as periodontal disease, diabetic retinopathy, tumour growth, anaphylaxis, multiple sclerosis, peptic ulcers, or a syncytial viral infection.

According to a further feature of the invention there is provided a method for the treatment of a human or animal patient suffering from, or subject to, conditions which can be ameliorated by the administration of an inhibitor of tryptase, for example conditions as hereinbefore described, which comprises the administration to the patient of an effective amount of compound of the invention or a composition containing a compound of the invention.

### Combination Therapy

As explained above, other pharmaceutically active agents can be employed in combination with the compound of formula I depending upon the disease being treated. For example, in the treatment of asthma, beta-adrenergic agonists such as albuterol, terbutaline, formoterol, fenoterol or prenaline can be included, as can anticholinergics such as ipratropium bromide, anti-inflammatory corticosteroids such as beclomethasone dipropionate, triamcinolone acetonide, flunisolide or dexamethasone, and anti-inflammatory agents such as sodium cromoglycate and nedocromil sodium. Thus, the present invention extends to a pharmaceutical composition comprising the compound of formula I and a second compound selected from the group consisting of a beta adrenergic agonist, an anticholinergic, an anti-inflammatory corticosteroid, and an anti-inflammatory agent; and a pharmaceutically acceptable carrier thereof. Particular pharmaceutical carriers having applications in this pharmaceutical composition are described herein.

Furthermore, the present invention extends to a method for treating a patient suffering from asthma, comprising administering the patient the compound of the present invention, and a second compound selected from the group consisting of a beta adrenergic agonist, an anticholinergic, an anti-inflammatory corticosteroid, and an anti-inflammatory agent. In such a combination method, the compound of the present invention can be administered prior to the administration of the second compound, the compound of the present invention can be administered after administration of the second compound, or the compound of the present invention and the second compound can be administered concurrently.

### Modes of Delivery

According to the invention, the compound of formula I, or a pharmaceutical composition comprising the compound, may be introduced parenterally, transmucosally, *e.g*., orally, nasally, pulmonarily, or rectally, or transdermally to a patient.

### Oral Delivery

Contemplated for use herein are oral solid dosage forms, which are described generally in Remington's Pharmaceutical Sciences, 18th Ed.1990 (Mack Publishing Co. Easton PA 18042) at Chapter 89, which is herein incorporated by reference. Solid dosage forms include tablets, capsules, pills, troches or lozenges, cachets or pellets. Also, liposomal or proteinoid encapsulation may be used to formulate the present compositions (as, for example, proteinoid microspheres reported in U.S. Patent No. 4,925,673). Liposomal encapsulation may be used and the liposomes may be derivatized with various polymers (*e.g.*, U.S. Patent No. 5,013,556). A description of possible solid dosage forms for a therapeutic is given by Marshall, K. In: Modem Pharmaceutics Edited by G.S. Banker and C.T. Rhodes Chapter 10, 1979, herein incorporated by reference. In general, the formulation will include a compound of the present invention, and inert ingredients that allow for protection against the stomach environment, and release of the biologically active material, i.e., a compound of the present invention, in the intestine.

Also specifically contemplated are oral dosage forms of the compound of the present invention. Such a compound may be chemically modified so that oral delivery is more efficacious. Generally, the chemical modification contemplated is the attachment of at least one moiety to the component molecule itself, where said moiety permits (a) inhibition of proteolysis; and (b) uptake into the blood stream from the stomach or intestine. Also desired is the increase in overall stability of the compound of the present invention, and increase in circulation time in the body. Examples of such moieties include: polyethylene glycol, copolymers of ethylene glycol and propylene glycol, carboxymethyl cellulose, dextran, polyvinyl alcohol, polyvinyl pyrrolidone and polyproline. Abuchowski and Davis, 1981, "Soluble Polymer-Enzyme Adducts" In: Enzymes as Drugs, Hocenberg and Roberts, eds., Wiley-Interscience, New York, NY, pp. 367-383; Newmark, et al., 1982, J. Appl. Biochem. 4:185-189. Other polymers that could be used are poly-1,3-dioxolane and poly-1,3,6-tioxocane. Preferred for pharmaceutical usage, as indicated above, are polyethylene glycol moieties.

For the compound of the present invention, the location of release may be the stomach, the small intestine (the duodenum, the jejunum, or the ileum), or the large intestine. One skilled in the art has available formulations that will not dissolve in the stomach, yet will release the material in the duodenum or elsewhere in the intestine. Preferably, the release will avoid the deleterious effects of the stomach environment, either by protection of the compound of the present invention, or by release of the compound beyond the stomach environment, such as in the intestine.

To ensure full gastric resistance a coating impermeable to at least pH 5 is essential. Examples of the more common inert ingredients that are used as enteric coatings are cellulose acetate trimellitate (CAT), hydroxypropylmethylcellulose phthalate (HPMCP), HPMCP 50, HPMCP 55, polyvinyl acetate phthalate (PVAP), Eudragit L30D, Aquateric, cellulose acetate phthalate (CAP), Eudragit L, Eudragit S, and shellac. These coatings may be used as mixed films.

A coating or mixture of coatings can also be used on tablets, which are not intended for protection against the stomach. This can include sugar coatings, or coatings that make the tablet easier to swallow. Capsules may consist of a hard shell (such as gelatin) for delivery of dry therapeutic i.e. powder; for liquid forms, a soft gelatin shell may be used. The shell material of cachets could be thick starch or other edible paper. For pills, lozenges, molded tablets or tablet triturates, moist massing techniques can be used.

The therapeutic can be included in the formulation as fine multi-particulates in the form of granules or pellets of particle size about 1 mm. The formulation of the material for capsule administration could also be as a powder, lightly compressed plugs or even as tablets. The therapeutic could be prepared by compression.

Colorants and flavoring agents may all be included. For example, the compound of the present invention may be formulated (such as by liposome or microsphere encapsulation) and then further contained within an edible product, such as a refrigerated beverage containing colorants and flavoring agents.

One may dilute or increase the volume of the therapeutic with an inert material. These diluents could include carbohydrates, especially mannitol, α-lactose, anhydrous lactose, cellulose, sucrose, modified dextrans and starch. Certain inorganic salts may be also be used as fillers including calcium triphosphate, magnesium carbonate and sodium chloride. Some commercially available diluents are Fast-Flo, Emdex, STA-Rx 1500, Emcompress and Avicell.

Disintegrants may be included in the formulation of the therapeutic into a solid dosage form. Materials used as disintegrants include, but are not limited to starch, including the commercial disintegrant based on starch, Explotab. Sodium starch glycolate, Amberlite, sodium carboxymethylcellulose, ultramylopectin, sodium alginate, gelatin, orange peel, acid carboxymethyl cellulose, natural sponge and bentonite may all be used. Another form of the disintegrants are the insoluble cationic exchange resins. Powdered gums may be used as disintegrants and as binders and these can include powdered gums such as agar, Karaya or tragacanth. Alginic acid and its sodium salt are also useful as disintegrants.

Binders may be used to hold the therapeutic agent together to form a hard tablet and include materials from natural products such as acacia, tragacanth, starch and gelatin. Others include methyl cellulose (MC), ethyl cellulose (EC) and carboxymethyl cellulose (CMC). Polyvinyl pyrrolidone (PVP) and hydroxypropylmethyl cellulose (HPMC) could both be used in alcoholic solutions to granulate the therapeutic.

An anti-frictional agent may be included in the formulation of the therapeutic to prevent sticking during the formulation process. Lubricants may be used as a layer between the therapeutic and the die wall, and these can include but are not limited to; stearic acid including its magnesium and calcium salts, polytetrafluoroethylene (PTFE), liquid paraffin, vegetable oils and waxes. Soluble lubricants may also be used such as sodium lauryl sulfate, magnesium lauryl sulfate, polyethylene glycol of various molecular weights, Carbowax 4000 and 6000.

Glidants that might improve the flow properties of the drug during formulation and to aid rearrangement during compression might be added. The glidants may include starch, talc, pyrogenic silica and hydrated silicoaluminate.

To aid dissolution of the therapeutic into the aqueous environment a surfactant might be added as a wetting agent. Surfactants may include anionic detergents such as sodium lauryl sulfate, dioctyl sodium sulfosuccinate and dioctyl sodium sulfonate. Cationic detergents might be used and could include benzalkonium chloride or benzethomium chloride. The list of potential non-ionic detergents that could be included in the formulation as surfactants are lauromacrogol 400, polyoxyl 40 stearate, polyoxyethylene hydrogenated castor oil 10, 50 and 60, glycerol monostearate, polysorbate 40, 60, 65 and 80, sucrose fatty acid ester, methyl cellulose and carboxymethyl cellulose. These surfactants could be present in the formulation of a compound of the present invention either alone or as a mixture in different ratios.

Additives that potentially enhance uptake of the compound of the present invention are, for instance, the fatty acids oleic acid, linoleic acid and linolenic acid. Controlled release oral formulation may be desirable. The drug could be incorporated into an inert matrix that permits release by either diffusion or leaching mechanisms, *e.g.*, gums. Slowly degenerating matrices may also be incorporated into the formulation. Some enteric coatings also have a delayed release effect.
Another form of a controlled release of this therapeutic is by a method based on the Oros therapeutic system (Alza Corp.), i.e. the drug is enclosed in a semipermeable membrane which allows water to enter and push drug out through a single small opening due to osmotic effects.

Other coatings may be used for the formulation. These include a variety of sugars that could be applied in a coating pan. The therapeutic agent could also be given in a film-coated tablet and the materials used in this instance are divided into 2 groups. The first are the non-enteric materials and include methyl cellulose, ethyl cellulose, hydroxyethyl cellulose, methylhydroxyethyl cellulose, hydroxypropyl cellulose, hydroxypropyl-methyl cellulose, sodium carboxy-methyl cellulose, providone and the polyethylene glycols. The second group consists of the enteric materials that are commonly esters of phthalic acid.

A mix of materials might be used to provide the optimum film coating. Film coating may be carried out in a pan-coater or in a fluidized bed or by compression coating.

### Pulmonary Delivery

Also contemplated herein is pulmonary delivery of the compound of the present invention, either alone, or in a pharmaceutical composition. The compound is delivered to the lungs of a mammal while inhaling and traverses across the lung epithelial lining to the blood stream. Other reports of this include Adjei et al., 1990, Pharmaceutical Research, 7:565-569; Adjei et al., 1990, International Journal of Pharmaceutics, 63:135-144 (leuprolide acetate); Braquet et al., 1989, Journal of Cardiovascular Pharmacology, 13(suppl. 5):143-146 (endothelin-1); Hubbard et al., 1989, Annals of Internal Medicine, Vol. III, pp. 206-212 (a1- antitrypsin); Smith et al., 1989, J.Clin. Invest. 84:1145-1146 (a-1-proteinase); Oswein et al., 1990, "Aerosolization of Proteins", Proceedings of Symposium on Respiratory Drug Delivery II, Keystone, Colorado, March, (recombinant human growth hormone); Debs et al., 1988, J. Immunol. 140:3482-3488 (interferon-γ and tumour necrosis factor alpha) and Platz et al., U.S. Patent No. 5,284,656 (granulocyte colony stimulating factor). A method and composition for pulmonary delivery of drugs for systemic effect is described in U.S. Patent No. 5,451,569, issued September 19, 1995 to Wong et al.

Contemplated for use in the practice of this invention are a wide range of mechanical devices designed for pulmonary delivery of therapeutic products, including but not limited to nebulizers, metered dose inhalers, and powder inhalers, all of which are familiar to those skilled in the art.

Some specific examples of commercially available devices suitable for the practice of this invention are the Ultravent nebulizer, manufactured by Mallinckrodt, Inc., St. Louis, Missouri; the Acorn II nebulizer, manufactured by Marquest Medical Products, Englewood, Colorado; the Ventolin metered dose inhaler, manufactured by Glaxo Inc., Research Triangle Park, North Carolina; and the Spinhaler powder inhaler, manufactured by Fisons Corp., Bedford, Massachusetts, to name only a few. All such devices require the use of formulations suitable for the dispensing of the compound of the present invention. Typically, each formulation is specific to the type of device employed and may involve the use of an appropriate propellant material, in addition to the usual diluents, adjuvants and/or carriers useful in therapy. Also, the use of liposomes, microcapsules or microspheres, inclusion complexes, or other types of carriers is contemplated. A chemically modified compound of the present invention may also be prepared in different formulations depending on the type of chemical modification or the type of device employed.

Formulations suitable for use with a nebulizer, either jet or ultrasonic, will typically comprise the compound of the present invention dissolved in water at a concentration of about 0.1 to 25 mg of compound per mL of solution. The formulation may also include a buffer and a simple sugar (*e.g.*, for stabilization and regulation of osmotic pressure). The nebulizer formulation may also contain a surfactant, to reduce or prevent surface induced aggregation of the compound caused by atomization of the solution in forming the aerosol.

Formulations for use with a metered-dose inhaler device will generally comprise a finely divided powder containing the compound of the invention suspended in a propellant with the aid of a surfactant. The propellant may be any conventional material employed for this purpose, such as a chlorofluorocarbon, hydrochlorofluorocarbon, hydrofluorocarbon, or hydrocarbon, including trichlorofluoromethane, dichlorodifluoromethane, dichlorotetrafluoroethanol, and 1,1,1,2-tetrafluoroethane, or combinations thereof. Suitable surfactants include sorbitan trioleate and soya lecithin. Oleic acid may also be useful as a surfactant.

Formulations for dispensing from a powder inhaler device will comprise a finely divided dry powder containing the compound of the invention, and may also include a bulking agent, such as lactose, sorbitol, sucrose, or mannitol in amounts which facilitate dispersal of the powder from the device, *e.g.*, 50 to 90% by weight of the formulation. The compound of the present invention should most advantageously be prepared in particulate form with an average particle size of less than 10 mm (or microns), most preferably 0.5 to 5 mm, for most effective delivery to the distal lung.

### Nasal Delivery

Nasal delivery of the compound of the present invention is also contemplated. Nasal delivery allows the passage of the compound to the blood stream directly after administering the therapeutic product to the nose, without the necessity for deposition of the product in the lung. Formulations for nasal delivery include those with dextran or cyclodextran.

### Transdermal Delivery

Various and numerous methods are known in the art for transdermal administration of a drug, *e.g*., via a transdermal patch, have applications in the present invention. Transdermal patches are described in for example, U.S. Patent Nos. 5,407,713, 5,352,456, 5,332,213, 5,336,168, 5,290,561, 5,254,346, 5,164,189, 5,163,899, 5,088,977, 5,087,240, 5,008,110, and 4,921,475, the disclosure of each of which is incorporated herein by reference in its entirety.

It can be readily appreciated that a transdermal route of administration may be enhanced by use of a dermal penetration enhancer, *e.g*., such as enhancers described in U.S. Patent Nos. 5,164,189, 5,008,110" and ,4,879,119, the disclosure of each of which is incorporated herein by reference in its entirety.

### Topical Administration

For topical administration, gels (water or alcohol based), creams or ointments containing compounds of the invention may be used. Compounds of the invention may also be incorporated in a gel or matrix base for application in a patch, which would allow a controlled release of compound through the transdermal barrier.

### Rectal Administration

Solid compositions for rectal administration include suppositories formulated in accordance with known methods and containing the compound of the invention.

### Dosages

The percentage of active ingredient in the composition of the invention may be varied, it being necessary that it should constitute a proportion such that a suitable dosage shall be obtained. Obviously, several unit dosage forms may be administered at about the same time. The dose employed will be determined by the physician, and depends upon the desired therapeutic effect, the route of administration and the duration of the treatment, and the condition of the patient. In the adult, the doses are generally from about 0.001 to about 50, preferably about 0.001 to about 5, mg/kg body weight per day by inhalation, from about 0.01 to about 100, preferably 0.1 to 70, more especially 0.5 to 10, mg/kg body weight per day by oral administration, and from about 0.001 to about 10, preferably 0.01 to 1, mg/kg body weight per day by intravenous administration. In each particular case, the doses will be determined in accordance with the factors distinctive to the subject to be treated, such as age, weight, general state of health and other characteristics which can influence the efficacy of the medicinal product.

Furthermore, the compound according to the invention may be administered as frequently as necessary in order to obtain the desired therapeutic effect. Some patients may respond rapidly to a higher or lower dose and may find much weaker maintenance doses adequate. For other patients, it may be necessary to have long-term treatments at the rate of 1 to 4 doses per day, in accordance with the physiological requirements of each particular patient. Generally, the active product may be administered orally 1 to 4 times per day. Of course, for some patients, it will be necessary to prescribe not more than one or two doses per day.

Naturally, a patient in whom administration of the compound of the present invention is an effective therapeutic regimen is preferably a human, but can be any animal. Thus, as can be readily appreciated by one of ordinary skill in the art, the methods and pharmaceutical compositions of the present invention are particularly suited to administration to any animal, particularly a mammal, and including, but by no means limited to, domestic animals, such as feline or canine subjects, farm animals, such as but not limited to bovine, equine, caprine, ovine, and porcine subjects, wild animals (whether in the wild or in a zoological garden), research animals, such as mice, rats, rabbits, goats, sheep, pigs, dogs, cats, etc., avian species, such as chickens, turkeys, songbirds, etc., *i.e*., for veterinary medical use.

### Preparatory Details

The compound of formula I may be prepared by the application or adaptation of known methods, by which is meant methods used heretofore or described in the literature, for example those described by R.C.Larock in Comprehensive Organic Transformations, VCH publishers, 1989, or as described herein.

In the reactions described hereinafter it may be necessary to protect reactive functional groups, for example, amino groups, to avoid their unwanted participation in the reactions. Conventional protecting groups may be used in accordance with standard practice, for examples see T.W. Greene and P.G.M.Wuts in "Protective Groups in Organic Chemistry" John Wiley and Sons, 1991.
In particular, the compound of formula I may be prepared as shown through Schemes I-III.

### Examples

The present invention may be better understood by reference to the following non-limiting Examples, which are provided as exemplary of the invention. The following Examples are presented in order to more fully illustrate particular embodiments of the invention. They should in no way be construed, however, as limiting the broad scope of the invention. The Reference Example below is provided to disclose how to make an intermediate used for making the compound of formula I.

In the nuclear magnetic resonance spectra (NMR), reported *infra,* the chemical shifts are expressed in ppm relative to tetramethylsilane. Abbreviations have the following significances: br = broad, dd = double doublet, s = singlet; m = multiplet.

### EXAMPLE 1

### [4-(5-Aminomethyl-2-fluoro-phenyl)-piperidin-1-yl]-[5-chloro-1-(2-methoxy-ethyl)-7-methyl-1H-indol-3-yl]-methanone hydrochloride

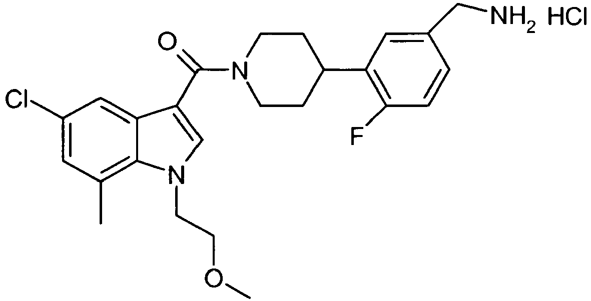

### A. 2,2,2-Trifluoro-N-(4-fluoro-3-pyridin-4-yl-benzyl)-acetamide hydrochloride

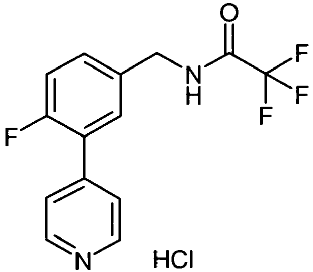

A flask is charged with NaHCO₃ (126 g, 1.5 mol), 3-bromo-4-fluorobenzylamine hydrochloride (120 g, 0.5 mole) and pyridine-4-boronic acid (67.6 g, 0.55 mmol) and isopropyl alcohol (750 mL) and water (375 mL) at room temperature. The suspension is degassed with N₂ for 1.0 h at 10 °C. Into the mixture is added 1,1'-bis(diphenylphosphino)ferrocene-palladium(II)dichloride dichloromethane complex (PdCl₂dppf-CH₂Cl₂, 16.4 g, 20 mmol). The reaction mixture is ramped to 80 °C while some part is distilled off until the internal temperature reached to 80 °C, and stirred for 10 h. After the reaction is completed (HPLC analysis), the mixture is cooled to room temperature, and aqueous 2 N HCl (750 mL) is added, and stirred for 0.5 h. The solution is washed with CH₂Cl₂ (750 mL and 500 mL). To the aqueous phase is charged 50% aqueous NaOH (100 mL) to adjust pH >13. After adding *n*-BuOAc (2.0 L), activated carbon (50 g) is added into the organic layer. This mixture is filtered through a pad of celite (50 g). Azeotropic distillation is performed. After adding an additional *n*-BuOAc (1.0 L), the reaction ics cooled to 5 °C. Trifluoroacetic anhydride (157 g, 0.6 mol) is slowly added into the solution at 5 °C. After the reaction is completed (HPLC analysis), the reaction mixture is washed with aqueous 10% Na₂CO₃(1.0 L). A solution of 5-6 N HCl in isopropanol (120 mL) is introduced into the crude organic layer at 10 °C. Additional *n*-BuOAc (1.0 L) is then added, the suspension is left overnight at room temperature. The resultant solid is filtered at 10 °C, and dried in oven at 50 °C to give 124 g (75%) of desired product as white solid: mp = 220 °C. Anal. Calcd for C₁₄H₁₀F₄N₂O-HCl: C, 50.24; H, 3.31; N, 8.37. Found: C, 50.16; H, 3.08; N, 8.38. MS (ESI) m/z 299 (M+H). ¹H NMR (300 MHz, D₂O) δ 8.70 (d, *J* = 6.9 Hz, 2 H), 8.14 (d, *J* = 6.9 Hz, 2H), 7.56-7.20 (m, 3H), 4.51 (s, 2H).

### B. 2,2,2-trifluoro-N-(4-fluoro-3-piperidin-4-yl-benzyl)-acetamide hydrochloride

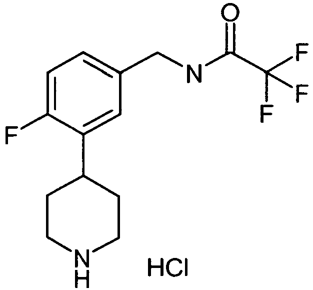

A Parr flask is charged with 2,2,2-trifluoro-*N*-(4-fluoro-3-pyridin-4-yl-benzyl)-acetamide hydrochloride (123 g, 0.37 mol) and MeOH (740 mL) at room temperature. Then, 5% Pt/C (36.9 g, 30 w/w %) is added. The reaction flask is placed in a Parr hydrogenation system and charged with H₂ at 50-60 psi. The mixture is shaken for >48 h while charging H₂ until the pressure reached a steady state (H₂ was refilled to 50-60 psi every 2-3 hours during day time while 10-20 psi is observed without any further refill after overnight). When HPLC analysis shows completion of the reaction, the reaction mixture is filtered through a pad of celite. The filtrate is distilled at 40-50 °C while adding n-BuOAc (1.25 L). After completion of distillation of MeOH, additional n-BuOAc (1 L) is added. The resultant suspension is allowed to cool to rt overnight. The suspension is cooled to 10 °C, filtered, and dried in oven at 50 °C to give 112 g (89%) of desired product as white solid: mp = 134 °C. Anal. Calcd for C₁₄H₁₀F₄N₂O-HCl: C, 50.24; H, 3.31; N, 8.37. Found: C, 50.16; H, 3.08; N, 8.38. MS (ESI) m/z 305.4 (M+H). ¹H NMR (300 MHz, D₂O) δ 7.16-6.98 (m, 3 H), 4.34 (s, 2H), 3.42 (d, *J* = 12.9 Hz, 2H), 3.14-2.99 (m, 3H), 1.98-1.81 (m, 4H).

### C. 5-Chloro-7-methyl-1H-indole

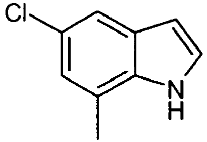

5-Chloro-7-methyl-1*H*-indole-2,3-dione (1.8g, 9.23mmol) is added neat to a 1.0 M solution of lithium aluminum hydride in ether (92mL). The reaction mixture is stirred at room temperature overnight. The reaction mixture is quenched with ice and is diluted with ethyl acetate (600mL). The organic phase is collected, washed with sat NH₄Cl (2 X100mL) and brine (50 mL). The organic is dried over magnesium sulfate, filtered and concentrated *in vacuo* to give the crude product. Purification by flash chromatography on SiO₂ eluting with 5% ethyl acetate/ heptane gives 1.27 g, (83% yield) of desired product. ¹H-NMR (CDCl₃, 300 MHz): δ 8.1 (bs, H), 7.45 (s, 1 H), 7.2 (d, 1 H), 7.0 (s, 1 H), 6.5 (d, H), 2.5 (s, 3H). LCMS *m*/*z*: [M+H]⁺=166

### D. 5-Chloro-1-(2-methoxy-ethyl)-7-methyl-1H-indole

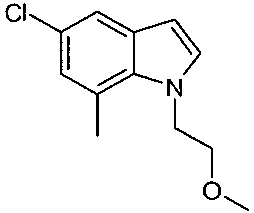

A mixture of powder KOH (2.97 g, 53 mmol) in DMSO (50 mL) is stirred at rt for 15 min. 5-Chloro-7-methyl-1*H*-indole (2.2 g, 13.28 mmol) is added. The reaction mixture is stirred for 1 h and then 2- methoxyethyl bromide (2.49 mL, 26.5 mmol) is added. This mixture is stirred at room temperature overnight. The mixture is partitioned between H₂O and EtOAc. The organic phase is collected, washed with water (3 X 100mL) and brine (50 mL). The organic is dried over Na₂SO₄, filtered and concentrated *in vacuo* to give the crude product. Purification by flash chromatography on SiO₂ eluting with 20% ethyl acetate/ heptane gives 2.85 g, (96% yield) of desired product. ¹H-NMR (CDCl₃, 300 MHz): δ 7.4 (s, 1 H), 7.15 (d, 1 H), 6.85 (s, 1 H), 4.45 (t, 2H), 3.65 (t, 2H), 3.3 (s, 3H), 2.6 (s, 3H). LCMS *m*/*z*: [M+H]⁺=224

### E. 1-[5-Chloro-1-(2-methoxy-ethyl)-7-methyl-1H-indol-3-yl]-2,2,2-trifluoro-ethanone

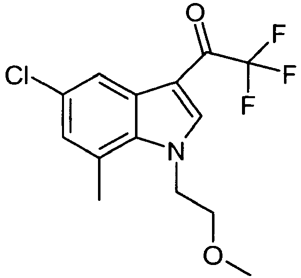

To a solution of 5-chloro-1-(2-methoxy-ethyl)-7-methyl-1*H*-indole(1.45 g, 6.5 mmol) in DMF (10 mL) at 0 °C is added TFAA (0.75 mL). After 2h the reaction mixture is poured into EtOAc and the organic layer washed with water and brine. The organic is dried over MgSO₄, filtered and concentrated *in vacuo* to give the crude product. The crude product is triturated with CH₂Cl₂ to give a white solid (1.2g, 58% yield). ¹H-NMR (DMSO-*d₆*, 300 MHz): δ 8.45 (s, 1 H), 8.15 (s, 1 H), 7.2 (s, 1 H), 4.7 (t, 2H), 3.7 (t, 2H), 3.2 (s, 3H), 2.7 (s, 3H). LCMS m/z: [M+H]⁺=320

### F. 5-Chloro-1-(2-methoxy-ethyl)-7-methyl-1H-indole-3-carboxylic acid

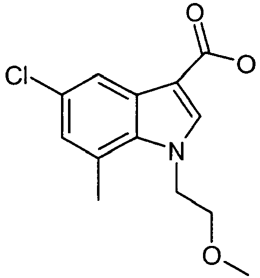

1-[5-chloro-1-(2-methoxy-ethyl)-7-methyl-1*H*-indol-3-yl]-2,2,2-trifluoro-ethanone (1.1 g, 3.43mmol) and 25 mL of 6M NaOH solution are heated to reflux for 2 days. The reaction is cooled to room temperature, diluted with water (100 ml) and acidified to pH=2 with concentrated HCl. The resulting white precipitate is collected to yield the desired product. (0.91 g, 98% yield). ¹H-NMR (CD₃OD, 300 MHz): δ12.2 (bs, 1 H), 8.0 (s, 1 H), 7.8 (s, 1 H), 7.0 (s, 1 H), 4.6 (t, 2H), 3.65 (t, 2H), 3.2 (s, 3H), 2.6 (s, 3H). LCMS *m*/*z*: [M+H]⁺=268

### G. N-(3-{1-[5-Chloro-1-(2-methoxy-ethyl)-7-methyl-1H-indole-3-carbonyl]-piperidin-4-yl}-4-fluoro-benzyl)-2,2,2-trifluoro-acetamide

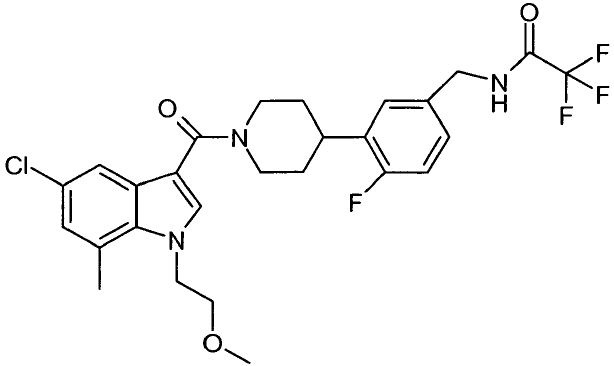

To a suspension of 5-chloro-1-(2-methoxy-ethyl)-7-methyl-1*H*-indole-3-carboxylic acid (0.608 g, 2.27 mmol), 2,2,2-trifluoro-*N*-(4-fluoro-3-piperidin-4-yl-benzyl)-acetamide hydrochloride (Example1B, 0.774 g, 2.27 mmol), and EDCI (0.523g, 2.72 mmol) in 50 mL CH₂Cl₂ is added Et₃N (0.76 ml, 5.45 mmol). The reaction is stirred at room temperature overnight. The reaction mixture is poured into EtOAc and the organic layer washed with sat NH₄Cl, water and brine. The organic is dried over MgSO₄, filtered and concentrated *in vacuo* to give the crude product. Purification by flash chromatography on SiO₂ eluting with 50% ethyl acetate/ heptane gives 0.72 g, (57% yield) of the desired product. ¹H-NMR (DMSO-*d₆*, 300 MHz): δ 10.0 (bs, 1H), 7.9 (d, 2H), 7.7 (s, 1 H), 7.55 (s, 1 H), 7.3 (d,1 H), 6.95 (s, 1 H), 4.6 (t, 21 H), 4.4 (m, 4H), 3.65 (t, 2H), 3.2 (s, 3H), 3.0-3.2 (m,3H), 2.65 (s, 3H), 1.6 (m, 2H), 1.8 (m, 2H). LCMS *m*/*z*: [M+H]⁺=554

### H. [4-(5-Aminomethyl-2-fluoro-phenyl)-piperidin-1-yl]-[5-chloro-1-(2-methoxy-ethyl)-7-methyl-1H-indol-3-yl]-methanone hydrochloride

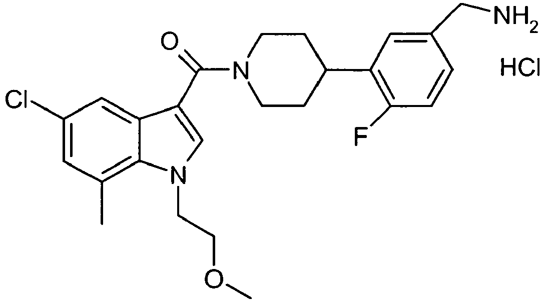

To a solution of N-(3-{1-[5-chloro-1-(2-methoxy-ethyl)-7-methyl-1*H*-indole-3-carbonyl]-piperidin-4-yl}-4-fluoro-benzyl)-2,2,2-trifluoro-acetamide (0.57g, 1.03 mmol) in 40 ml MeOH and 20 mL H₂O was added K₂CO₃ (1.42g, 10.3 mmol). The reaction mixture is heated to 50 °C overnight. The reaction mixture is concentrated *in vacuo* to remove most of the methanol. The residue is partitioned between H₂O and EtOAc. The two layers are separated, and the organic layer is washed with H₂O and brine, dried over Na₂SO₄, filtered, and concentrated *in vacuo.* The residue is taken up in 20 ml ether and 2.0 N HCl/Et₂O (5.0 mL, 10.0 mmol) is added dropwise. A solid precipitate forms and the ethereal solution is decanted off. The solid is washed with additional Et₂O and then isolated by filtration to give 0.325 g (64% yield) of the desired product. ¹H-NMR(DMSO-*d₆*, 300 MHz): δ 8.4 (bs, 2H), 7.75 (s, H), 7.6 (d, 2H), 7.4 (m, H), 7.2 (m, 1 H), 6.95 (s, 1 H), 4.6 (m, 3H), 4.4 (m, 4H), 4.0 (t, 2H), 3.65 (t, 2H), 3.2 (s, 3H), 3.0-3.2 (m,3H), 2.65 (s, 3H), 1.6 (m, 2H), 1.8 (m, 2H). LCMS *m*/*z*: [M+H]⁺=458

### EXAMPLE 2

### [4-(5-Aminomethyl-2-fluoro-phenyl)-piperidin-1-yl]-[6-fluoro-1-(2-methoxy-ethyl)-7-methyl-1H-indol-3-yl]-methanone hydrochloride

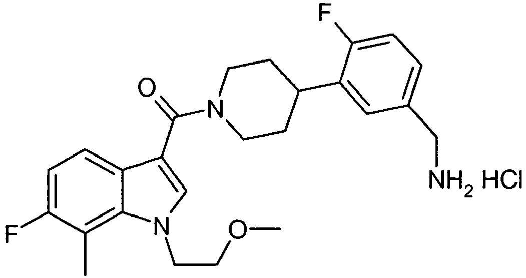

### A. 1-(2-Amino-4-fluoro-3-methyl-phenyl)-2-chloro-ethanone

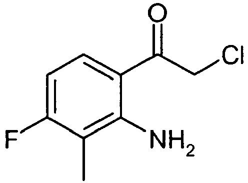

The title compound is prepared according to the procedure by Glennon, R. A. et al. J. Med. Chem. 1980, 23, 1222-1226 with 3-fluoro-2-methylphenylamine as the starting material. ¹H NMR (CD₃CN) δ 7.65-7.60 (m, H), 6.84 (bs, 2H), 6.44-6.38 (m, H), 4.77 (s, 2H), 2.02 (m, 3H).

### B. 6-Fluoro-7-methyl-1H-indole

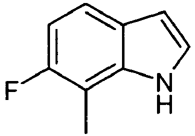

The title compound is prepared according to the procedure by Glennon, R. A. et al. J. Med. Chem. 1980, 23, 1222-1226 using 1-(2-amino-4-fluoro-3-methyl-phenyl)-2-chloro-ethanone as the starting material. ¹H NMR (CD₃CN) δ 9.32 (bs, 1 H), 7.38-7.34 (m, 1 H), 7.25-7.23 (m, 1 H), 7.85-7.78 (m, 1 H), 6.46-6.44 (m, 1 H), 2.38 (s, 3H).

### C. 6-Fluoro-1-(2-methoxy-ethyl)-7-methyl-1H-indole

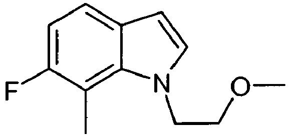

To a solution of 6-fluoro-7-methyl-1*H*-indole (1.9 g, 12.8 mmol) in 20 ml *N,N-*dimethylacetamide at rt is then added sodium hydride (369 mg, 14.61 mmol). The resulting mixture is stirred for 30 minutes at RT. 2-Bromoethylmethyl ether (2.4 ml, 25.54 mmol) is added and the resulting mixture is stirred at rt overnight. The mixture is diluted with 100 mL of water and 50 mL of ethyl acetate. The organic is separated and the aqueous phase is extracted again with 50 mL of ethyl acetate. The organic phase is washed with brine then separated and dried (MgSO₄). The organic phase is concentrated *in vacuo* and the crude residue is flash chromatographed over SiO₂ (eluted with heptane:EtOAc = 95:5) to afford 2.2 g (83%) of the title compound. ¹H NMR (CD₃CN) δ7.37-7.32 (m, 1 H), 7.12 (d, 1 H), 6.87-6.80 (m, 1 H), 6.40 (d, H), 4.46 (t, 2H), 3.64 (t, 2H), 3.23 (s, 3H), 2.87 (d, 3H).

### D. 2,2,2-Trifluoro-1-[6-fluoro-1-(2-methoxy-ethyl)-7-methyl-1H-indol-3-yl]-ethanone

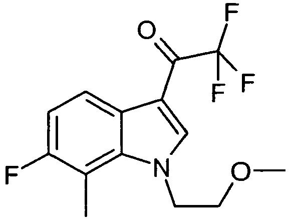

To a solution of 6-fluoro-1-(2-methoxy-ethyl)-7-methyl-1*H*-indole (2.20 g, 10.62 mmol) in *N,N*-dimethylformamide at 0 °C is added trifluoroacetic anhydride (1.8 mL, 12.94 mmol). The resulting mixture is stirred at 0 °C until completion. The mixture is diluted with 100 mL of water and the aqueous phase is extracted with 50 mL of ethyl acetate (x3). The organic phase is washed with brine then separated and dried (MgS0₄). The organic phase is concentrated *in vacuo* and the crude residue is flash chromatographed over SiO₂ (eluted with heptane:EtOAc = 95:5) to afford 2.95 g (92%) of the title compound as a white solid. ¹H NMR (CD₃CN) δ 8.15-8.10 (m, 2H), 7.15-7.08 (m, 1 H), 4.61 (t, 2H), 3.74 (t, 2H), 3.25 (s, 3H), 2.58 (m, 3H).

### E. 6-Fluoro-1-(2-methoxy-ethyl)-7-methyl-1H-indole-3-carboxylic acid

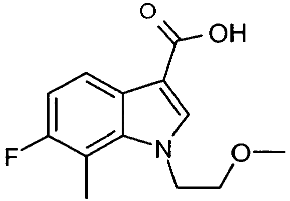

A solution of 2,2,2-trifluoro-1-[6-fluoro-1-(2-methoxy-ethyl)-7-methyl-1*H*-indol-3-yl]-ethanone (2.77 g, 9.13 mmol) in a solution of 6.25 N sodium hydroxide (35 mL, 219 mmol) is refluxed (~150 °C) until completion. The mixture is cooled to rt and then to 0 °C. The mixture is acidified with a solution of 6 N HCl to reach a pH ~2-3. The suspension is filtered and the cake rinsed with water (2 x 15 mL). Flash freeze the solid and lyophilized to afford 2.17 g (95%) of the title compound as an off-white solid. ¹H NMR (DMSO-d₆): δ 12.06 (bs, 1 H), 7.96 (s, 1 H), 7.90-7.85 (m, 1 H), 7.02 (t, H), 4.58 (t, 2H), 3.68 (t, 2H), 3.22 (s, 3H), 2.56 (m, 3H).

### F. 2,2,2-Trifluoro-N-(4-fluoro-3-{1-[6-fluoro-1-(2-methoxy-ethyl)-7-methyl-1H-indole-3-carbonyl]-piperidin-4-yl}-benzyl)-acetamide

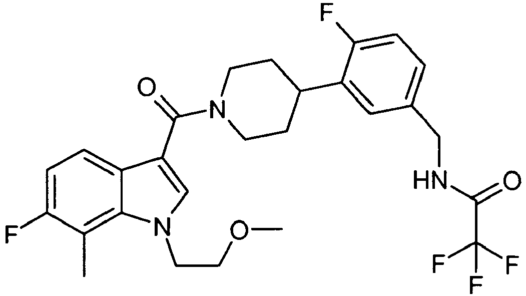

To a solution of 6-fluoro-1-(2-methoxy-ethyl)-7-methyl-1*H*-indole-3-carboxylic acid (626 mg, 2.49 mmol) in dichloromethane (40 mL) and *N,N*-dimethylformamide (2 mL) is added 1-(3-dimethylaminopropyl)-3-ethylcarbodiimide hydrochloride (601 mg, 3.13 mmol), 1-hydroxy-benzotriazole (369 mg, 2.73 mmol) and triethylamine (1.1 mL, 7.86 mmol). The resulting mixture is stirred for 20 minutes at rt. 2,2,2-trifluoro-*N*-(4-fluoro-3-piperidin-4-yl-phenyl)-acetamide hydrochloride [Example 1 B] (936 mg, 2.75 mmol) is added and heated at 40 °C for 4 hours and rt over night. The mixture is poured into water and the organic layer separated. The aqueous phase is extracted with ethyl acetate (x3). The organic phases are washed with brine then separated and dried (MgSO₄). The organic phase is concentrated *in vacuo* and the crude residue is flash chromatographed over SiO₂ (eluted with heptane:EtOAc = 15:85) to afford 1.31 g (98%) of the title compound as a viscous oil. ¹H NMR (CD₃CN): δ 8.01 (bs, 1 H), 7.57-7.52 (m, 1 H), 7.41 (s, 1 H), 7.28-7.25 (m, 1 H), 7.18-7.13 (m, 1 H), 7.07-7.03 (m, 1 H), 6.95-6.89 (m, 1 H), 4.54-4.38 (m, 6H), 3.68 (t, 2H), 3.24 (s, 3H), 3.12-3.01 (m, 3H), 2.57 (m, 3H), 1.87-1.62 (m, 4H).

### G. [4-(5-Aminomethyl-2-fluoro-phenyl)-piperidin-1-yl]-[6-fluoro-1-(2-methoxy-ethyl)-7-methyl-1H-indol-3-yl]-methanone hydrochloride

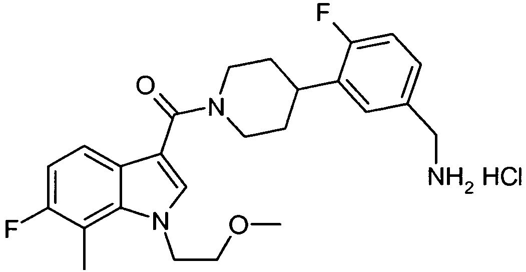

To a solution of 2,2,2-trifluoro-*N*-(4-fluoro-3-{1-[6-fluoro-1-(2-methoxy-ethyl)-7-methyl-1*H*-indole-3-carbonyl]-piperidin-4-yl}-benzyl)-acetamide (1.31 g, 2.44 mmol) in methanol:water (11:5) is added potassium carbonate (3.38 g, 24.49 mmol). The resulting mixture is stirred at rt over night. The solvent was removed *in vacuo* and the aqueous residue was partitioned between ethyl acetate and water. The organic layer is separated and the aqueous phase is extracted twice with ethyl acetate. Combined all the organics, washed with brine, dried over MgSO₄, filtered and concentrated down *in vacuo* to yield a viscous oil. To the later oil, HCl in dioxane (10 mL, 40.0 mmol) is added and stirred for 20 minutes. The mixture is vacuum dry and the residue is triturated with ether over night. The suspension is filtered and the cake is rinsed with ether twice. The solid is dried under vacuum to afford 758 mg (65%) of the title compound as a white solid. ¹H NMR (DMSO-*d₆*): δ 8.29 (bs, 3H), 7.64 (s, 1 H), 7.58-7.51 (m, 2H), 7.37 (m, 1 H), 7.22 (t, 1 H), 6.98 (t, 1 H), 4.56 (t, 2H), 4.43 (bd, 2H), 4.00 (m, 2H), 3.68 (m, 2H), 3.22 (s, 3H), 3.16-3.06 (m, 3H), 2.56 (s, 3H), 1.82-1.60 (m, 4H).

### EXAMPLE 3

### [4-(5-Aminomethyl-2-fluoro-phenyl)-piperidin-1-yl]-[5-fluoro-1-(2-methoxy-ethyl)-7-methyl-1H-indol-3-yl]-methanone hydrochloride

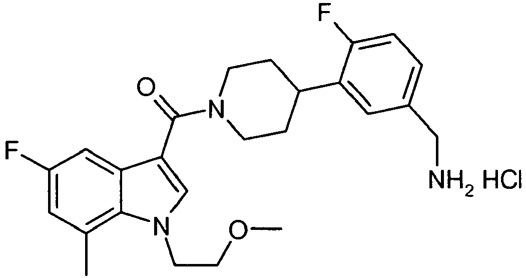

### A. 1-(2-Amino-5-fluoro-3-methyl-phenyl)-2-chloro-ethanone

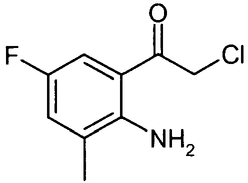

The title compound is prepared in a similar manner as Example 2A using 4-fluoro-2-methyl-phenylamine as the starting material. ¹H NMR (CD₃CN): δ7.32-7.28 (m, 1H), 7.13-7.09 (m, 1 H), 6.51 (bs, 2H), 4.78 (s, 2H), 2.12 (s, 3H).

### B. 5-Fluoro-7-methyl-1H-indole

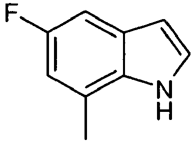

The title compound is prepared in a similar manner as Example 2B using 1-(2-amino-5-fluoro-3-methyl-phenyl)-2-chloro-ethanone as the starting material. ¹H NMR (CD₃CN): δ9.32 (bs, 1 H), 7.30 (t, 1 H), 7.12-7.08 (m, 1 H), 6.78-6.74 (m, 1 H), 6.46-6.44 (m, 1 H), 2.47 (s, 3H).

### C. 5-Fluoro-1-(2-methoxy-ethyl)-7-methyl-1H-indole

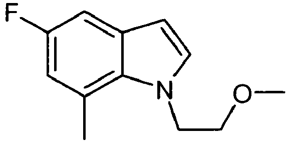

The title compound is prepared in a similar manner as Example 1D using 5-fluoro-7-methyl-1*H*-indole as the starting material. ¹H NMR (CD₃CN): δ 7.18 (d, 1 H), 7.09-7.05 (m, 1H), 6.72-6.69 (m, 1 H), 6.39 (d, 1 H), 4.47 (t, 2H), 3.63 (t, 2H), 3.22 (s, 3H), 2.66 (d, 3H).

### D. 2,2,2-Trifluoro-1-[5-fluoro-1-(2-methoxy-ethyl)-7-methyl-1H-indol-3-yl]-ethanone

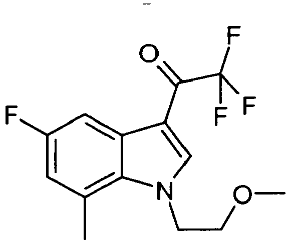

The title compound is prepared in a similar manner as Example 2D using 5-fluoro-1-(2-methoxy-ethyl)-7-methyl-1*H*-indole as the starting material. ¹H NMR (CD₃CN): δ 8.16 (m, 1 H), 7.86-7.82 (m, 1 H), 6.96-6.92 (m, 1 H), 4.60 (t, 2H), 3.73 (t, 2H), 3.25 (s, 3H), 2.69 (m, 3H).

### E. 5-Fluoro-1-(2-methoxy-ethyl)-7-methyl-1H-indole-3-carboxylic acid

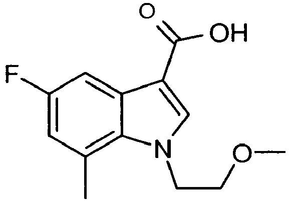

The title compound is prepared in a similar manner as Example 2E using 2,2,2-trifluoro-1-[5-fluoro-1-(2-methoxy-ethyl)-7-methyl-1*H*-indol-3-yl]-ethanone as the starting material. ¹H NMR (DMSO-*d₆*): δ12.08 (bs, 1 H), 8.00 (s, 1 H), 7.58-7.55 (m, 1 H), 6.87 (d, 1 H), 4.56 (t, 2H), 3.67 (t, 2H), 3.22 (s, 3H), 2.67 (s, 3H).

### F. 2,2,2-Trifluoro-N-(4-fluoro-3-{1-[5-fluoro-1-(2-methoxy-ethyl)-7-methyl-1H-indole-3-carbonyl]-piperidin-4-yl}-benzyl)-acetamide

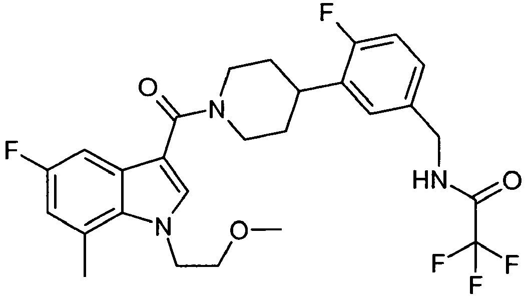

The title compound is prepared in a similar manner as Example 2F using 5-fluoro-1-(2-methoxy-ethyl)-7-methyl-1*H*-indole-3-carboxylic acid and 2,2,2-trifluoro-*N*-(4-fluoro-3-piperidin-4-yl-benzyl)-acetamide hydrochloride as the starting materials. ¹H NMR (CD₃CN): δ8.17 (bs, 1 H), 7.48 (s, 1 H), 7.30-7.24 (m, 2H), 7.17-7.12 (m, H), 7.06-7.00 (m, 1 H), 6.79-6.75 (m, 1 H), 4.53-4.49 (m, 4H), 4.37 (d, 2H), 3.66 (t, 2H), 3.23 (s, 3H), 3.19-3.01 (m, 3H), 2.66 (m, 3H), 1.84-1.61 (m, 4H).

### G. [4-(5-Aminomethyl-2-fluoro-phenyl)-piperidin-1-yl]-[5-fluoro-1-(2-methoxy-ethyl)-7-methyl-1H-indol-3-yl]-methanone hydrochloride

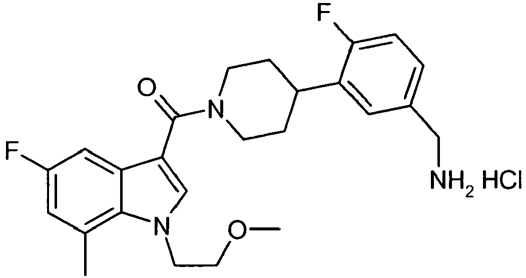

The title compound is prepared in a similar manner as Example 2G using 2,2,2-trifluoro-*N*-(4-fluoro-3-{1-[5-fluoro-1-(2-methoxy-ethyl)-7-methyl-1*H*-indole-3-carbonyl]-piperidin-4-yl}-benzyl)-acetamide as the starting material. ¹H NMR (DMSO-*d₆*): δ 8.28 (bs, 3H), 7.71 (s, 1 H), 7.56-7.54 (m, 1 H), 7.37 (m, 1 H), 7.27-7.20 (m, 2H), 6.87 (d, 1 H), 4.56 (m, 2H), 4.42 (br d, 2H), 4.00 (m, 2H), 3.66 (m, 2H), 3.22 (s, 3H), 3.16-3.07 (m, 3H), 2.67 (s, 3H), 1.82-1.64 (m, 4H).

### EXAMPLE 4

### [4-(5-Aminomethyl-2-fluoro-phenyl)-piperidin-1-yl]-[4-fluoro-1-(2-methoxy-ethyl)-7-methyl-1H-indol-3-yl]-methanone hydrochloride

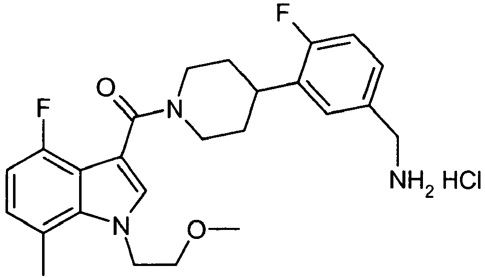

### A. 1-(2-Amino-6-fluoro-3-methyl-phenyl)-2-chloro-ethanone

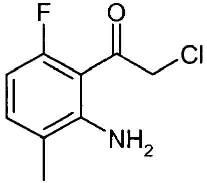

The title compound is prepared in a similar manner as Example 2A using 5-fluoro-2-methyl-phenylamine as the starting material. ¹H NMR (CDCl₃): δ 7.15-7.10 (m, 1H), 6.49 (bs, 2H), 6.30 (dd, 1 H), 4.74 (s, 2H), 2.12 (s, 3H).

### B. 4-Fluoro-7-methyl-1H-indole

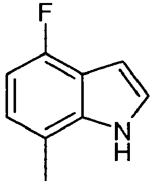

The title compound is prepared in a similar manner as Example 2B using 1-(2-amino-6-fluoro-3-methyl-phenyl)-2-chloro-ethanone as the starting material. ¹H NMR (CDCl₃): δ8.12 (bs, 1 H), 7.18 (t, 1 H), 6.90-6.86 (m, 1 H), 6.73-6.67 (m, 1 H), 6.65-6.63 (m, 1H), 2.45 (s, 3H).

### C. 4-Fluoro-1-(2-methoxy-ethyl)-7-methyl-1H-indole

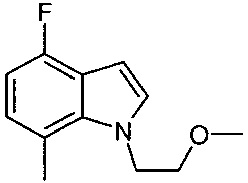

The title compound is prepared in a similar manner as Example 2C using 4-fluoro-7-methyl-1*H*-indole as the starting material. ¹H NMR (CDCl₃): δ 7.02 (d, 1 H), 6.80-6.76 (m, 1 H), 6.66-6.60 (m, 1 H), 6.54 (d, 1 H), 4.48 (t, 2H), 3.65 (t, 2H), 3.28 (s, 3H), 2.63 (d, 3H).

### D. 2,2,2-Trifluoro-1-[4-fluoro-1-(2-methoxy-ethyl)-7-methyl-1H-indol-3-yl]-ethanone

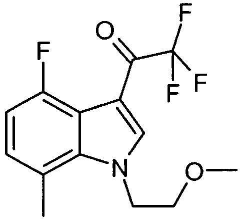

The title compound is prepared in a similar manner as Example 2D using 4-fluoro-1-(2-methoxy-ethyl)-7-methyl-1*H*-indole as the starting material. ¹H NMR (CD₃CN): δ 8.14 (m, 1 H), 7.10-7.05 (m, 1 H), 6.93-6.86 (m, 1 H), 4.63 (t, 2H), 3.72 (t, 2H), 3.25 (s, 3H), 2.66 (m, 3H).

### E. 4-Fluoro-1-(2-methoxy-ethyl)-7-methyl-1H-indole-3-carboxylic acid

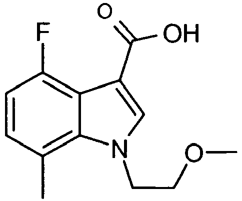

The title compound is prepared in a similar manner as Example 2E using 2,2,2-trifluoro-1-[4-fluoro-1-(2-methoxy-ethyl)-7-methyl-1*H*-indol-3-yl]-ethanone as the starting material. ¹H NMR (DMSO-*d₆*): δ 11.89 (bs, 1 H), 7.98 (s, 1 H), 6.95-6.91 (m, 1 H), 6.82-6.76 (m, 1 H), 4.59 (t, 2H), 3.66 (t, 2H), 3.22 (s, 3H), 2.63 (s, 3H).

### F. 2,2,2-Trifluoro-N-(4-fluoro-3-{1-[4-fluoro-1-(2-methoxy-ethyl)-7-methyl-1H-indole-3-carbonyl]-piperidin-4-yl}-benzyl)-acetamide

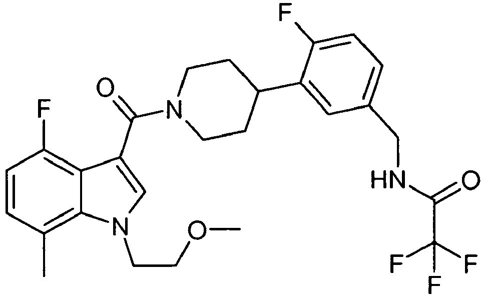

The title compound is prepared in a similar manner as Example 2F using 4-fluoro-1-(2-methoxy-ethyl)-7-methyl-1*H*-indole-3-carboxylic acid and 2,2,2-trifluoro-*N*-(4-fluoro-3-piperidin-4-yl-benzyl)-acetamide hydrochloride as the starting materials. ¹H NMR (CDCl₃): δ7.31 (s, 1 H), 7.15-7.10 (m, 2H), 7.03-6.97 (m, 1 H), 6.91-6.84 (m, 2H), 6.75-6.68 (m, 1 H), 4.53-4.46 (m, 4H), 3.70 (t, 2H), 3.31 (s, 3H), 3.1-3.02 (m, 4H), 2.66 (m, 3H), 1.74 (bs, 5H).

### G. [4-(5-Aminomethyl-2-fluoro-phenyl)-piperidin-1-yl]-[4-fluoro-1-(2-methoxy-ethyl)-7-methyl-1H-indol-3-yl]-methanone hydrochloride

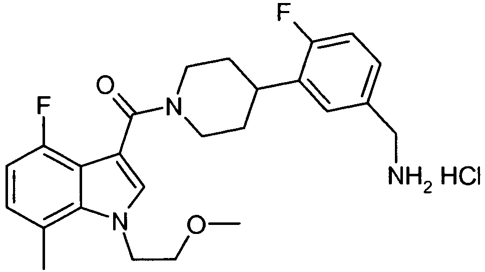

The title compound is prepared in a similar manner as Example 2G using 2,2,2-trifluoro-*N*-(4-fluoro-3-{1-[4-fluoro-1-(2-methoxy-ethyl)-7-methyl-1*H*-indole-3-carbonyl]-piperidin-4-yl}-benzyl)-acetamide as the starting material. ¹H NMR (DMSO-*d₆*): δ 8.33 (bs, 3H), 7.51 (m, 2H), 7.40-7.35 (m, 1 H), 7.24-7.18 (m, 1 H), 6.92-6.87 (m, 2H), 6.79-6.72 (m, 1 H), 4.56 (m, 2H), 4.30 (bs, 2H), 4.00 (dd, 2H), 3.66 (m, 2H), 3.22 (s, 3H), 3.16-2.99 (m, 3H), 2.64 (s, 3), 1.73-1.60 (m, 4H).

### EXAMPLE 5

### [4-(5-Aminomethyl-2-fluoro-phenyl)-piperidin-1-yl]-[4-fluoro-1-(2-methoxy-ethyl)-7-trifluoromethoxy-1H-indol-3-yl]-methanone hydrochloride

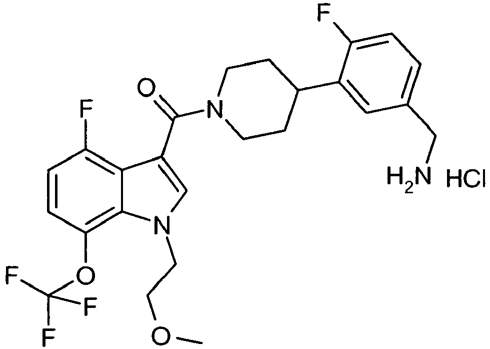

### A. 1-Fluoro-2-nitro-4-trifluoromethoxy-benzene and 4-Fluoro-2-nitro-1-trifluoromethoxy-benzene

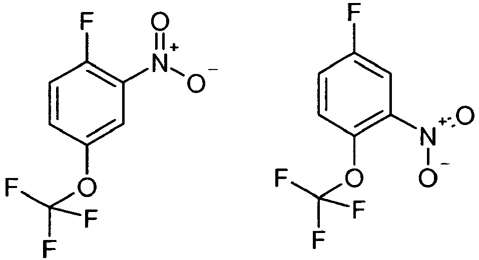

To a mixture of 1-fluoro-4-trifluoromethoxy-benzene (31.57 g, 0.18 mol) in conc. H₂SO₄ (100 mL) at 0°C is added conc. HNO₃ (30 mL) dropwise over a 10 min period. After the mixture is stirred at 0 °C for 1 h, it was poured into ice. The mixture is extracted with EtOAc. The organic extract is washed with H₂O (3x) and brine, dried over MgSO₄, filtered, and concentrated *in vacuo* to yield 38 g (96%) of the product as a mixture of 1-fluoro-2-nitro-4-trifluoromethoxy-benzene and 4-fluoro-2-nitro-1-trifluoromethoxy-benzene (~30/70, based upon ¹⁹F NMR). ¹H NMR (CDCl₃): δ 8.00-7.90 and 7.80-7.65 (m, 1 H), 7.60-7.25 (m, 2H); ¹⁹F NMR (CDCl₃) δ -57.59 and -58.11 (s, 3F), -109.07 and -117.90 (t, *J* = 6.2 and d, *J* = 6.2, 1F).

### B. 2-Fluoro-5-trifluoromethoxy-phenylamine and 5-Fluoro-2-trifluoromethoxy-phenylamine

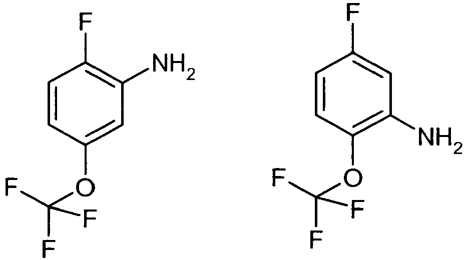

A mixture of 1-fluoro-2-nitro-4-trifluoromethoxy-benzene and 4-fluoro-2-nitro-1-trifluoromethoxy-benzene and Raney Ni (2800) in MeOH (250 mL) is hydrogenated at 40 psi for 5 h (or until no more H₂ is consumed). The catalyst is filtered off, and the filtrate is concentrated *in vacuo.* The residue is redissolved in CH₂Cl₂, dried over MgSO₄, filtered, and concentrated *in vacuo* to yield 27.4 g of the product (dark brown liquid) as a mixture of 2-fluoro-5-trifluoromethoxy-phenylamine and 5-fluoro-2-trifluoromethoxy-phenylamine (~30/70, based upon ¹⁹F NMR). ¹H NMR (CDCl₃): δ 7.15-6.90 (m, 1 H), 6.70-6.30 (m, 2H), 3.60-4.25 (br m, 1H); ¹⁹F NMR (CDCl₃) δ -57.77 and -57.86 (s, 3F), -113.83 and -137.09 (1 H); MS 196 (M+1, 100%).

### C. (5-Fluoro-2-trifluoromethoxy-phenyl)-carbamic acid ethyl ester

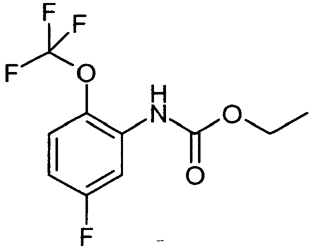

To a solution of 2-fluoro-5-trifluoromethoxy-phenylamine and 5-fluoro-2-trifluoromethoxy-phenylamine (27.4 g, 0.14 mol) and pyridine (15.3 mL) in THF (200 mL) is added ethyl chloroformate (16.1 mL, 0.19 mol) dropwise over a 3 min period. More pyridine and chloroformate (~1.8 eq) are added until all starting material disappears. The reaction mixture is partitioned between H₂O and EtOAc. The two layers are separated, and the organic layer is washed with HCl (1 M), H₂O, and brine, dried over MgSO₄, filtered, and concentrated *in vacuo.* The crude material is purified on silica gel with heptane/EtOAc (100/0 to 70/30) as eluant to yield 22.66 g (60%) of the product as a yellow liquid. ¹H NMR (CDCl₃): δ 8.10-7.95 (m, 1 H), 7.25-7.15 (m, 1 H), 6.93 (br s, 1 H), 6.80-6.65 (m, 1 H), 4.27 (q, *J* = 7.2 Hz, 2H), 1.34 (t, *J* = 7.1 Hz, 3H); ¹⁹F NMR (CDCl₃) δ -58.53 (s, 3F), -112.08 (s, 1 F); MS 309 (M+CH₃CN+1, 100%), 268 (M+1).

### D. (3-Fluoro-2-iodo-6-trifluoromethoxy-phenyl)-carbamic acid ethyl ester

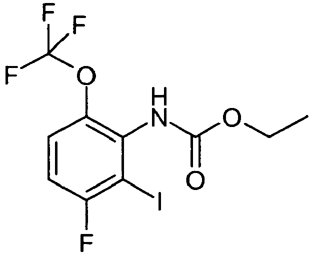

To a solution of (5-fluoro-2-trifluoromethoxy-phenyl)-carbamic acid ethyl ester (1.0 g, 3.74 mmol) in THF (20 mL) at -78 °C is added sec-BuLi (6.4 mL, 1.4 M in cyclohexane, 8.98 mol) dropwise over a 10 min period. After 1 h, a solution of I₂ (1.3 g, 5.23 mmol) in THF (7 mL) is added dropwise over a 5 min period. After 30 min, the reaction mixture is quenched with sat NH₄Cl, and the cooling bath is removed. The mixture is partitioned with H₂O and EtOAc. The two layers are separated, and the organic layer is washed with H₂O and brine, dried over MgSO₄, filtered, and concentrated in vacuo. The crude material is purified on silica gel with heptane/EtOAc (100/0 to 70/30) as eluant to yield 1.23 g (83%) of the product as a white solid. ¹H NMR (CDCl₃): δ 7.35-7.25 (m, 1 H), 7.15-7.00 (m, 1H), 6.19 (br s, 1 H), 4.25 (q, *J* = 7.1 Hz, 2H), 1.31 (t, *J* = 7.1 Hz, 3H); ¹⁹F NMR (CDCl₃) -57.54 (s, 3F), - 89.14 (t, *J* = 6.2 Hz, 1 F); MS 435 (M+CH₃CN+1, 100%), 394 (M+1).

### E. (3-Fluoro-6-trifluoromethoxy-2-trimethylsilanylethynyl-phenyl)-carbamic acid ethyl ester

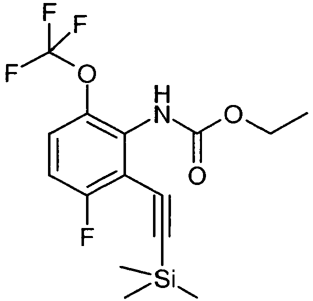

A mixture of (3-fluoro-2-iodo-6-trifluoromethoxy-phenyl)-carbamic acid ethyl ester (480 mg, 1.22 mmol), TMS-acetylene (229 µL, 1.59 mol), TEA (256 µL, 1.83 mmol), Cul (12 mg, 5% mol), and bistriphenylphosphinepalladium (II) chloride (43 mg, 5% mol) in degassed THF (10 mL) is stirred at rt for 1 h. More TMS-acetylene is added in order to drive the reaction to completion. The mixture is partitioned between H₂O and EtOAc. The two layers are separated, and the organic layer is washed with brine, dried over MgSO₄, filtered, and concentrated *in vacuo.* The crude material was purified on silica gel with heptane/EtOAc (100/0 to 80/20) as eluant to yield 380 mg (87%) of the product as a brown solid. ¹H NMR (CDCl₃): δ 7.30-7.15 (m, 1 H), 7.05-6.95 (m, 1 H), 6.33 (br s, 1 H), 4.24 (q, *J* = 7.1 Hz, 2H), 1.30 (t, *J* = 7.2 Hz, 3H), 0.27 (s, 3H); ¹⁹F NMR (CDCl₃) δ -57.54 (s, 3F), -108.17 (d, *J* = 6.2, 1 F); MS 364 (M+1, 100%).

### F. 4-Fluoro-7-trifluoromethoxy-1H-indole

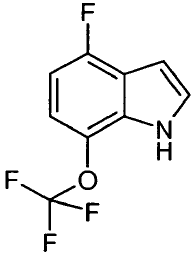

A mixture of (3-fluoro-6-trifluoromethoxy-2-trimethylsilanylethynyl-phenyl)-carbamic acid ethyl ester (390 mg, 1.07 mmol) and KOH (600 mg, 10.7 mmol) in *t*-BuOH (10 mL) is heated at 80 °C for 6h. The solvent is removed *in vacuo,* and the residue is partitioned between H₂O and Et₂O. The two layers are separated, and the organic layer is washed with brine, dried over MgSO₄, filtered, and concentrated *in vacuo.* The crude material is purified on silica gel with heptane/EtOAc (100/0 to 80/20) as eluant 176 mg (75%) of the product as a clear yellow liquid. ¹H NMR (CDCl₃): δ 8.46 (br, s, 1H), 7.30-7.15 (m, 1 H), 7.10-6.95 (m, 1H), 6.85-6.55 (m, 2H); ¹⁹F NMR (CDCl₃) δ -57.75 (s, 3F), -122.66 (d, *J* = 9.0 Hz, 1 F).

### G. 4-Fluoro-1-(2-methoxy-ethyl)-7-trifluoromethoxy-1H-indole

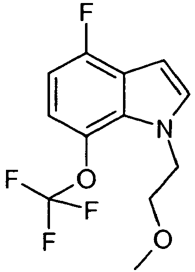

A mixture of powder KOH (200 mg, 3.56 mmol) in DMSO (5 mL) is stirred at rt for 10 min. A solution of 4-fluoro-7-trifluoromethoxy-1*H*-indole (170 mg, 0.78 mmol) in DMSO (5 mL) is then added. After 30 min, 2-methoxyethyl bromide (147 L, 1.56 mmol) is added. After this reaction mixture was stirred at rt for 4 h, it is partitioned between H₂O and Et₂O. The two layers are separated, and the aqueous layer is extracted with Et₂O once. The combined organic extracts are washed with H₂O (2X) and brine, dried over MgSO₄, filtered, and concentrated *in vacuo.* The crude material is purified on silica gel with heptane/EtOAc (100/0 to 50/50) as eluant to yield 128 mg (59%) of the product as a yellow oil. ¹H NMR (CDCl₃): δ 7.14 (d, *J* = 3.1 Hz, 1 H), 7.05-6.90 (m, 1 H), 6.69 (t, *J* = 1.2 hz, 1 H), 6.59 (d, *J* = 3.2 Hz, 2H), 4.45 (t, *J* = 5.3 Hz, 2H), 3.69 (t, *J* = 5.3 Hz, 2H), 3.30 (s, 3H); ¹⁹F NMR (CDCl₃) δ -56.08 (s, 3F), - 123.48 (d, *J* = 9.0 Hz, 1 F); MS 278 (M+1, 100%).

### H. 2,2,2-Trifluoro-1-[4-fluoro-1-(2-methoxy-ethyl)-7-trifluoromethoxy-1H-indol-3-yl]-ethanone

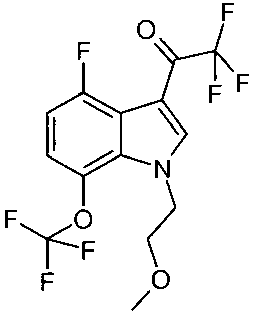

To a mixture of 4-fluoro-1-(2-methoxy-ethyl)-7-trifluoromethoxy-1*H*-indole(128 mg, 0.46 mmol) in DMF (5 mL) is added TFAA (77 µL, 0.55 mmol). This mixture is stirred at rt for 2 h. More TFAA (231 µL, 1.65 mmol) is added, and the reaction mixture is heated at 50 °C overnight. The mixture is partitioned between H₂O and EtOAc. The two layers are separated, and the organic layer is washed with H₂O and brine, dried over MgSO₄, filtered, and concentrated *in vacuo.* The crude material is purified on silica gel with heptane/EtOAc (95/5 to 70/30) as eluant to yield 120 mg (69%) of the product as a white solid. ¹H NMR (CDCl₃): δ 8.01 (d, *J* = 1.6 Hz, 1 H), 7..25-7.15 (m, 1 H), 6.98 (t, *J* = 0.9 Hz, 1 H), 4.55 (t, *J* = 4.8 Hz, 2H), 3.73 (t, *J* = 5.0 Hz, 2H), 3.32 (s, 3H); ¹⁹F NMR (CDCl₃) δ -56.79 (s, 3F), -71.35 (s, 3F), -108.13 (d, *J* = 9.0 Hz, 1 F); MS 374 (M+1, 100%).

### 1. 4-Fluoro-1-(2-methoxy-ethyl)-7-trifluoromethoxy-1H-indole-3-carboxylic acid

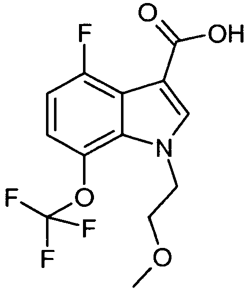

A mixture of 2,2,2-trifluoro-1-[4-fluoro-1-(2-methoxy-ethyl)-7-trifluoromethoxy-1*H-*indol-3-yl]-ethanone (120 mg, 0.32 mmol) in MeOH (5 mL) and NaOH (5 M, 10 mL) is heated at 80 °C for 1 h. This mixture is concentrated *in vacuo* to remove the methanol. The residue is diluted with H₂O, and then acidified to pH 2 with HCl (3 M). The acidified mixture is extracted with EtOAc (2X). The combined organic extracts are washed with H₂O and brine, dried over MgSO₄, filtered, and concentrated in vacuo to yield 79 mg (76%) of the product as a white powder. ¹H NMR (CDCl₃): δ 7.97 (s, 1 H), 7.20-7.10 (m, 1 H), 6.90 (t, *J* = 9.7 Hz, 1 H), 4.50 (t, *J* = 5.0 Hz, 2H), 3.72 (t, *J* = 5.0 Hz, 2H), 3.32 (s, 3H); ¹⁹F NMR (EDCl₃) δ -56.74 (s, 3F), -113.47 (s, 1 F); MS 363 (M+CH₃CN+1), 322 (M+1, 100%).

### J. 2,2,2-Trifluoro-N-(4-fluoro-3-{1-[4-fluoro-1-(2-methoxy-ethyl)-7-trifluoromethoxy-1H-indole-3-carbonyl]-piperidin-4-yl}-benzyl)-acetamide

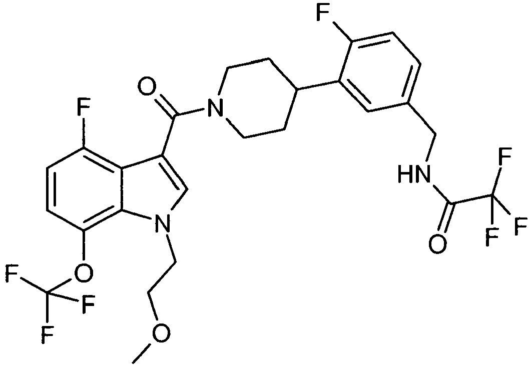

A mixture of 4-fluoro-1-(2-methoxy-ethyl)-7-trifluoromethoxy-1*H*-indole-3-carboxylic acid (79 mg, 0.24 mmol), Et₃N (74 µL, 0.52 mmol), 2,2,2-trifluoro-*N*-(4-fluoro-3-piperidin-4-yl-benzyl)-acetamide hydrochloride (100 mg, 0.29 mmol), and EDCI (60 mg, 031 mmol) in CH₂Cl₂ (20 mL) is stirred at rt for 5 h. The mixture is partitioned between H₂O and CH₂Cl₂. The two layers are separated, and the organic layer is washed with brine, dried over MgSO₄, filtered, and concentrated *in vacuo.* The crude material is purified on silica gel with heptane/EtOAc (50/50 to 0/100) as eluant to yield 127 mg (87%) of the product as a white solid. ¹H NMR (CDCl₃): δ 7.40 (s, 1 H), 7.20-7.10 (m, 2H), 7.10-6.95 (m, 2H), 6.79 (t, *J* = 0.6, 1 H),6.69 (br s, 1 H), 4.90 (br s, 1 H), 4.55-4.35 (m, 4H), 3.95 (br s, 1 H), 3.72 (t, *J* = 5.1 Hz, 2H), 3.32 (s, 3H), 3.25-2.70 (m, 3H), 1.95-1.60 (m, 4H); ¹⁹F NMR (CDCl₃) δ -56.81 (s, 3F), -75.33 (s, 3F), -119.00 (s, 1 F), -121.37 (d, *J* = 9.3 Hz, 1 F); MS 608 (M+1, 100%).

### K. [4-(5-Aminomethyl-2-fluoro-phenyl)-piperidin-1-yl]-[4-fluoro-1-(2-methoxy-ethyl)-7-trifluoromethoxy-1H-indol-3-yl]-methanone hydrochloride

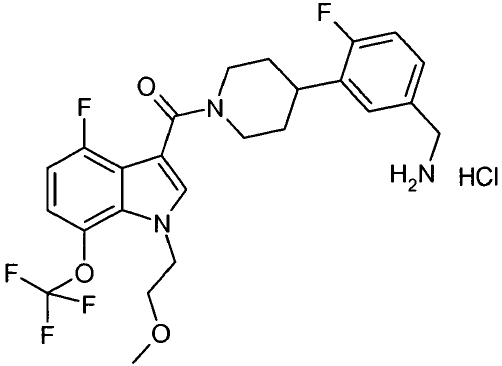

To a mixture of 2,2,2-Trifluoro-*N*-(4-fluoro-3-{1-[4-fluoro-1-(2-methoxy-ethyl)-7-trifluoromethoxy-1*H*-indole-3-carbonyl]-piperidin-4-yl}-benzyl)-acetamide (120 mg, 0.2 mmol) in MeOH (20 mL) is added aqueous K₂CO₃ (200 mg, 1.6 mmol, dissolved in 4 mL H₂O). This mixture is stirred at rt overnight. LC/MS indicates the reaction is completed. The reaction mixture is concentrated *in vacuo* to remove most of the methanol. The residue is partitioned between H₂O and EtOAc. The two layers are separated, and the organic layer is washed with H₂O and brine, dried over MgSO₄, filtered, and concentrated *in vacuo.* The residue is dissolved in Et₂O, and HCl in Et₂O (1.0 M, 1 mL) is added. The resulting suspension is concentrated *in vacuo,* and then dried *in vacuo* for 5 h to yield 85 mg (83%) of the product as beige solid. ¹H NMR (CD₃OD): δ 7.75-7.50 (m, 2H), 7..50-7.40 (m, 1 H), 7.40-7.30 (m, 1 H), 7.30-7.10 (m, 3H), 6.95-6.85 (m, 1 H), 4.54 (t, *J* = 5.0 Hz, 2H), 4.40-3.85 (m, 6H), 3.72 (t, *J* = 5.1 Hz, 2H), 3.60-2.90 (m, 2H), 2.15-1.55(m, 6H); ¹⁹F NMR (CD₃OD) δ -58.47 (s, 3F), -119.72 (s, 1 F), -123.00 (d, *J* = 5.9 Hz, 1 F); MS 512 (M+1, 100%).

### EXAMPLE 6

### [4-(5-Aminomethyl-2-fluoro-phenyl)-piperidin-1-yl]-[4-chloro-1-(2-methoxy-ethyl)-7-methyl-1H-indol-3-yl]-methanone hydrochloride

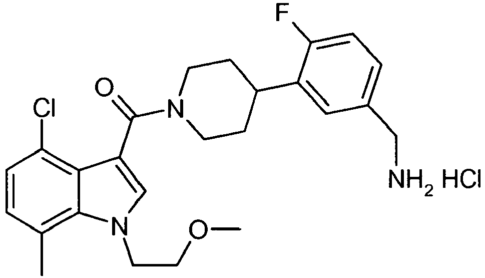

### A. 1-(2-Amino-6-chloro-3-methyl-phenyl)-2-chloro-ethanone

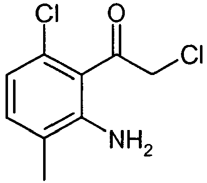

The title compound is prepared in a similar manner as Example 2A using 5-chloro-2-methylphenylamine as the starting material. ¹H NMR (CDCl₃): δ 7.05 (d, 1 H), 6.69 (d, 2H), 4.89 (bs, 2H), 4.72 (s, 2H), 2.14 (s, 3H).

### B. 4-Chloro-7-methyl-1H-indole

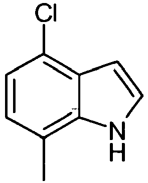

The title compound is prepared in a similar manner as Example 2B using 1-(2-amino-6-chloro-3-methyl-phenyl)-2-chloro-ethanone as the starting material. ¹H NMR (CDCl₃): δ 8.15 (bs, 1 H), 7.24 (t, 1 H), 7.07-7.05 (m, 1 H), 6.94-6.91 (m, 1 H), 6.69-6.66 (m, 1 H), 2.47 (s, 3H).

### C. 4-Chloro-1-(2-methoxy-ethyl)-7-methyl-1H-indole

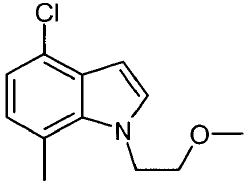

The title compound is prepared in a similar manner as Example 2C using 4-chloro-7-methyl-1*H*-indole as the starting material. ¹H NMR (CDCl₃): δ 7.12 (d, 1 H), 7.00-6.97 (m, 1 H), 6.84-6.82 (m, 1 H), 6.59 (d, 1 H), 4.51 (t, 2H), 3.68 (t, 2H), 3.30 (s, 3H), 2.67 (d, 3H).

### D. 2,2,2-Trifluoro-1-[4-Chloro-1-(2-methoxy-ethyl)-7-methyl-1H-indol-3-yl]-ethanone

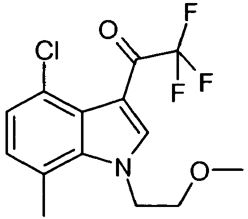

The title compound is prepared in a similar manner as Example 2D using 4-chloro-1-(2-methoxy-ethyl)-7-methyl-1*H*-indole as the starting material. ¹H NMR (CDCl₃): δ7.96 (m, 1 H), 7.22.-7.19 (m, 1 H), 7.00-6.98 (m, 1 H), 4.59 (t, 2H), 3.72 (t, 2H), 3.31 (s, 3H), 2.68 (m, 3H).

### E. 4-Chloro-1-(2-methoxy-ethyl)-7-methyl-1H-indole-3-carboxylic acid

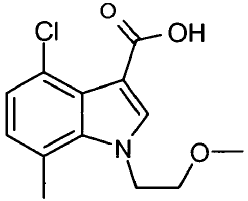

The title compound is prepared in a similar manner as Example 2E using 2,2,2-trifluoro-1-[4-chloro-1-(2-methoxy-ethyl)-7-methyl-1*H*-indol-3-yl]-ethanone as the starting material. ¹H NMR (DMSO-*d₆*): δ11.95 (bs, 1 H), 7.98 (s, 1 H), 7.08-7.06 (m, 1 H), 6.96-6.93 (m, 1 H), 4.59 (t, 2H), 3.65 (t, 2H), 3.22 (s, 3H), 2.66 (m, 3H).

### F. N-(3-{1-[4-Chloro-1-(2-methoxy-ethyl)-7-methyl-1H-indole-3-carbonyl]-piperidin-4-yl}-4-fluoro-benzyl)-2,2,2-trifluoro-acetamide

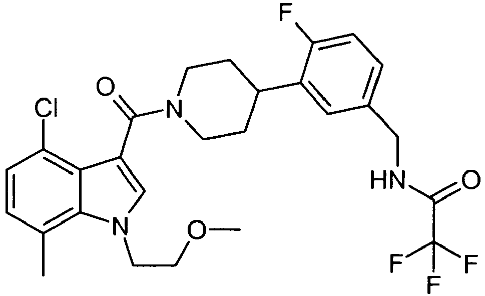

The title compound is prepared in a similar manner as Example 2F using 4-chloro-1-(2-methoxy-ethyl)-7-methyl-1*H*-indole-3-carboxylic acid and 2,2,2-trifluoro-*N*-(4-fluoro-3-piperidin-4-yl-phenyl)-acetamide hydrochloride as the starting material. ¹H NMR (EDCl₃): δ 7.16-7.09 (m, 3H), 7.04-6.96 (m, 2H), 6.89-6.86 (m, 1 H), 5.00 (bs, 2H), 4.52-4.49 (m, 2H), 4.46-4.42 (m, 2H), 3.68 (t, 2H), 3.30 (s, 3H), 3.19-3.06 (m, 3H), 2.92-2.83 (m, 1H), 2.68 (m, 3H), 1.91-1.63 (m, 4H).

### G. [4-(5-Aminomethyl-2-fluoro-phenyl)-piperidin-1-yl]-[4-chloro-1-(2-methoxy-ethyl)-7-methyl-1H-indol-3-yl]-methanone hydrochloride

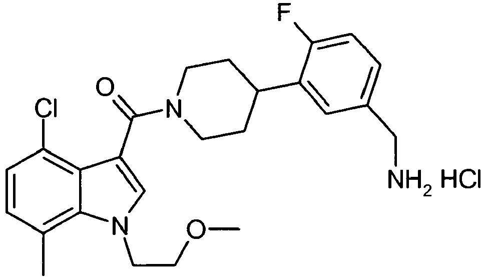

The title compound is prepared in a similar manner as Example 2G using *N*-(3-{1-[4-chloro-1-(2-methoxy-ethyl)-7-methyl-1*H*-indole-3-carbonyl]-piperidin-4-yl}-4-fluorobenzyl)-2,2,2-trifluoro-acetamide as the starting material. ¹H NMR (DMSO-*d₆*): δ 8.38 (br s, 3 H), 7.51 (bs, 2H), 7.40-7.36 (m, 1 H), 7.24-7.17 (m, 1 H), 7.03-7.00 (m, 1 H), 6.93-6.91 (m, 1 H), 4.77-4.73 (m, 1 H), 4.57-4.54 (m, 2H), 4.18 (bs, H), 4.01-3.97 (m, 2H), 3.65 (t, 2H), 3.21 (s, 3H), 3.10 (bs, 2H), 2.86 (bs, 1 H), 2.66 (s, 3H), 1.81-1.62 (m, 4H).

### EXAMPLE 7

### [4-(5-Aminomethyl-2-fluoro-phenyl)-piperidin-1-yl]-(1-butyl-7-fluoro-4-trifluoromethoxy-1H-indol-3-yl)-methanone hydrochloride.

### A. 1-Butyl-7-fluoro-4-trifluoromethoxy-1H-indole

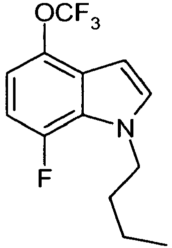

The title product (0.780 g, 98%) is obtained in a similar manner as described in Example 1D except using 7-fluoro-4-trifluoromethoxy-1H-indole (0.636 g, 2.9 mmol) as the starting material and n-butyl bromide as the alkylating agent. ¹H NMR (300 MHz, (CDCl₃): δ 7.27 (s, 1 H), 7.07 (d, 1 H), 6.83 (d, 1 H), 6.56 (d, 1 H), 4.27 (t, 2H), 1.82 (m, 2H), 1.32 (m, 2H), 0.94 (t, 3H); ¹⁹F NMR (CDCl₃) δ -57.69 (s, 3F), -136.80 (s, 1 H).

### B: 1-(1-Butyl-7-fluoro-4-trifluoromethoxy-1H-indol-3-yl )-2,2,2-trifluoro-ethanone.

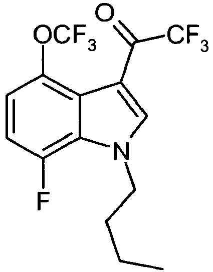

The title product (0.932 g, 93%) is obtained in a similar manner as described in Example 1 E except using 1-butyl-7-fluoro-4-trifluoromethoxy-1*H*-indole (0.780 g, 2.83 mmol) as the starting material. ¹H NMR (300 MHz, (CDCl₃): δ 7.27 (s, 1 H), 7.07 (d, 1 H), 6.83 (d, 1 H), 6.56 (d, 1 H), 4.27 (t, 2H), 1.82 (m, 2H), 1.32 (m, 2H), 0.94 (t, 3H); ¹⁹F NMR (CDCl₃) δ -57.90 (s, 3F), -71.10 (s, 3H), -134.86 (d, *J* = 12.9 Hz, 1 H).

### C: 1-Butyl-7-fluoro-4-trifluoromethoxy-1H-indole-3-carboxylic acid.

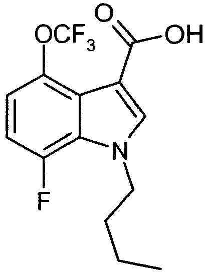

A mixture of 1-(1-butyl-7-fluoro-4-trifluoromethoxy-1*H*-indol-3-yl)-2,2,2-trifluoroethanone (23.4 g, 62.6 mmol) in MeOH (100 mL) and 5 M NaOH (100 mL) is heated at 80 °C overnight. LC/MS indicates that the reaction is complete. The reaction mixture is cooled to rt, and then concentrated *in vacuo* to remove most of the MeOH. The residue is dissolved in H₂O, and then washed with Et₂O once. The aqueous layer is slowly acidified to pH ∼2 with conc. HCl. The acidified suspension is extracted with Et₂O, and the organic extract is washed with H₂O and brine, dried over MgSO₄, filtered, and concentrated *in vacuo.* The residue is suspended in DCM/heptane (10/90). The white powder 1-butyl-7-fluoro-4-trifluoromethoxy-1*H-*indole-3-carboxylic acid (19.4 g, 96%) in the suspension is collected by suction filtration and air-dried. ¹H NMR (CDCl₃): δ 8.02 (s, 1 H), 7.15-7.05 (m, 1 H), 7.00-6.90 (m, 1H), 4.49 (t, *J* = 5.0 Hz, 2H), 3.75 (t, *J* = 4.9 Hz, 2H), 3.33 (s, 3H); ¹⁹F NMR (CDCl₃) δ -57.74 (s, 3F), -135.65 (d, *J* = 11.3 Hz, 1 F); MS 361 (M+CH₃CN+1), 320 (M+1, 100%).

### D: N-{3-[1-(1-Butyl-7-fluoro-4-trifluoromethoxy-1H-indole-3-carbonyl)-piperidin-4-yl]-4-fluoro-benzyl}-2,2,2-trifluoro-acetamide.

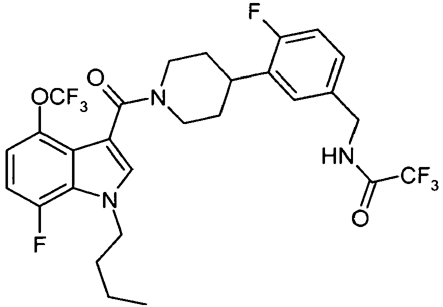

A mixture of 1-butyl-7-fluoro-4-trifluoromethoxy-1H-indole-3-carboxylic acid (19.1 g, 59.6 mmol), Et₃N (24.8 mL, 177.9 mmol), 2,2,2-trifluoro-N-(4-fluoro-3-piperidin-4-yl-benzyl)-acetamide hydrochloride (26.4 g, 77.5 mmol), and EDCI (17.1 g, 89.3 mmol) in CH₂Cl₂ is stirred at rt overnight. Both TLC and LC/MS indicate that the reaction is completed. The mixture is partitioned between H₂O and CH₂Cl₂. The two layers are separated, and the organic layer is washed with brine, dried over MgSO₄, filtered, and concentrated *in vacuo.* The crude material is purified on silica gel with heptane/EtOAc (40/60 to 0/100) as eluant to give the title compound (36 g, 99%) as a white foam. ¹H NMR (CDCl₃): δ 7.37 (s, 1H), 7.20-7.10 (m, 2H), 7.10-6.85 (m, 4H), 4.95 (br s, 1 H), 4.60-4.35 (m, 4H), 3.90 (br s, 1 H), 3.73 (t, *J* = 5.0 Hz, 2H), 3.32 (s, 3H), 3.25-2.70 (m, 3H), 2.05-1.50(m, 4H); ¹⁹F NMR (CDCl₃) δ -57.54 (s, 3F), -75.39 (s, 3F), -119.31 (s, 1 F), -134.96 (d, *J* = 11.3 Hz, 1 F); MS 608 (M+1, 100%).

### E: [4-(5-Aminomethyl-2-fluoro-phenyl)-piperidin-1-yl]-(1-butyl-7-fluoro-4-trifluoromethoxy-1H-indol-3-yl)-methanone hydrochloride.

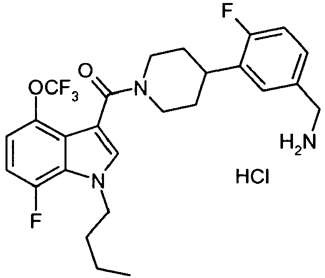

The title compound is prepared in a similar manner as Example 2G using *N*-{3-[1-(1-Butyl-7-fluoro-4-trifluoromethoxy-1*H*-indole-3-carbonyl)-piperidin-4-yl]-4-fluorobenzyl}-2,2,2-trifluoro-acetamide as the starting material. ¹H NMR (DMSO-d₆): δ 8.30 (br s, 2H), 7.79 (s, 1 H), 7.49 (d, 1 H), 7.40-7.38 (m, 1 H), 7.20 (t, 1 H), 7.18-7.02 (m, 2H), 4.32 (t, 2H), 4.01-3.95 (m, 2H), 3.19-3.09 (m, 4H), 1.80-1.74 (m, 2H), 1.70-1.60 (m, 4H), 1.30-1.20 (m, 2H), 0.89 (t, *J* =7.2Hz, 3H). ¹⁹F NMR (DMSO-d₆) δ -56.76 (s, 3F), -75.39 (s, 3F), -119.29 (s, 1 F), -135.15 (d, *J*=12.9 Hz, 1 F). MS 510 (M+1, 100%).

### EXAMPLE 8

### [4-(5-Aminomethyl-2-fluoro-phenyl)-piperidin-1-yl]-[4,7-dichloro-1-(2-methoxy-ethyl)-1H-indol-3-yl]-methanone hydrochloride

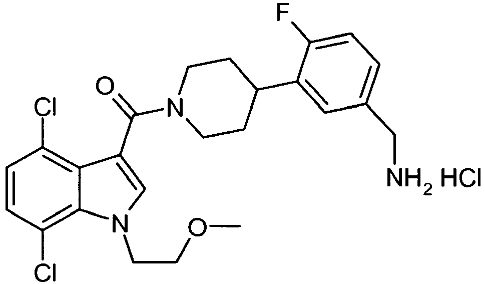

### A. 4,7-Dichloro-1H-indole-3-carboxylic acid methyl ester

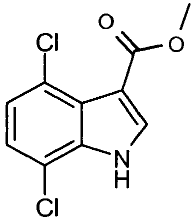

N-(2, 5-Dichloro-phenyl)-hydroxylamine is prepared according to the procedure by Evans, D. A. et al. Org. Lett. 2006, 8, 3351-3354 with 2, 5-dichloronitrobenzene as the starting material. The crude product is used for the next step without any purification following the procedure by Jih Ru Hwu et al. J. Org. Chem. 1994, 59, 1577-1582 to give the title compound. ¹H NMR (CD₃OD): δ 8.03 (s, 1 H), 7.22-7.15 (m, 2H), 3.86 (s, 3H).

### B. 4,7-Dichloro-1-(2-methoxy-ethyl)-1H-indole-3-carboxylic acid methyl ester

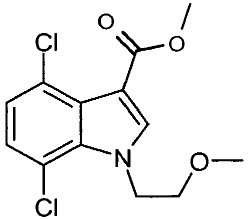

The title compound is prepared in a similar manner as Example 2C using 4, 7-dichloro-1*H*-indole-3-carboxylic acid methyl ester as the starting material. ¹H NMR (CDCl₃):δ7.73 (s, 1 H), 7.07 (d, 2H), 4.64 (t, 2H), 3.81 (s, 3H), 3.67 (t, 2H), 3.22 (s, 3H).

### C. 4.7-Dichloro-1-(2-methoxy-ethyl)-1H-indole-3-carboxylic acid

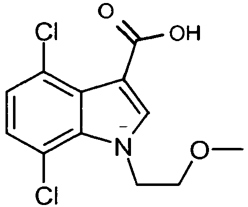

To a solution of 4,7-dichloro-1-(2-methoxy-ethyl)-1*H*-indole-3-carboxylic acid methyl ester (285 mg, 0.94 mmol) in THF:MeOH:H₂O (1:1:1) (15 mL) is added lithium hydroxide hydrate (270 mg, 6.43 mmol) and the mixture is stirred at rt overnight. The solvents were removed *in vacuo* and the aqueous residue flash freeze and lyophilized. The solid is triturated with DCM:MeOH (8:2), filtered and the filtrate is evaporated *in vacuo* and vacuum dried to give the title compound. ¹H NMR (CD₃OD): δ7.47 (s, 1 H), 7.10-7.07 (m, 2H), 4.70 (t, 2H), 3.72 (t, 2H), 3.27 (s, 3H).

### D. N-(3-{1-[4,7-Dichloro-1-(2-methoxy-ethyl)-1H-indole-3-carbonyl]-piperidin-4-yl}-4-fluoro-benzyl)-2,2,2-trifluoro-acetamide

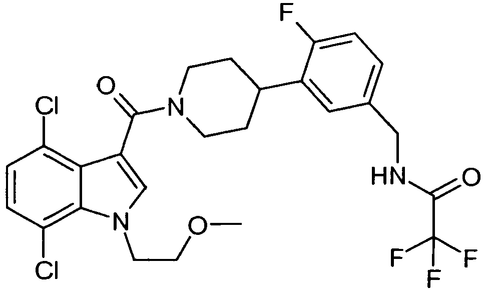

The title compound is prepared in a similar manner as Example 2F using 4,7-dichloro-1-(2-methoxy-ethyl)-1*H*-indole-3-carboxylic acid and 2,2,2-trifluoro-*N*-(4-fluoro-3-piperidin-4-yl-benzyl)-acetamide hydrochloride as the starting materials. MS (ES⁺): 574, 576 & 578 ([M+H], x2 "Cl" pattern).

### E. [4-(5-Aminomethyl-2-fluoro-phenyl)-piperidin-1-yl]-[4,7-dichloro-1-(2-methoxyethyl)-1H-indol-3-yl]-methanone hydrochloride

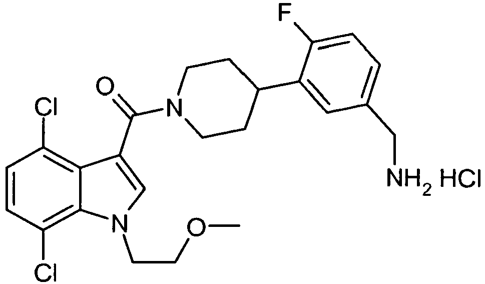

The title compound is prepared in a similar manner as Example 2G using *N*-(3-{1-[4, 7-dichloro-1-(2-methoxy-ethyl)-1*H*-indole-3-carbonyl]-piperidin-4-yl}-4-fluoro-benzyl)-2,2,2-trifluoro-acetamide as the starting material. ¹H NMR (DMSO-*d₆*): δ8.26 (bs, 3H), 7.64 (s, 1 H), 7.49 (br d, 1 H), 7.39-7.34 (m, 1 H), 7.27-7.21 (m, 2H), 7.18-7.14 (m, 1 H), 4.77-4.70 (m, 2H), 4.00 (m, 2H), 3.84 (bs, 2H), 3.70 (t, 2H), 3.22 (s, 3H), 3.13 (bs, 2H), 2.92-2.84 (m, H), 1.89-1.63 (m, 4H).

### EXAMPLE 9

### [4-(5-Aminomethyl-2-fluoro-phenyl)-piperidin-1-yl]-[7-chloro-4-fluoro-1-(2-methoxyethyl)-1H-indol-3-yl]-methanone hydrochloride

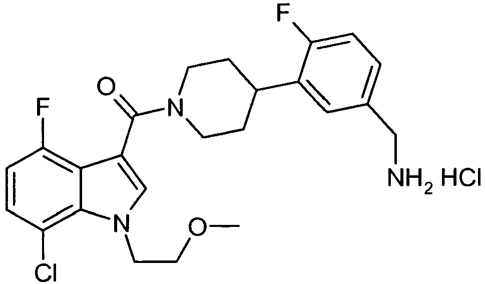

### A. 7-Chloro-4-fluoro-1H-indole-3-carboxylic acid methyl ester

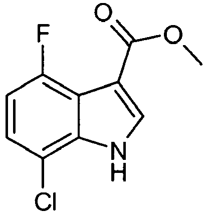

N-(2-Chloro-5-fluorophenyl)-hydroxylamine is prepared according to the procedure by Evans, D. A. et al. Org. Lett. 2006, 8, 3351-3354 using 2-chloro-5-fluoronitrobenzene as the starting material. The crude mixture was used in the next step without any purification.
The titled compound is prepared according to the procedure by Jih Ru Hwu et al. J. Org. Chem. 1994, 59, 1577-1582 using the crude N-(2-chloro-5-fluorophenyl)-hydroxylamine. ¹H NMR (CD₃OD): δ 8.02 (s, 1 H), 7.20 (dd, 1 H), 6.87 (dd, 1 H), 3.86 (s, 3H).

### B. 7-Chloro-4-fluoro-1-(2-methoxy-ethyl)-1H-indole-3-carboxylic acid methyl ester

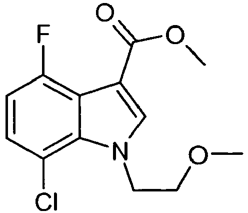

The title compound is prepared in a similar manner as Example 2C using 7-chloro-4-fluoro-1*H*-indole-3-carboxylic acid methyl ester as the starting material. ¹H NMR (CDCl₃): δ 7.74 (s, 1 H), 7.07 (dd, 1 H), 6.78 (dd, 1 H), 4.64 (t, 2H), 3.81 (s, 3H), 3.69 (t, 2H), 3.24 (s, 3H).

### C. 7-Chloro-4-fluoro-1-(2-methoxy-ethyl)-1H-indole-3-carboxylic acid

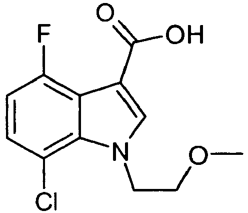

The title compound is prepared in a similar manner as Example 10C using 7-chloro-4-fluoro-1-(2-methoxy-ethyl)-1*H*-indole-3-carboxylic acid methyl ester as the starting material. ¹H NMR (CD₃OD): δ 7.62 (s, 1 H), 7.09 (dd, 1 H), 6.77-6.71 (m, 1 H), 4.71 (t, 2H), 3.74 (t, 2H), 3.28 (s, 3H).

### D. N-(3-{1-[7-Chloro-4-fluoro-1-(2-methoxy-ethyl)-1H-indole-3-carbonyl]-piperidin-4-yl}-4-fluoro-benzyl)-2,2,2-trifluoro-acetamide

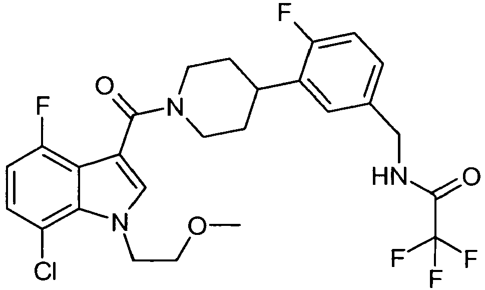

The title compound is prepared in a similar manner as Example 2F using 7-chloro-4-fluoro-1-(2-methoxy-ethyl)-1*H*-indole-3-carboxylic acid and 2,2,2-trifluoro-*N*-(4-fluoro-3-piperidin-4-yl-benzyl)-acetamide hydrochloride as the starting materials. MS (ES⁺): 558 & 560 ([M+H], x1 "Cl" pattern).

### E. [4-(5-Aminomethyl-2-fluoro-phenyl)-piperidin-1-yl]-[7-chloro-4-fluoro-1-(2-methoxy-ethyl)-1H-indol-3-yl]-methanone hydrochloride

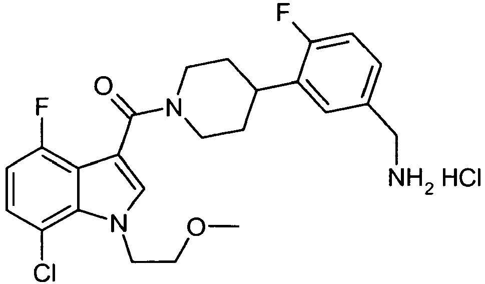

The title compound is prepared in a similar manner as Example 2G using *N*-(3-{1-[7-chloro-4-fluoro-1-(2-methoxy-ethyl)-1*H*-indole-3-carbonyl]-piperidin-4-yl}-4-fluorobenzyl)-2,2,2-trifluoro-acetamide as the starting material. ¹H NMR (DMSO-*d₆*): δ 8.26 (bs, 3H), 7.66 (s, 1 H), 7.53-7.50 (m, 1 H), 7.39-7.34 (m, 1 H), 7.26-7.22 (m, 2H), 6.93 (dd, 1 H), 4.71 (t, 2H), 4.00 (m, 2H), 3.71 (t, 2H), 3.37 (bs, 3H), 3.22 (s, 3H), 3.16-3.08 (m, 2H), 1.82-1.52 (m, 4H).

### EXAMPLE 10

### [4-(5-Aminomethyl-2-fluoro-phenyl)-piperidin-1-yl]-[7-chloro-1-(2-methoxy-ethyl)-4-trifluoromethoxy-1H-indol-3-yl]-methanone hydrochloride

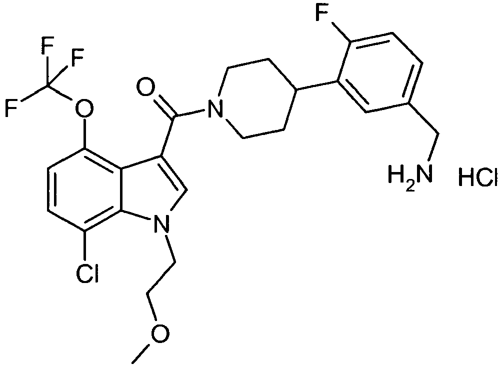

### A. 4-Chloro-2-nitro-1-trifluoromethoxy-benzene and 1-Chloro-2-nitro-4-trifluoromethoxy-benzene

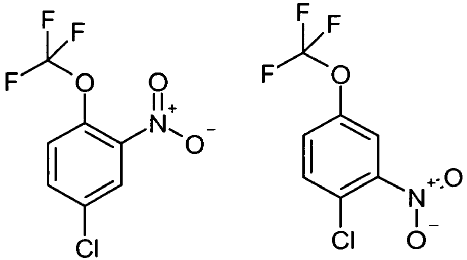

To a mixture of 1-chloro-4-trifluoromethoxy-benzene (9.24 g, 47 mol) in conc. H₂SO₄ (30 mL) at 0°C is added conc. HNO₃ (10 mL) dropwise over a 5 min period. After the mixture is stirred at 0 °C for 1 h, it was poured into ice. More H₂O is added, and the resulting mixture is extracted with EtOAc. The organic extract is washed with H₂O (3X) and brine, dried over MgSO₄, filtered, and concentrated *in vacuo* to yield 10.87 g (95%) of the product as a mixture of 1-chloro-2-nitro-4-trifluoromethoxy-benzene and 4-chloro-2-nitro-1-trifluoromethoxy-benzene (∼50/50, based upon ¹⁹F NMR). ¹H NMR (CDCl₃): δ 8.05-7.95 and 7.85-7.70 (m, 1 H), 7.70-7.55 and 7.50-7.35 (m, 2H); ¹⁹F NMR (CDCl₃) δ -57.32 and -57.84 (s, 3F), -109.07 and -117.90 (t, *J* = 6.2Hz and d, *J* = 6.2 Hz, 1 F).

### B. 7-Chloro-4-trifluoromethoxy-1H-indole

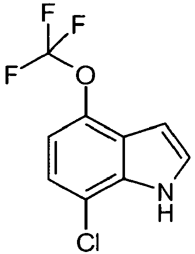

To a mixture of 4-chloro-2-nitro-1-trifluoromethoxy-benzene and 1-chloro-2-nitro-4-trifluoromethoxy-benzene (10.0 g, 41.4 mmol) in THF (100 mL) at -78 °C is added vinylmagnesium bromide (177 mL, 0.7 M in THF, 124 mmol) slowly over a 10 min period. After this mixture is stirred at -78 °C for 1 h, sat. NH₄Cl is added, and the cooling bath is removed. The reaction mixture is partitioned between H₂O and EtOAc, and the two layers are separated. The organic layer is washed with H₂O and brine, dried over MgSO₄, filtered, and concentrated *in vacuo.* The crude material is purified on silica gel with heptane/EtOAc (100/0 to 70/30) as eluant to give 1.34 g (13%) of the product as a brown oil. ¹H NMR (CDCl₃): δ 8.48 (br, s, 1 H), 7.35-7.20 (m, 1 H), 7.20-7.10 (m, 1 H), 7.00-6.85 (m, 1 H), 6.70-6.65 (m, 1 H); ¹⁹F NMR (CDCl₃) δ -57.41 (s, 3F); MS 235 (M+, 100%).

### C. 7-Chloro-1-(2-methoxy-ethyl)-4-trifluoromethoxy-1H-indole

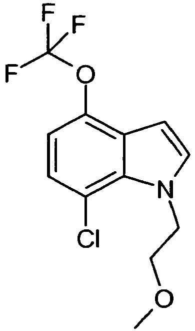

A mixture of 7-chloro-4-trifluoromethoxy-1*H*-indole (1.34 g, 5.69 mmol), powder KOH (1.59 g, 28.4 mmol) in DMSO (10 mL) is stirred at rt for 5 min. 2-Methoxyethyl bromide (803 µL, 8.53 mmol) is added. After the reaction mixture was stirred at rt overnight, it is partitioned between H₂O and Et₂O. The two layers are separated, and the aqueous layer is extracted with Et₂O (3X). The combined organic extracts are washed with H₂O and brine, dried over MgSO₄, filtered, and concentrated *in vacuo.* The crude material is purified on silica gel with heptane/EtOAc (100/0 to 70/30) as eluant to yield 1.16 g (69%) of the product as a clear yellow liquid. ¹H NMR (CDCl₃): δ 7.20-7.05 (m, 2H), 6.95-6.85 (m, 1 H), 6.60-6.55 (m, 1 H), 4.45 (t, *J* = 5.2 Hz, 2H), 3.69 (t, *J* = 5.3 Hz, 2H), 3.29 (s, 3H); ¹⁹F NMR (CDCl₃) δ -57.33 (s, 3F); MS 248 (100%), 293 (M+, 100%)

### D. 1-[7-Chloro-1-(2-methoxy-ethyl)-4-trifluoromethoxy-1H-indol-3-yl]-2,2,2-trifluoroethanone

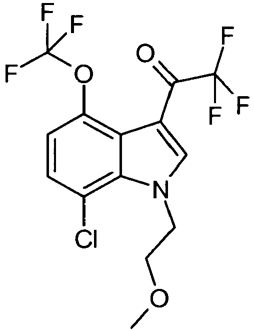

A mixture of 7-chloro-1-(2-methoxy-ethyl)-4-trifluoromethoxy-1*H*-indole (1.16 g, 3.95 mmol) and TFAA (1.65 mL, 11.8 mmol) in DMF (20 mL) is heated at 45 °C overnight. TLC indicates ∼90% conversion. More TFAA (∼0.4 mL) is added, and the reaction mixture is stirred at rt for another 4 h. The mixture is then partitioned between H₂O and Et₂O. The two layers are separated, and the organic layer is washed with H₂O and brine, dried over MgSO₄, filtered, and concentrated *in vacuo.* The crude material is purified on silica gel with hepatane/EtOAc (95/5 to 50/50) as eluant to yield 1.13 g (73%) of the product as a green viscous oil. ¹H NMR (CDCl₃): δ 8.05-8.00 (m, 1 H), 7.34 (d, *J* = 8.6 Hz, 1 H), 7.20-7.10 (m, 1 H), 4.81 (t, *J* = 4.8 Hz, 2H), 3.79 (t, *J* = 4.8 Hz, 2H), 3.33 (s, 3H); ¹⁹F NMR (CDCl₃) δ -58.33 (s, 3F), -71.94 (s, 3F); MS 390 (M+1, 100%).

### E. 7-Chloro-1-(2-methoxy-ethyl)-4-trifluoromethoxy-1H-indole-3-carboxylic acid

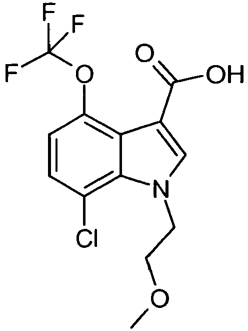

A mixture of 1-[7-chloro-1-(2-methoxy-ethyl)-4-trifluoromethoxy-1*H*-indol-3-yl]-2,2,2-trifluoro-ethanone (106 mg, 0.27 mmol) in MeOH (10 mL) and NaOH (5 M, 5 mL) is heated at 80 °C overnight. This mixture is concentrated *in vacuo* to remove the methanol. The residue is diluted with H₂O, and then washed with Et₂O once. The aqueous layer is acidified to pH 2 with HCl (3 M). The acidified mixture is extracted with Et₂O (2X). The combined organic extracts are washed with H₂O and brine, dried over MgSO₄, filtered, and concentrated in vacuo to yield 59 mg (64%) of the product as a beige powder. ¹H NMR (CDCl₃): δ 8.02 (s, 1 H), 7.23 (s, 1 H), 7.15-7.05 (m, 1 H), 4.76 (t, J = 5.1 Hz, 2H), 3.78 (t, J = 5.2 Hz, 2H), 3.33 (s, 3H); ¹⁹F NMR (CDCl₃): δ -57.48 (s, 3F); MS 379 (M+CH₃CN+1), 338 (M+1, 100%).

### F. N-(3-{1-[7-Chloro-1-(2-methoxy-ethyl)-4-trifluoromethoxy-1H-indole-3-carbonyl]-piperidin-4-yl}-4-fluoro-benzyl)-2,2,2-trifluoro-acetamide

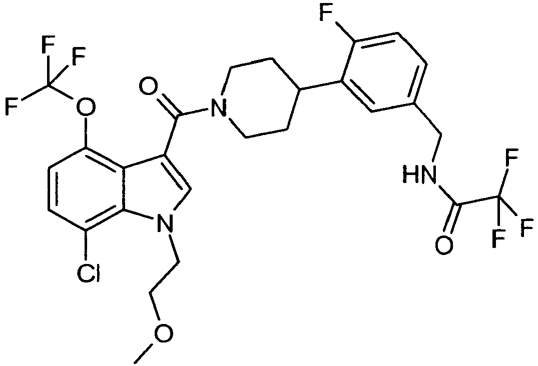

A mixture of 7-chloro-1-(2-methoxy-ethyl)-4-trifluoromethoxy-1*H*-indole-3-carboxylic acid (59 mg, 0.18 mmol), Et₃N (73 µL, 0.55 mmol), 2,2,2-trifluoro-*N*-(4-fluoro-3-piperidin-4-yl-benzyl)-acetamide hydrochloride (77 mg, 0.23 mmol), and EDCI (50 mg, 0.26 mmol) in CH₂Cl₂ (10 mL) is stirred at rt overnight. The mixture is partitioned between H₂O and CH₂Cl₂. The two layers are separated, and the organic layer is washed with brine, dried over MgSO₄, filtered, and concentrated *in vacuo.* The crude material is purified on silica gel with heptane/EtOAc (50/50 to 0/100) as eluant to give 60 mg (54%) of the product as a white powder. ¹H NMR (CDCl₃): δ 7.33 (s, 1 H), 7.25-7.10 (m, 4H), 7.10-6.90 (m, 2H), 4.95 (br s, 1 H), 4.70 (t, *J* = 5.4 Hz, 2H), 4.46 (d, *J*= 5.4 Hz, 2H), 3.95-3.60 (m, 3H), 3.31 (s, 3H), 3.25-3.00 (m, 2H), 3.00-2.75 (m, 1 H), 2.05-1.50(m, 4H); ¹⁹F NMR (CDCl₃) δ -57.19 (s, 3F), -75.39 (s, 3F), -119.31 (s, 1 F); MS 624 (M+1, 100%).

### G-[4-(5-Aminomethyl-2-fluoro-phenyl)-piperidin-1-yl]-[7-chloro-1-(2-methoxy-ethyl)-4-trifluoromethoxy-1H-indol-3-yl]-methanone hydrochloride

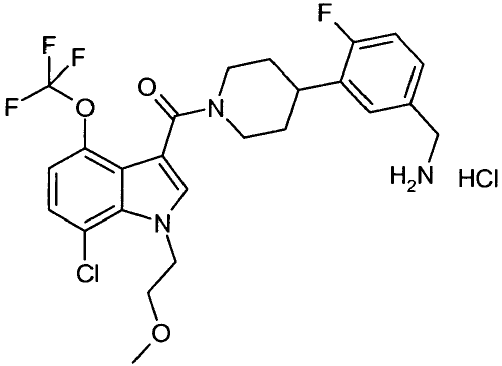

A mixture of *N*-(3-{1-[7-chloro-1-(2-methoxy-ethyl)-4-trifluoromethoxy-1*H*-indole-3-carbonyl]-piperidin-4-yl}-4-fluoro-benzyl)-2,2,2-trifluoro-acetamide (60 mg, 0.096 mmol) in MeOH (10 mL) is added aqueous K₂CO₃ (106 mg, 0.77 mmol, dissolved in 3 mL H₂O). This mixture is stirred at rt overnight. LC/MS indicates the reaction is completed. The reaction mixture is concentrated *in vacuo* to remove most of the methanol. The residue is partitioned between H₂O and EtOAc. The two layers are separated, and the organic layer is washed with H₂O and brine, dried over MgSO₄, filtered, and concentrated *in vacuo.* The residue is dissolved in Et₂O, and HCl in Et₂O (1.0 M, 2 mL) is added. The resulting suspension is concentrated *in vacuo,* and then dried *in vacuo* to yield 37 mg (68%) of the product as a beige solid. ¹H NMR (DMSO-d₆): δ 8.26 (br,s 3H), 7.71 (s, 1 H), 7.55-7.45 (m, 1 H), 7.45-7.30 (m, 2H), 7.30-7.1.5 (m, 1 H), 7.15-7.05 (m, 1 H), 4.90-4.55 (m, 3H), 3.99 (d, *J* = 5.5 Hz, 2H), 3.90-3.60 (m, 3H), 3.23 (s, 3H), 3.20-2.70 (m, 3H), 1.95-1.45(m, 4H); ¹⁹F NMR (DMSO-*d₆*): δ -56.31 (s, 3F), -119.17 (s, 1 F); MS 528 (M+1, 100%).

### EXAMPLE 11

### [4-(5-Aminomethyl-2-fluoro-phenyl)-piperidin-1-yl]-[1-propyl-7-fluoro-4-trifluoromethoxy-1H-indol-3-yl)-methanone hydrochloride

### A. 1-Propyl-7-fluoro-4-trifluoromethoxy-1H-indole

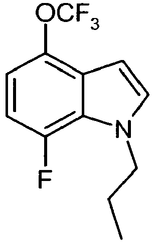

The title product (0.48 g, 36%) is obtained in a similar manner as described in Example 1D except using 7-fluoro-4-trifloromethoxy-1*H*-indole (1.118 g, 5.10 mmol) as the starting material and *n*-propyl bromide as the alkylating agent. ¹H NMR (300 MHz, CDCl₃): δ 7.26 (s, 1 H), 7.07 (d, 1 H), 6.84 (d, 1 H), 6.55 (d, 1 H), 4.23 (t, 2H), 1.86 (m, 2H), 0.923 (t, 3H); ¹⁹F NMR (CDCl₃) δ -57.69 (s, 3F), -136.80 (d, *J* = 12.9 Hz, 1 F).

### B: 1-(1-Propyl-7-fluoro-4-trifluoromethoxy-1H-indol-3-yl)-2,2,2-trifluoro-ethanone.

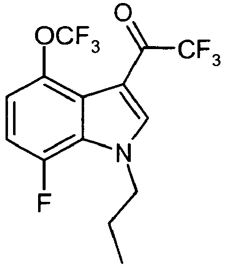

The title product (0.400 g, 57%) is obtained in a similar manner as described in Example 1E except using 1-propyl-7-fluoro-4-trifluoromethoxy-1*H*-indole (0.512 g, 1.96 mmol) as the starting material. ¹H NMR (300 MHz, CDCl₃): δ 7.9 (s, 1H), 7.1-7.2 (m, 1 H), 7.1-7.0, 1 H), 4.28 (t, 2H), 1.98 (m, 2H), 1.0 (t, 3H); ¹⁹F NMR (CDCl₃) δ -58 (s, 3F), -71 (s, 3F), -134 (s, 1 F).

### C: 1-Propyl-7-fluoro-4-trifluoromethoxy-1H-indole-3-carboxylic acid.

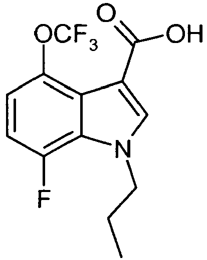

A mixture of 1-(1-propyl-7-fluoro-4-trifluoromethoxy-1*H*-indol-3-yl)-2,2,2-trifluoroethanone (0.787 g, 2.20 mmol) in 6.25 N NaOH (15 mL) is heated at 100 °C for 2 hr then cooled to rt over 3 d. The reaction mixture is cooled to rt, and then diluted with H₂O and CH₂Cl₂. The aqueous layer is washed with CH₂Cl₂ then slowly acidified to pH ∼3 with conc. HCl. The solid precipitate is collected by suction filtration and airdried to give the title product (33%). ¹H NMR (CDCl₃): δ 12.13 (s, 1H), 8.23 (s, 1H), 7.16-7.12 (m, 2H), 4.31 (t, *J* = 7.5 Hz, 2H), 1.80 (m, 2H), 0.84 (t, *J* = 7.5 Hz, 3H); ¹⁹F NMR (CDCl₃): δ -56.74 (s, 3F), -134.77 (s, 1 F).

### D: N-{3-[1-(1-Propyl-7-fluoro-4-trifluoromethoxy-1H-indole-3-carbonyl)-piperidin-4-yl]-4-fluoro-benzyl}-2,2,2-trifluoro-acetamide.

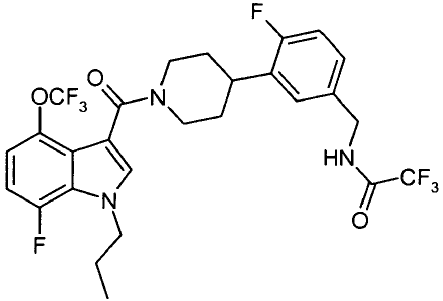

The title product (0.360 g, 86%) is obtained in a similar manner as described in Example 1G except using 1-propyl-7-fluoro-4-trifluoromethoxy-1 H-indole-3-carboxylic acid (0.215 g, 0.70 mmol) as the starting material. ¹H NMR (CDCl₃): δ 7.31 (s, 1 H), 7.13-7.11 (m, 2H), 7.04 (d, *J* = 9.6 Hz, 1 H), 6.93-6.87 (m, 2H), 6.60 (bs, 1 H), 4.49 (d, J = 6 Hz, 2H), 4.24 (t, *J* = 7.2 Hz, 2H), 4.95 (br s, 1 H), 3.90 (br s, 1 H), 3.10 (m), 1.95 (m, 2H), 0.96 (t, *J* = 7.5 Hz, 3H); ¹⁹F NMR (CDCl₃): δ -57.71 (s, 3F), -75.54 (s, 3F), - 119.20 (s, 1 F), -135.90 (d, *J* = 12.9 Hz, 1 F).

### E: [4-(5-Aminomethyl-2-fluoro-phenyl)-piperidin-1-yl]-(1-propyl-7-fluoro-4-trifluoromethoxy-1H-indol-3-yl)-methanone hydrochloride.

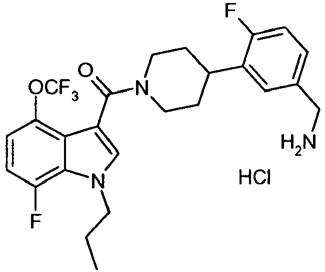

The title compound is prepared in a similar manner as Example 1 H using *N*-{3-[1-(1-propyl-7-fluoro-4-trifluoromethoxy-1*H*-indole-3-carbonyl)-piperidin-4-yl]-4-fluoro-benzyl}-2,2,2-trifluoro-acetamide as the starting material. ¹H NMR (DMSO-*d₆*): δ 8.32 (br s, 2H), 7.80 (s, 1 H), 7.48 (d, *J* = 5.4 Hz, 1 H), 7.39-7.38 (m, 1 H), 7.25-7.22 (t, 1 H), 7.21-7.7.06 (m, 2H), 4.29 (t, *J* = 6.9Hz, 2H), 3.99 (d, 2H), 3.11-3.07 (m, 2H), 3.06-2.80 (bs, 1 H), 1.85-1.70 (m, 2H), 1.69-1.61 (m, 4H), 0.85 (t, *J* = 7.5Hz, 3H); ¹⁹F NMR (DMSO-*d₆*): δ -56.75 (s, 3F), -119.35 (s, 1 F), -135.15 (d, *J* =12.9 Hz, 1 F). MS 496 (M+1, 100%).

### EXAMPLE 12

### [4-(5-Aminomethyl-2-fluoro-phenyl)-piperidin-1-yl]-[4-chloro-1-(2-methoxy-ethyl)-7-trifluoromethoxy-1H-indol-3-yl]-methanone hydrochloride

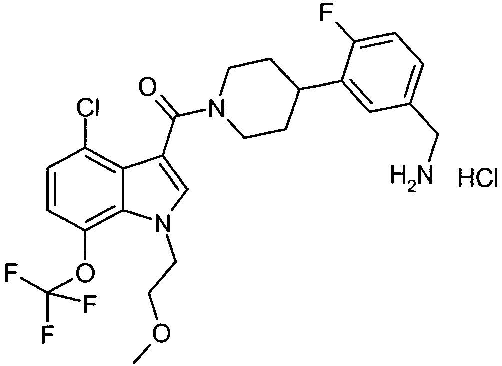

### A. 4-Chloro-7-trifluoromethoxy-1H-indole

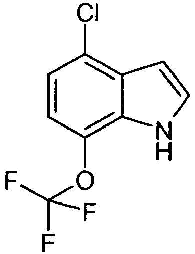

To a mixture of 4-chloro-2-nitro-1-trifluoromethoxy-benzene and 1-chloro-2-nitro-4-trifluoromethoxy-benzene (10.0 g, 41.4 mmol, example 10-A) in THF (100 mL) at -78 °C is added vinylmagnesium bromide (177 mL, 0.7 M in THF, 124 mmol) slowly over a 10 min period. After this mixture is stirred at -78 °C for 1 h, sat. NH₄Cl is added, and the cooling bath is removed. The reaction mixture is partitioned between H₂O and EtOAc, and the two layers are separated. The organic layer is washed with H₂O and brine, dried over MgSO₄, filtered, and concentrated *in vacuo.* The crude material is purified on silica gel with heptane/EtOAc (100/0 to 70/30) as eluant to give 1.51 g (15%) of the product as a brown oil. ¹H NMR (CDCl₃): δ 8.48 (br, s, 1 H), 7.35-7.20 (m, 1 H), 7.15-6.95 (m, 2H), 6.75-6.65 (m, 1 H); ¹⁹F NMR (CDCl₃): δ -57.51 (s, 3F); MS 235 (M+, 100%).

### B. 4-Chloro-1-(2-methoxy-ethyl)-7-trifluoromethoxy-1H-indole

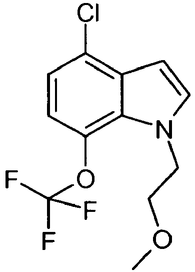

A mixture of 4-chloro-7-trifluoromethoxy-1*H*-indole (1.51 g, 6.41 mmol), powder KOH (1.80 g, 32.0 mmol) in DMSO (15 mL) is stirred at rt for 5 min. 2-Methoxyethyl bromide (1.2 mL, 12.8 mmol) is added. After the reaction mixture was stirred at rt overnight, it is partitioned between H₂O and Et₂O. The two layers are separated, and the aqueous layer is extracted with Et₂O (3X). The combined organic extracts are washed with H₂O and brine, dried over MgSO₄, filtered, and concentrated *in vacuo.* The crude material is purified on silica gel with heptane/EtOAc (100/0 to 70/30) as eluant to yield 1.11 g (58%) of the product as a clear yellow oil. ¹H NMR (CDCl₃): δ 7.20 (d, *J* = 3.2 Hz, 1 H), 7.10-6.90 (m, 2 H), 6.61 (d, *J* = 3.1 Hz, 1 H), 4.45 (t, *J* = 5.2 Hz, 2H), 3.69 (t, *J* = 5.2 Hz, 2H), 3.29 (s, 3H); ¹⁹F NMR (CDCl₃): δ -56.58 (s, 3F); MS 248 (100%), 293 (M+).

### C. 1-[4-Chloro-1-(2-methoxy-ethyl)-7-trifluoromethoxy-1H-indol-3-yl]-2,2,2-trifluoroethanone

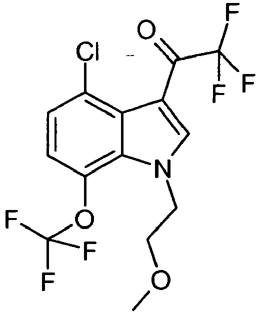

A mixture of 4-chloro-1-(2-methoxy-ethyl)-7-trifluoromethoxy-1*H*-indole (1.11 g, 3.78 mmol) and TFAA (1.42 mL, 0.2 mmol) in DMF (15 mL) is heated at 40 °C overnight. The reaction mixture is cooled to rt and stirred at rt for overnight. More TFAA (400 µL) is added, and the reaction mixture is heated at 60 °C for 2 h. The mixture is then partitioned between H₂O and Et₂O. The two layers are separated, and the organic layer is washed with H₂O and brine, dried over MgSO₄, filtered, and concentrated *in vacuo.* The crude material is purified on silica gel with hepatane/EtOAc (100/0 to 70/30) as eluant to yield 0.89 g (61 %) of the product as a green oil. ¹H NMR (CDCl₃): δ 8.03 (s, 1 H), 7.35-7.20 (m, 1 H), 7.20-7.10 (m, 1 H), 4.55 (t, *J* = 4.8 Hz, 2H), 3.72 (t, *J* = 4.9 Hz, 2H), 3.31 (s, 3H); ¹⁹F NMR (CDCl₃): δ -56.56 (s, 3F), -71.42 (s, 3F); MS 390 (M+1, 100%).

### D. 4-Chloro-1-(2-methoxy-ethyl)-7-trifluoromethoxy-1H-indole-3-carboxylic acid

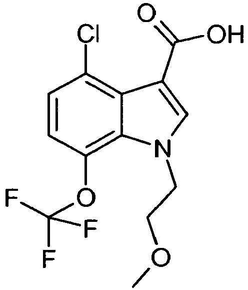

A mixture of 1-[4-chloro-1-(2-methoxy-ethyl)-7-trifluoromethoxy-1*H*-indol-3-yl]-2,2,2-trifluoro-ethanone (890 mg, 2.28 mmol) in MeOH (20 mL) and NaOH (5 M, 10 mL) is heated at 80 °C for 5 h. The reaction mixture is cooled to rt and stirred at this temperature overnight. This mixture is concentrated *in vacuo* to remove the methanol. The residue is diluted with H₂O, and then washed with Et₂O once. The organic wash is extracted with Et₂O (2X). The combined aqueous layers are acidified to pH 1 with HCl (3 M). The acidified mixture is extracted with Et₂O (2X). The combined organic extracts are washed with H₂O and brine, dried over MgSO₄, filtered, and concentrated in vacuo to yield 620 mg (80%) of the product as a beige powder. ¹H NMR (CDCl₃): δ 8.02 (s, 1 H), 7..35-7.15 (m, 1 H), 7.10-7.05 (m, 1 H), 4.50 (t, *J* = 4.9 Hz, 2H), 3.71 (t, *J* = 5.1 Hz, 2H), 3.31 (s, 3H); ¹⁹F NMR (CDCl₃): δ -56.52 (s, 3F); MS 379 (M+CH₃CN+1), 338 (M+1, 100%).

### E. N-(3-{1-[4-Chloro-1-(2-methoxy-ethyl)-7-trifluoromethoxy-1H-indole-3-carbonyl]-piperidin-4-yl}-4-fluoro-benzyl)-2,2,2-trifluoro-acetamide

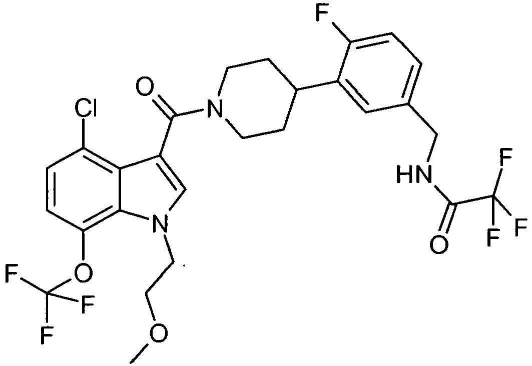

A mixture of 4-chloro-1-(2-methoxy-ethyl)-7-trifluoromethoxy-1*H*-indole-3-carboxylic acid (260 mg, 0.77 mmol), Et₃N (322 µL, 2.31 mmol), 2,2,2-trifluoro-*N*-(4-fluoro-3-piperidin-4-yl-benzyl)-acetamide hydrochloride (341 mg, 1.0 mmol), and EDCI (221 mg, 1.15 mmol) in CH₂Cl₂ (20 mL) is stirred at rt overnight. The mixture is partitioned between H₂O and CH₂Cl₂. The two layers are separated, and the organic layer is washed with brine, dried over MgSO₄, filtered, and concentrated *in vacuo.* The crude material is purified on silica gel with heptane/EtOAc (50/50 to 0/100) as eluant to give 280 mg (58%) of the product as a white powder. ¹H NMR (CDCl₃): δ 7.33 (m, 1 H), 7.20-6.90 (m, 5H), 6.74 (br s, 1 H), 5.00 (m, 1 H), 4.60-4.35 (m, 4H), 3.90-3.55 (m, 3H), 3.30 (s, 3H), 3.25-3.00 (m, 2H), 3.00-2.75 (m, 1H), 2.10-1.55 (m, 4H); ¹⁹F NMR (CDCl₃): δ -56.58 (s, 3F), -75.32 (s, 3F), -118.91 (s, 1 F); MS 624 (M+1, 100%).

### F. [4-(5-Aminomethyl-2-fluoro-phenyl)-piperidin-1-yl]-[4-chloro-1-(2-methoxy-ethyl)-7-trifluoromethoxy-1H-indol-3-yl]-methanone hydrochloride

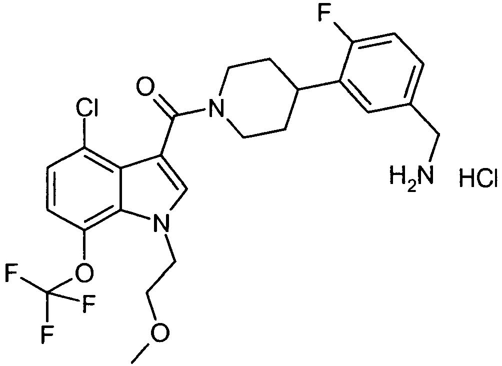

A mixture of *N*-(3-{1-[4-chloro-1-(2-methoxy-ethyl)-7-trifluoromethoxy-1*H*-indole-3-carbonyl]-piperidin-4-yl}-4-fluoro-benzyl)-2,2,2-trifluoro-acetamide (270 mg, 0.43 mmol) in MeOH (10 mL) is added aqueous K₂CO₃ (475 mg, 3.44 mmol, dissolved in 3 mL H₂O). This mixture is stirred at rt overnight. LC/MS indicates the reaction is completed. The reaction mixture is concentrated *in vacuo* to remove most of the methanol. The residue is partitioned between H₂O and EtOAc. The two layers are separated, and the organic layer is washed with H₂O and brine, dried over MgSO₄, filtered, and concentrated *in vacuo.* The residue is dissolved in Et₂O, and HCl in Et₂O (1.0 M, 3 mL) is added. The resulting suspension is concentrated *in vacuo,* and then dried *in vacuo to* yield 126 mg (51%) of the product as a white solid. ¹H NMR (DMSO-*d₆*): δ 8.36 (br,s 3H), 7.71 (s, 1 H), 7.60-7.45 (m, 1 H), 7.45-7.30 (m, 1 H), 7.35-7.10 (m, 3H), 4.75 (br d, J = 13.1, 1 H), 4.48 (t, J = 5.0, 2H), 4.10-3.90 (m, 2H), 3.75-3.50 (m, 3H), 3.21 (s, 3H), 3.20-3.05 (m, 2H), 3.00-2.80 (m, 1H), 1.95-1.45(m, 4H); ¹⁹F NMR (DMSO-*d₆*): δ -55.73 (s, 3F), -119.37 (s, 1 F); MS 528 (M+1, 100%).

### EXAMPLE 13

### [4-(5-Aminomethyl-2-fluoro-phenyl)-piperidin-1-yl]-[7-fluoro-1-(2-methoxy-ethyl)-4-trifluoromethyl-1H-indol-3-yl]-methanone hydrochloride

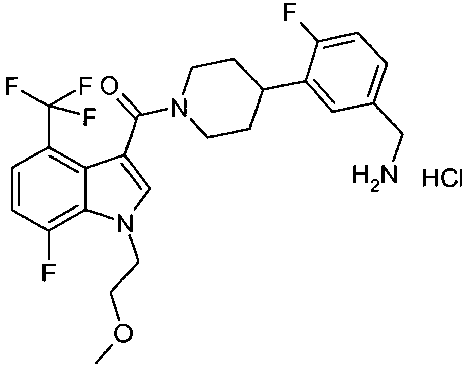

### A. 7-Fluoro-4-trifluoromethyl-1H-indole

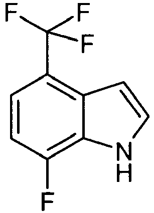

To a mixture of 1-fluoro-2-nitro-4-trifluoromethyl-benzene (3.27 mL, 23.3 mmol) in THF (100 mL) at -78 °C is added vinylmagnesium bromide (100 mL, 0.7 M in THF, 70 mmol) slowly over a 30 min period. After this mixture is stirred at -78 °C for 30 min, sat. NH₄Cl is added, and the cooling bath is removed. The reaction mixture is partitioned between H₂O and EtOAc, and the two layers are separated. The organic layer is washed with H₂O and brine, dried over MgSO₄, filtered, and concentrated *in vacuo.* The crude material is purified on silica gel with heptane/EtOAc (100/0 to 50/50) as eluant to give 0.93 g (19%) of the product as a brown oil. ¹H NMR (CDCl₃): δ 8.53 (br, s, 1 H), 7.50-7.15 (m, 2 H), 7.00-6.85 (m, 1 H), 6.85-6.70 (m, 1 H); ¹⁹F NMR (CDCl₃): δ -60.56 (s, 3F), -129.63 (d, *J* = 11.3 Hz, 1 F); MS 203 (M+, 100%).

### B. 7-Fluoro-1-(2-methoxy-ethyl)-4-trifluoromethyl-1H-indole

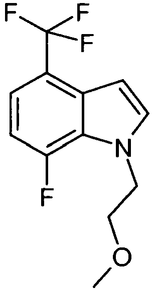

To a mixture of 7-fluoro-4-trifluoromethyl-1*H*-indole (0.91 g, 4.48 mmol), powder KOH (1.26 g, 22.4 mmol) in DMSO (15 mL) is added 2-methoxyethyl bromide (0.63 mL, 6.72 mmol). After the reaction mixture was stirred at rt overnight, it is partitioned between H₂O and Et₂O. The two layers are separated, and the aqueous layer is extracted with Et₂O. The organic extract is washed with H₂O and brine, dried over MgSO₄, filtered, and concentrated *in vacuo.* The crude material is purified on silica gel with heptane/EtOAc (100/0 to 50/50) as eluant to yield 0.66 g (56%) of the product as a clear yellow oil. ¹H NMR (CDCl₃): δ 7.40-7.20 (m, 2H), 6.95-6.85 (m, 1 H), 6.66 (br s, 1 H), 4.48 (t, *J* = 5.2 Hz, 2H), 3.72 (t, *J* = 5.1 Hz, 2H), 3.30 (s, 3H); ¹⁹F NMR (EDCl₃): δ -60.64 (s, 3F), -130.33 (d, *J* =11.3 Hz, 1 F); MS 262 (M+1, 100%)

### C. 2,2,2-Trifluoro-1-[7-fluoro-1-(2-methoxy-ethyl)-4-trifluoromethyl-1H-indol-3-yl]-ethanone

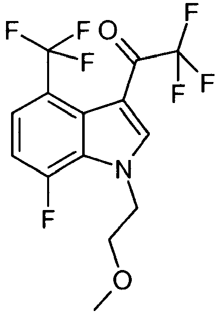

A mixture of 7-fluoro-1-(2-methoxy-ethyl)-4-trifluoromethyl-1 *H*-indole (660 mg, 2.53 mmol) and TFAA (1.05 mL, 7.51 mmol) in DMF (15 mL) is heated at 45 °C for 3 days. The mixture is partitioned between H₂O and Et₂O. The two layers are separated, and the organic layer is washed with H₂O and brine, dried over MgSO₄, filtered, and concentrated *in vacuo.* The crude material is purified on silica gel with hepatane/EtOAc (100/0 to 70/30) as eluant to yield 0.30 g (33%) of the product as a light green powder. ¹H NMR (CDCl₃): δ 8.07 (d, *J* = 1.7 Hz, 1 H), 7.68 (dd, *J* = 4.6, 8.5 Hz, 1 H), 7.13 (dd, *J* = 8.8, 11.4 Hz, 1 H), 4.58 (t, *J* = 4.6 Hz, 2H), 3.76 (t, *J* = 4.3 Hz, 2H), 3.32 (s, 3H); ¹⁹F NMR (CDCl₃): δ -58.68 (s, 3F), -72.32 (s, 3F), -129.24 (d, *J* = 14.1 Hz, 1 F); MS 338 (M-19), 358 (M+1, 100%).

### D. 7-Fluoro-1-(2-methoxy-ethyl)-4-trifluoromethyl-1H-indole-3-carboxylic acid

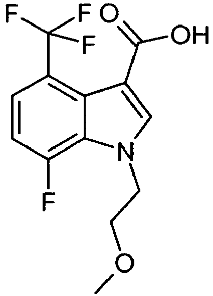

A mixture of 2,2,2-trifluoro-1-[7-fluoro-1-(2-methoxy-ethyl)-4-trifluoromethyl-1*H*-indol-3-yl]-ethanone (285 mg, 0.8 mmol) in MeOH (15 mL) and NaOH (5 M, 5 mL) is heated at 90 °C overnight. This mixture is concentrated *in vacuo* to remove the methanol. The residue is diluted with H₂O, and then washed with EtOAc once. The aqueous layer is acidified to pH 2 with HCl (3 M). The acidified mixture is extracted with Et₂O (2X). The combined organic extracts are washed with H₂O and brine, dried over MgSO₄, filtered, and concentrated in vacuo to yield 170 mg of the crude product (contained 7-methoxy-1-(2-methoxy-ethyl)-4-trifluoromethyl-1*H*-indole-3-carboxylic acid as side product) as a beige powder. This material is used in the next step without further purification.

### E. 2,2,2-Trifluoro-N-(4-fluoro-3-{1-[7-fluoro-1-(2-methoxy-methyl)-4-trifluoromethyl-1H-indole-3-carbonyl]-piperidin-4-yl}-benzyl)-acetamide

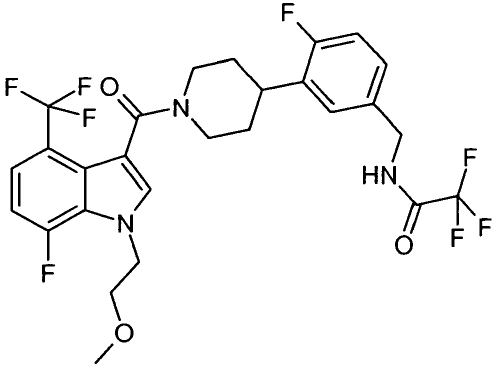

A mixture of 7-fluoro-1-(2-methoxy-ethyl)-4-trifluoromethyl-1 *H*-indole-3-carboxylic acid (170 mg, 0.56 mmol), Et₃N (234 µL, 1.68 mmol), 2,2,2-trifluoro-*N*-(4-fluoro-3-piperidin-4-yl-benzyl)-acetamide hydrochloride (248 mg, 0.73 mmol), and EDCI (160 mg, 0.83 mmol) in CH₂Cl₂ (10 mL) is stirred at rt overnight. The mixture is partitioned between H₂O and EtOAc. The two layers are separated, and the organic layer is washed with brine, dried over MgSO₄, filtered, and concentrated *in vacuo.* The crude material is purified on silica gel with heptane/EtOAc (25/75 to 0/100) as eluant to give 93 mg (28%) of the product as a white powder. ¹H NMR (CDCl₃): δ 7.50-7.35 (m, 1 H), 7.35-7.25 (m, 1 H), 7.15-7.05 (m, 2H), 7.05-6.90 (m, 2H), 6.65 (br s, 1 H), 5.10-4.90 (m, 1 H), 4.60-4.40 (m, 4H), 4.05-3.80 (m, 3H), 3.30 (s, 3H), 3.20-3.00 (m, 2H), 3.00-2.75 (m, 1 H), 2.10-1.50 (m, 4H); ¹⁹F NMR (CDCl₃): δ -58.00 and -59.17 (total 3F), -75.36 (s, 3F), -118.5 and -119.31 (total 1 F), -129.30 (s, 1 F); MS 592 (M+1, 100%).

### F. [4-(5-Aminomethyl-2-fluoro-phenyl)-piperidin-1-yl]-[7-fluoro-1-(2-methoxy-ethyl)-4-trifluoromethyl-1H-indol-3-yl]-methanone hydrochloride

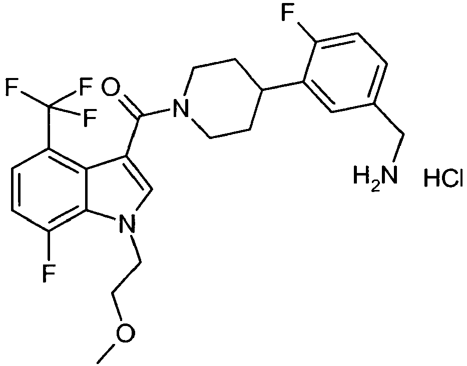

A mixture of 2,2,2-trifluoro-*N*-(4-fluoro-3-{1-[7-fluoro-1-(2-methoxy-ethyl)-4-trifluoromethyl-1*H*-indole-3-carbonyl]-piperidin-4-yl}-benzyl)-acetamide (85 mg, 0.14 mmol) in MeOH (3 mL) is added aqueous K₂CO₃ (158 mg, dissolved in 1.5 mL H₂O). This mixture is stirred at rt for 2 days. The reaction mixture is concentrated *in vacuo* to remove most of the methanol. The residue is partitioned between H₂O and EtOAc. The two layers are separated, and the organic layer is washed with H₂O and brine, dried over MgSO₄, filtered, and concentrated *in vacuo.* The residue is dissolved in Et₂O, and HCl in Et₂O (1.0 M, 2 mL) is added. The resulting suspension is concentrated *in vacuo,* and then dried *in vacuo* to yield 70 mg (92%) of the product as a white solid. ¹H NMR (DMSO-*d₆*): δ 8.33 (br,s 3H), 7.78 (s, 1H), 7.60-7.45 (m, 2H), 7.45-7.30 (m, 1 H), 7.30-7.15 (m, 2H), 4.80-4.65 (m, 1 H), 4.60-4.45 (m, 2H), 4.10-3.95 (m, 2H), 3.75-3.65 (m, 3H), 3.21 (s, 3H), 3.15-3.05 (m, 2H), 2.95-2.75 (m, 1 H), 1.95-1.75 (m, 1 H), 1.75-1.55 (m, 3H); ¹⁹F NMR (DMSO-*d₆*): δ -55.33 (s, 3F), -119.29 (s, 1F), -127.56 (d, *J* = 11.3 Hz, 1 F); MS 496 (M+1, 100%).

### EXAMPLE 14

### [4-(5-Aminomethyl-2-fluoro-phenyl)-piperidin-1-yl]-[7-methoxy-1-(2-methoxy-ethyl)-4-trifluoromethyl-1H-indol-3-yl]-methanone hydrochloride

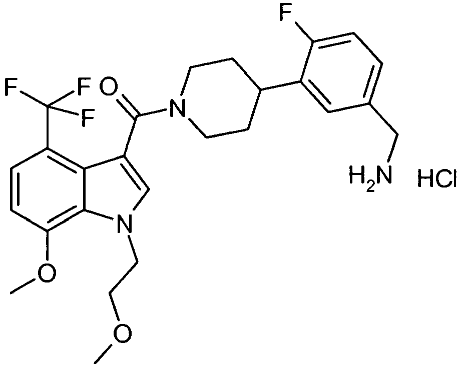

### A. 2,2,2-Trifluoro-N-(4-fluoro-3-{1-[7-methoxy-1-(2-methoxy-ethyl)-4-trifluoromethyl-1H-indole-3-carbonyl]-piperidin-4-yl)-benzyl)-acetamide

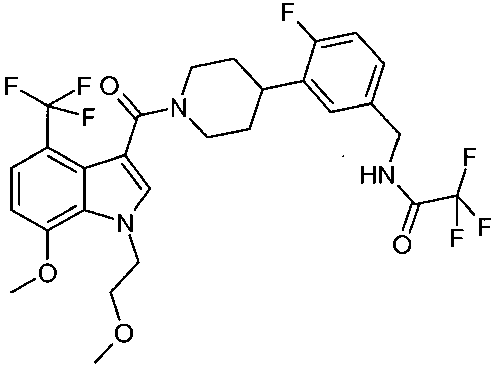

The title compound is isolated as a side product from the preparation of example13E. ¹H NMR (CDCl₃): δ 7..42 (d, *J* = 8.2 Hz, 1 H), 7.25-7.15 (m, 1 H), 7.15-7.05 (m, 2H), 7.05-6.90 (m, 1 H), 6.68 (br d, *J* = 8.3 Hz, 2H), 5.10-4.85 (m, 1 H), 4.65-4.50 (m, 2H), 4.50-4.35 (m, 2H), 3.99 (s, 3H), 3.80-3.70 (m, 1 H), 3.68 (t, *J* = 5.3 Hz, 2H), 3.28 (s, 3H), 3.20-2.95 (m, 2H), 2.95-2.75 (m, 1 H), 2.05-1.50 (m, 4H); ¹⁹F NMR (CDCl₃): δ -57.58 and -58.83 (total 3F), -75.36 (s, 3F); MS 604 (M+1, 100%).

### B. [4-(5-Aminomethyl-2-fluoro-phenyl)-piperidin-1-yl]-[7-methoxy-1-(2-methoxyethyl)-4-trifluoromethyl-1H-indol-3-yl]-methanone hydrochloride

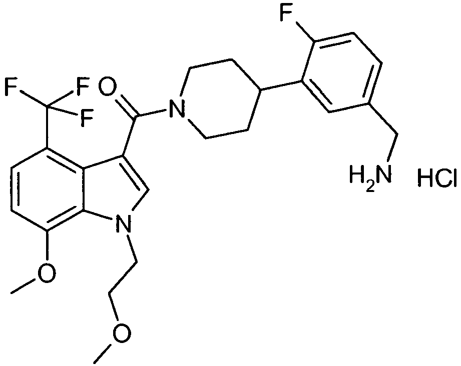

A mixture of 2,2,2-trifluoro-*N*-(4-fluoro-3-{1-[7-methoxy-1-(2-methoxy-ethyl)-4-trifluoromethyl-1*H*-indole-3-carbonyl]-piperidin-4-yl}-benzyl)-acetamide (55 mg, 0.091 mmol) in MeOH (3 mL) is added aqueous K₂CO₃ (100 mg, dissolved in 1.5 mL H₂O). This mixture is stirred at rt for 2 days. The reaction mixture is concentrated *in vacuo* to remove most of the methanol. The residue is partitioned between H₂O and EtOAc. The two layers are separated, and the organic layer is washed with H₂O and brine, dried over MgSO₄, filtered, and concentrated *in vacuo.* The residue is dissolved in Et₂O, and HCl in Et₂O (1.0 M, 3 mL) is added. The resulting suspension is concentrated *in vacuo,* and then dried *in vacuo* to yield 45 mg (90%) of the product as a white solid. ¹H NMR (DMSO-*d₆*): δ 8.27 (br,s 3H), 7.75-7.30 (m, 4H), 7.30-7.15 (m, 1 H), 6.89 (d, *J* = 8.3 Hz, 1 H), 4.85-4.65 (m, 1 H), 4.65-4.45 (m, 2H), 4.10-3.85 (m, 5H), 3.80-3.50 (m, 3H), 3.21 (s, 3H), 3.15-2.95 (m, 2H), 2.90-2.65 (m, 1 H), 1.95-1.40 (m, 4H); ¹⁹F NMR (DMSO-*d₆*): δ -56.83 (s, 3F), -119.23 (s, 1 F); MS 508 (M+1, 100%).

### EXAMPLE 15

### [4-(5-Aminomethyl-2-fluoro-phenyl)-piperidin-1-yl]-[7-fluoro-1-(2-methoxy-ethyl)-4-methyl-1H-indol-3-yl]-methanone hydrochloride

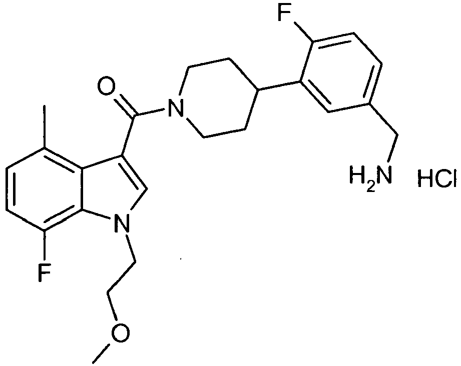

### A. 7-Fluoro-4-methyl-1H-indole

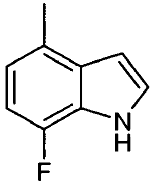

To a mixture of 1-fluoro-2-nitro-4-methyl-benzene (5.17 g, 33.3 mmol) in THF (100 mL) at -78 °C is added vinylmagnesium bromide (100 mL, 1.0 M in THF, 100 mmol) slowly over a 30 min period. After this mixture is stirred at -78 °C for 30 min, sat. NH₄Cl is added, and the cooling bath is removed. The reaction mixture is partitioned between H₂O and EtOAc, and the two layers are separated. The organic layer is washed with H₂O and brine, dried over MgSO₄, filtered, and concentrated *in vacuo.* The crude material is purified on silica gel with heptane/EtOAc (100/0 to 50/50) as eluant to give 1.19 g of the product (contained ∼50% starting material) as a brown oil. This crude material is used in the next step without further purification. ¹H NMR (CDCl₃): δ 8.32 (br, s, 1 H), 7.30-7.20 (m, 1 H), 6.90-6.70 (m, 2H), 6.65-6.55 (m, 1 H), 2.51 (s, 3); ¹⁹ F NMR (CDCl₃): δ -138.96 (s, 3F)

### B. 7-Fluoro-1-(2-methoxy-ethyl)-4-methyl-1H-indole

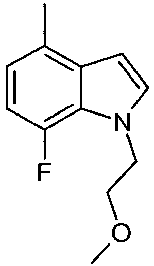

A mixture of 7-fluoro-4-methyl-1*H*-indole (0.5 g, 3.35 mmol), powder KOH (0.93 g, 16.7 mmol) in DMSO (10 mL) is stirred at rt for 5 min. 2-Methoxyethyl bromide (0.47 mL, 5.02 mmol) is then added. After the reaction mixture was stirred at rt overnight, it is partitioned between H₂O and Et₂O. The two layers are separated, and the aqueous layer is extracted with Et₂O. The organic extract is washed with H₂O and brine, dried over MgSO₄, filtered, and concentrated *in vacuo.* The crude material is purified on silica gel with heptane/EtOAc (100/0 to 70/30) as eluant to yield 0.38 g (54%) of the product as a clear yellow oil. ¹H NMR (CDCl₃): δ 7.11 (d, *J* = 3.2 Hz, 1 H), 6.85-6.70 (m, 2 H), 6.47 (t, *J* = 2.7 Hz, 1 H), 4.44 (t, *J* = 5.3 Hz, 2H), 3.72 (t, *J* = 5.0 Hz, 2H), 3.30 (s, 3H), 2.48 (s, 3H); ¹⁹F NMR (EDCl₃): δ -139.71 (t, *J* = 8.5 Hz, 1 F); MS 208 (M+1, 100%)

### C. 2,2,2-Trifluoro-1-[7-fluoro-1-(2-methoxy-ethyl)-4-methyl-1H-indol-3-yl ]-ethanone

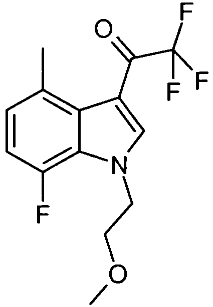

A mixture of 7-fluoro-1-(2-methoxy-ethyl)-4-methyl-1*H*-indole (385 mg, 1.86 mmol) and TFAA (0.69 mL, 5.02 mmol) in DMF (6 mL) is heated at 40 °C overnight. The mixture is partitioned between H₂O and Et₂O. The two layers are separated, and the organic layer is washed with H₂O and brine, dried over MgSO₄, filtered, and concentrated *in vacuo.* The crude material is purified on silica gel with heptane/EtOAc (100/0 to 50/50) as eluant to yield 381 mg (67%) of the product as a yellow crystalline solid. ¹H NMR (CDCl₃): δ 7.97 (d, *J* = 1.5 Hz, 1 H), 7.05-6.85 (m, 2H), 4.51 (t, *J* = 4.9 Hz, 2H), 3.75 (t, *J* = 4.9 Hz, 2H), 3.31 (s, 3H), 2.77 (s, 3H); ¹⁹F NMR (CDCl₃): δ -70.5 (s, 3F), -138.82 (d, *J* = 8.5 Hz, 1 F); MS 304 (M+1, 100%).

### D. 7-Fluoro-1-(2-methoxy-ethyl)-4-methyl-1H-indole-3-carboxylic acid

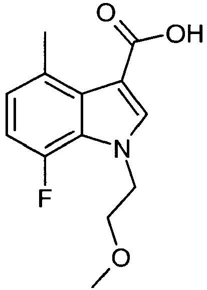

A mixture of 2,2,2-trifluoro-1-[7-fluoro-1-(2-methoxy-ethyl)-4-methyl-1*H*-indol-3-yl ]-ethanone (360 mg, 1.18 mmol) in MeOH (15 mL) and NaOH (5 M, 10 mL) is heated at 85 °C for 3 h. This mixture is concentrated *in vacuo* to remove the methanol. The residue is diluted with H₂O, and then washed with EtOAc once. The aqueous layer is acidified to pH 2 with HCl (3 M). The acidified mixture is extracted with EtOAc. The organic extract is washed with H₂O and brine, dried over MgSO₄, filtered, and concentrated in vacuo to yield 262 mg (88%) of the product as a beige powder. ¹H NMR (CDCl₃): δ 7.97 (s, 1 H), 6.95-6.80 (m, 2 H), 4.47 (t, *J* = 5.0 Hz, 2H), 3.74 (t, *J* = 5.1 Hz, 2H), 3.31 (s, 3H), 2.81 (s, 3H); ¹⁹F NMR (CDCl₃): δ -139.36 (d, *J* = 5.6 Hz, 1 F); MS 252 (M+1, 100%).

### E. 2,2,2-Trifluoro-N-(4-fluoro-3-{1-[7-fluoro-1-(2-methoxy-ethyl-4-methyl-1H-indole-3-carbonyl]-piperidin-4-yl}-benzyl)-acetamide

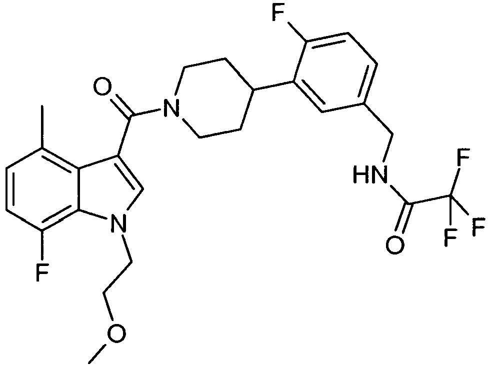

A mixture of 7-fluoro-1-(2-methoxy-ethyl)-4-methyl-1*H*-indole-3-carboxylic acid (250 mg, 1.0 mmol), Et₃N (418 µL, 3.0 mmol), 2,2,2-trifluoro-*N*-(4-fluoro-3-piperidin-4-yl-benzyl)-acetamide hydrochloride (448 mg, 1.3 mmol), and EDCI (286 mg, 1.5 mmol) in CH₂Cl₂ (15 mL) is stirred at rt overnight. The mixture is partitioned between H₂O and EtOAc. The two layers are separated, and the organic layer is washed with brine, dried over MgSO₄, filtered, and concentrated *in vacuo.* The crude material is purified on silica gel with heptane/EtOAc (50/50 to 0/100) as eluant to give 330 mg (61%) of the product as a white powder. ¹H NMR (CDCl₃): δ 7.19 (s, 1H), 7.15-7.10 (m, 2H), 7.05-6.95 (m, 1 H), 6.90-6.75 (m, 2H), 6.60 (br s, 1 H), 5.15-4.70 (br m, 1 H), 4.55-4.35 (m, 4H), 4.30-3.80 (br m, 1 H), 3.71 (t, *J* = 5.1 Hz, 2H), 3.29 (s, 3H), 3.20-2.75 (m, 3H), 2.48 (s, 3H), 2.05-1.55 (m, 4H); ¹⁹F NMR (CDCl₃): δ -75.33 (s, 3F), - 118.72 (s, 1 F), -139.25 (d, *J* = 5.6 Hz, 1 F); MS 538 (M+1, 100%).

### F. [4-(5-Aminomethyl-2-fluoro-phenyl)-piperidin-1-yl]-[7-fluoro-1-(2-methoxy-ethyh-4-methyl-1H-indol-3-yl]-methanone hydrochloride

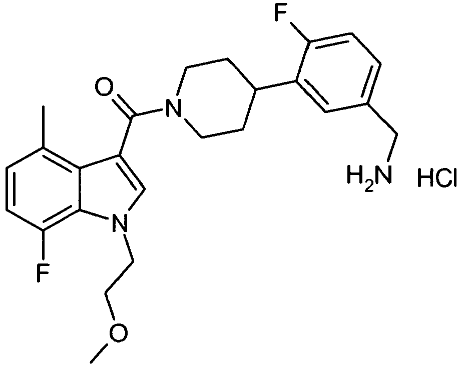

A mixture of 2,2,2-trifluoro-*N*-(4-fluoro-3-{1-[7-fluoro-1-(2-methoxy-ethyl)-4-methyl-1*H-*indole-3-carbonyl]-piperidin-4-yl}-benzyl)-acetamide (315 mg, 0.59 mmol) in MeOH (5 mL) is added aqueous K₂CO₃ (815 mg, dissolved in 5 mL H₂O). This mixture is stirred at rt overnight. The reaction mixture is concentrated *in vacuo* to remove most of the methanol. The residue is partitioned between H₂O and EtOAc. The two layers are separated, and the organic layer is washed with H₂O and brine, dried over MgSO₄, filtered, and concentrated *in vacuo.* The residue is dissolved in Et₂O, and HCl in Et₂O (1.0 M, 3 mL) is added. The resulting suspension is concentrated *in vacuo,* and then dried *in vacuo* to yield 250 mg (88%) of the product as a white solid. ¹H NMR (DMSO-*d₆*): δ 8.29 (br,s 3H), 7.60-7.50 (m, 2H), 7.45-7.30 (m, 1 H), 7.30-7.15 (m, 1 H), 6.95-6.75 (m, 2H), 4.95-4.45 (m, 1 H), 4.44 (d, *J* = 5.2 Hz, 2H), 4.25-3.70 (m, 3H), 3.67 (t, *J* = 4.9 Hz, 2H), 3.21 (s, 3H), 3.20-2.70 (m, 3H), 2.39 (s, 3H), 1.95-1.45 (m, 4H); ¹⁹F NMR (DMSO-*d₆*): δ -119.43 (s, 1 F), -138.33 (d, *J* = 11.3 Hz, 1 F); MS 442 (M+1, 100%).

### EXAMPLE 16

### [4-(5-Aminomethyl-2-fluoro-phenyl)-piperidin-1-yl]-[4,6-dichloro-1-(2-methoxy-ethyl)-1H-indol-3-yl]-methanone hydrochloride

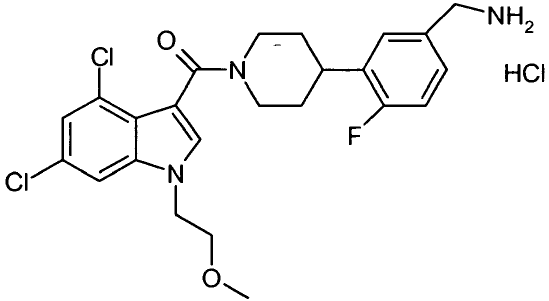

### A. 4,6-Dichloro-1-(2-methoxy-ethyl)-1H-indole

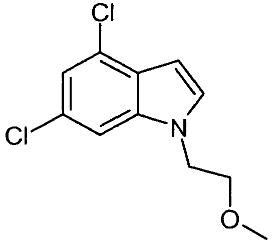

The title compound is prepared in a similar manner as Example 1 D using 4, 6-dichloro-1*H*-indole as the starting material. ¹H NMR (CDCl₃, 300 MHz): δ 7.25 (m, H), 7.2 (m, 1 H), 7.1 (s, H), 6.6 (m, 1 H), 4.2 (t, 2H), 3.7(t, 2H), 3.3(s, 3H). LCMS *m*/*z*: [M+H]⁺=244.

### B. 1-[4,6-Dichloro-1-(2-methoxy-ethyl)-1H-indol-3-yl]-2,2,2-trifluoro-ethanone

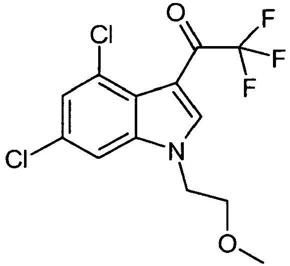

The title compound is prepared in a similar manner as Example 1 E using 4, 6-dichloro-1-(2-methoxy-ethyl)-1*H*-indole as the starting material. ¹H NMR (CDCl₃, 300 MHz): δ 8.0 (s, 1 H), 7.35 (m, 2H), 4.3 (t, 2H), 3.7 (t, 2H), 3.3 (s, 3H). LCMS *m*/*z*: [M+H]⁺=340.

### C. 4,6-Dichloro-1-(2-methoxy-ethyl)-1H-indole-3-carboxylic acid

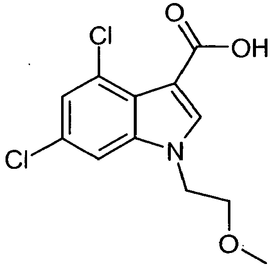

The title compound is prepared in a similar manner as Example 2E using 1-[4, 6-dichloro-1-(2-methoxy-ethyl)-1*H*-indol-3-yl]-2,2,2-trifluoro-ethanone as the starting material. ¹H NMR (DMSO-*d₆*, 300 MHz): δ 7.95 (bs, H), 7.7 (s, 1 H), 7.2 (s, 1 H), 5.8 (s, 1 H), 4.4 (t, 2H), 3.6 (t, 2H), 3.2 (s, 3H). LCMS *m*/*z*: [M+H]⁺=288.

### D. N-{3-{1-[4,6-Dichloro-1-(2-methoxy-ethyl)-1H-indole-3-carbonyl]-piperidin-4-yl}-4-fluoro-benzyl)-2,2,2-trifluoro-acetamide

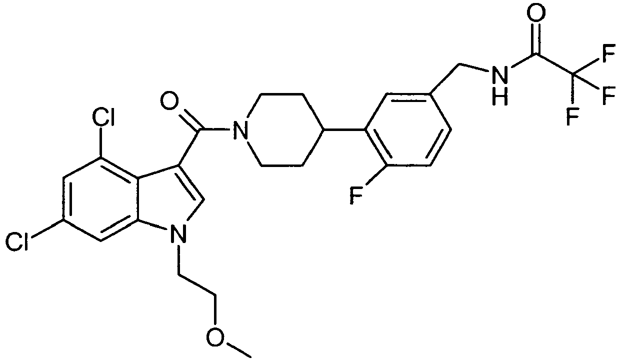

The title compound is prepared in a similar manner as Example 1 G using 4, 6-dichloro-1-(2-methoxy-ethyl)-1*H*-indole-3-carboxylic acid as the starting material. ¹H NMR (CDCl₃, 300 MHz): δ 7.3 (m, 2H), 7.2 (m, 3H), 7.0 (m, H), 6.8 (bs, 1H), 5.0 (m, 1 H), 4.5 (m, 2H), 4.2 (m, 2H), 3.8 (m, 1 H), 3.7 (t, 2H), 3.3(s, 3H), 3.2 (m, 2H), 2.9 (m,1H), 1.9(m, 4H), 1.75 (m, 2H). LCMS *m*/*z*: [M+H]⁺=544.

### E. [4-(5-Aminomethyl-2-fluoro-phenyl)-piperidin-1-yl]-[4,6-dichloro-1-(2-methoxy ethyl)-1H-indol-3-yl]-methanone hydrochloride

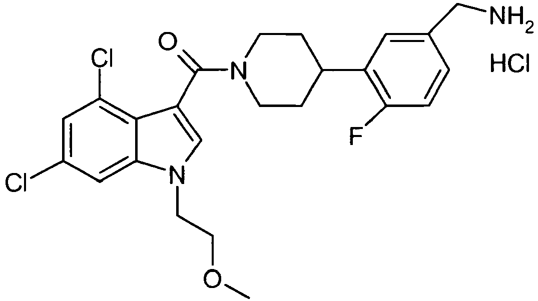

The title compound is prepared in a similar manner as Example 1 H using *N*-(3-{1-[4, 6-dichloro-1-(2-methoxy-ethyl)-1*H*-indole-3-carbonyl]-piperidin-4-yl}-4-fluoro-benzyl)-2,2,2-trifluoro-acetamide as the starting material. ¹H NMR (DMSO-*d₆*, 300 MHz): δ 8.3 (bs, 2H), 7.8 (s, 1 H), 7.7 (s, 1 H), 7.5 (m, 1 H), 7.35 (m, H), 7.2 (m, 2H), 4.8 (m, H), 4.4 (t, 2H), 4.0 (m, 2H), 3.6 (m, 3H), 3.3 (s, 3H), 3.2 (m, 2H), 2.9 (m, H), 1.9-1.6 (m, 4H). LCMS *m*/*z*: [M+H]⁺=478.

### EXAMPLE 17

### [4-(5-Aminomethyl-2-fluoro-phenyl)-piperidin-1-yl]-[4,7-difluoro-1-(2-methoxy-ethyl)-1H-indol-3-yl]-methanone

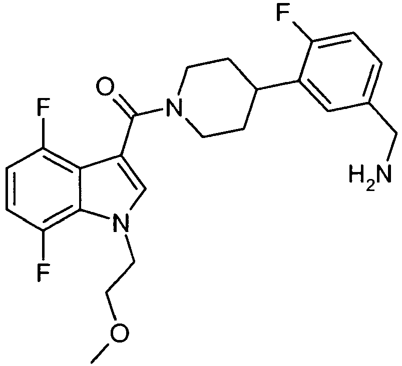

### A. 4,7-Difluoro-1H-indole

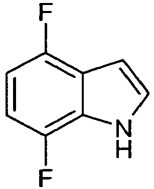

To a mixture of 1,4-difluoro-2-nitro-benzene (10 mL, 92.2 mmol) in THF (80 mL) at - 78 °C is added vinylmagnesium bromide (277 mL, 0.28 mmol) slowly over a 10 min period. After this mixture is stirred at -78 °C for 1 h, sat. NH₄Cl is added, and the cooling bath is removed. The reaction mixture is partitioned between H₂O and EtOAc, and the two layers are separated. The organic layer is washed with H₂O and brine, dried over MgSO₄, filtered, and concentrated *in vacuo.* The crude material is purified on silica gel with heptane/EtOAc (100/0 to 70/30) as eluant to give 1.14 g (8%) of the product as a brown oil. ¹H NMR (CDCl₃): δ 8.40 (br, s, 1 H), 7.21 (t, *J* = 2.7 Hz, 1 H), 6.90-6.70 (m, 1 H), 6.70-6.60 (m, 2H); ¹⁹F NMR (CDCl₃): δ -127.15 (m, 1F), -140.22 (m, 1 F).

### A. 4,7-Difluoro-1-(2-methoxy-ethyl)-1H-indole

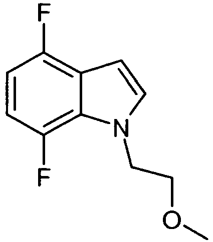

The title compound is prepared in a similar manner as Example 1 D using 4, 7-difluoro-1 H-indole as the starting material. ¹H NMR (CDCl₃, 300 MHz): δ 7.2 (m, H), 7.1 (m, H), 7.0 (s, H), 6.65 (m, H), 6.55 (m, H), 4.35 (t, 2H), 3.6 (t, 2H), 3.2 (s, 3H). LCMS *m*/*z*: [M+H]⁺=212.

### B. 1-[4,7-Difluoro-1-(2-methox-ethyl)-1H-indol-3-yl]-2,2,2-trifluoro-ethanone

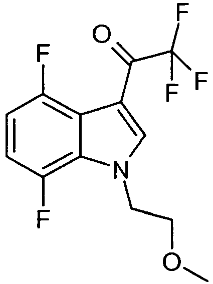

The title compound is prepared in a similar manner as Example 1 E using 4, 7-difluoro-1-(2-methoxy-ethyl)-1*H*-indole as the starting material. ¹H NMR (CD₃OD, 300 MHz): δ 8.15 (s, 1 H), 7.05 (m, 1 H), 6.9 (m, H), 4.75 (t, 2H), 3.7 (t, 2H), 3.3 (s, 3H). LCMS *m*/*z*: [M+H]⁺=308.

### C. 4, 7-Difluoro-1-(2-methoxy-ethyl)-1H-indole-3-carboxylic acid

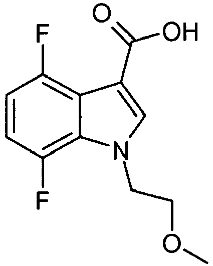

The title compound is prepared in a similar manner as Example 2E using 1-[4,7-difluoro-1-(2-methoxy-ethyl)-1*H*-indol-3-yl]-2,2,2-trifluoro-ethanone as the starting material. ¹H NMR (CDCl₃, 300 MHz): δ 7.95 (bs, 1 H), 6.8 (m, 2H), 4.45 (t, 2H), 3.75 (t, 2H), 3.3 (s, 3H). LCMS *m*/*z*: [M+H]⁺=256.

### D. N-(3-{1-14,7-Difluoro-1-(2-methoxy-ethyl)-1H-indole-3-carbonyl]-piperidin-4-yl}-4-fluoro-benzyl)-2,2,2-trifluoro-acetamide

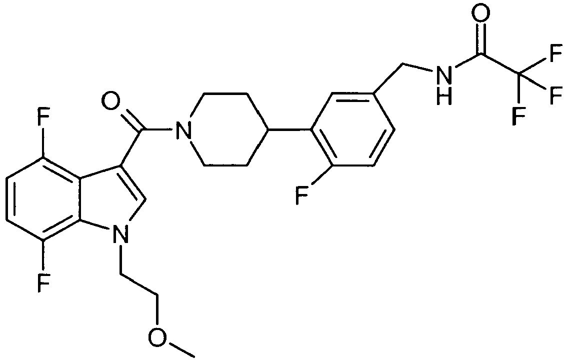

The title compound is prepared in a similar manner as Example 1 G using 4, 7-difluoro-1-(2-methoxy-ethyl)-1*H*-indole-3-carboxylic acid as the starting material. ¹H NMR (DMSO)-*d₆*, 300 MHz): δ 7.6 (s, 1H), 7.2 (m, 1H), 7.1 (m, 2H), 6.95 (m, 1 H), 6.85 (m, 1 H), 4.45 (m, 2H), 4.3 (m, 2H), 3.65 (m, 3H), 3.3 (m, 2H), 3.2 (s, 3H), 2.95 (m, 3H), 1.6 (m, 3H). LCMS *m*/*z*: [M+H]⁺=541.

### E. [4-(5-Aminomethyl-2-fluoro-phenyl)-piperidin-1-yl]-[4,7-Difluoro-1-(2-methoxy ethyl)-1H-indol-3-yl]-methanone

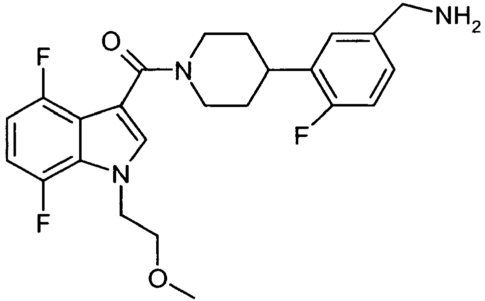

The title compound is prepared in a similar manner as Example 1 H using *N*-(3-{1-[4,7-difluoro-1-(2-methoxy-ethyl)-1H-indole-3-carbonyl]-piperidin-4-yl}-4-fluoro-benzyl)-2,2,2-trifluoro-acetamide as the starting material. ¹H NMR (DMSO-d₆, 300 MHz): δ 7.7 (s, 1H), 7.35 (m, 1 H), 7.25 (m, 2H), 7.0 (m, 1 H), 6.8 (m, 1H) 4.5 (m, 3H), 3.6 (m, 2H), 3.25 (m, 2H), 3.3 (s, 3H), 2.9 (m, 3H), 1.9-1.6 (m, 3H). LCMS *m*/*z*: [M+H]⁺=445.

### EXAMPLE 18

### [4-(5-Aminomethyl-2-fluoro-phenyl)-piperidin-1-yl]:[4,6-difluoro-1-(2-methoxy-ethyl)-1H-indol-3-yl]-methanone hydrochloride

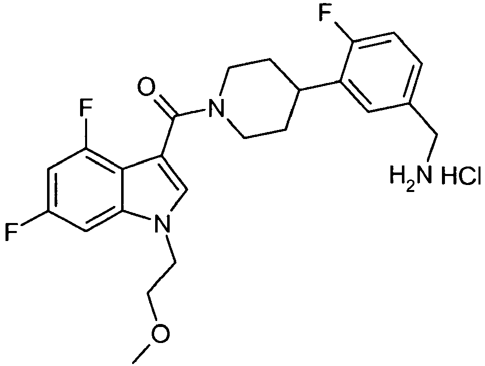

### A. 4,6-Difluoro-1-(2-methoxy-ethyl)-1H-indole

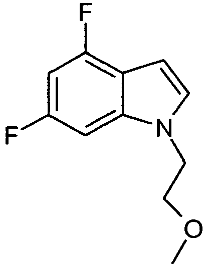

The title compound is prepared in a similar manner as Example 1D using 4, 6-difluoro-1 H-indole as the starting material. ¹H NMR (CDCl₃, 300 MHz): δ 7.2 (m, 1 H), 7.1 (m, 1 H), 6.85 (s, 1H), 6.5 (m, 2H), 4.2 (t, 2H), 3.7(t, 2H), 3.3(s, 3H). LCMS *m*/*z*: [M+H]⁺=212.

### B. 1-[4,6-Difluoro-1-(2-methoxy-ethyl)-1H-indol-3-yl]-2,2,2-trifluoro-ethanone

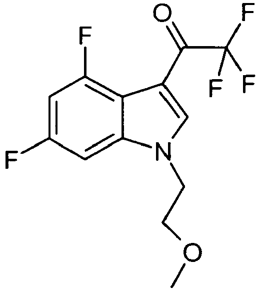

The title compound is prepared in a similar manner as Example 1 E using 4, 6-difluoro-1-1*H*-indole as the starting material. ¹H NMR (DMSO-*d₆*, 300 MHz): δ 8.5 (s, 1 H), 7.65 (m, 1 H), 7.1 (m, 1 H), 4.5 (t, 2H), 3.7 (t, 2H), 3.2 (s, 3H). LCMS *m*/*z*: [M+H]⁺=308.

### C. 4,6-Difluoro-1-(2-methoxy-ethyl)-1H-indole-3-carboxylic acid

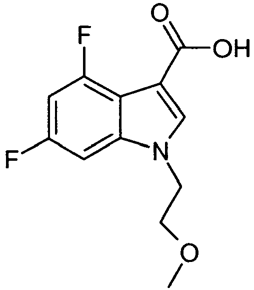

The title compound is prepared in a similar manner as Example 2E using 1-[4,6-difluoro-1-(2-methoxy-ethyl)-1*H*-indol-3-yl]-2,2,2-trifluoro-ethanone as the starting material. ¹H NMR (DMSO-*d₆*, 300 MHz): δ 12.05 (s, 1 H), 8.05 (s, 1 H), 7.4 (m, 1 H), 6.95 (m, 1 H). 4.4 (t, 2H), 3.6 (t, 2H), 3.2 (s, 3H). LCMS *m*/*z*: [M+H]⁺=256.

### D. N-(3-{1-[4,6-Difluoro-1-(2-methoxy-ethyl)-1H-indole-3-carbonyl]-piperidin-4-yl}-4-fluoro-benzyl)-2,2,2-trifluoro-acetamide

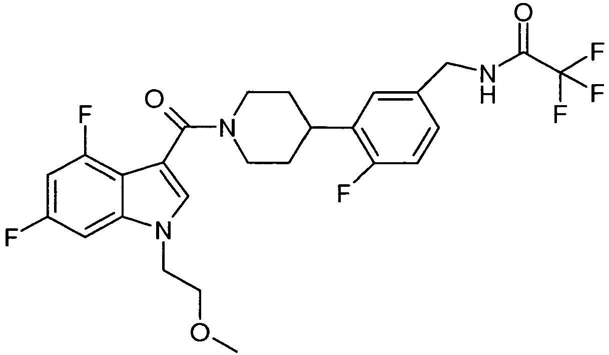

The title compound is prepared in a similar manner as Example 1 G using 4, 6-difluoro-1-(2-methoxy-ethyl)-1*H*-indole-3-carboxylic acid as the starting material. ¹H NMR (DMSO-*d₆*, 300 MHz): δ 7.6 (s, 1H), 7.4 (m, 1 H), 7.3 (m, 1 H), 7.1 (m, 2H), 6.9, (m, 1 H), 4.4 (m, 4H), 3.8 (m, 1 H), 3.7 (t, 2H), 3.3 (m, 2H), 3.2(s, 3H), 3.0 (m, 2H), 2.9, 1.75(m, 2H), 1.65 (m, 2H). LCMS *m*/*z*: [M+H]⁺=542.

### E. [4-(5-Aminomethyl-2-fluoro-phenyl)-piperidin-1-yl]-[4,6-difluoro-1-(2-methoxyethyl)-1H-indol-3-yl]-methanone hydrochloride

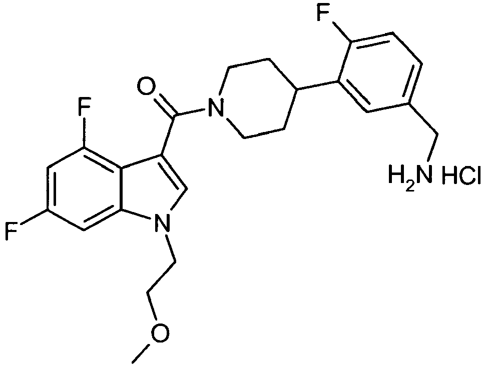

The title compound is prepared in a similar manner as Example 1 H using *N*-(3-{1-[4, 6-difluoro-1-(2-methoxy-ethyl)-1*H*-indole-3-carbonyl]-piperidin-4-yl}-4-fluoro-benzyl)-2,2,2-trifluoro-acetamide as the starting material. ¹H NMR (DMSO-*d₆*, 300 MHz): δ 7.6 (s, 1 H), 7.35 (m, 1 H), 7.25 (m, 2H), 7.0 (m, 1 H), 6.8 (m, 1 H) 4.5 (m, 3H), 3.3 (m, 2H), 3.2 (s, 3H), 2.9 (m, 2H), 1.9-1.6 (m, 4H). LCMS *m*/*z*: [M+H]⁺=445.

### EXAMPLE 19

### [4-(5-Aminomethyl-2-fluoro-phenyl)-piperidin-1-yl]-[4,5-difluoro-1-(2-methoxy-ethyl)-1H-indol-3-yl]-methanone hydrochloride

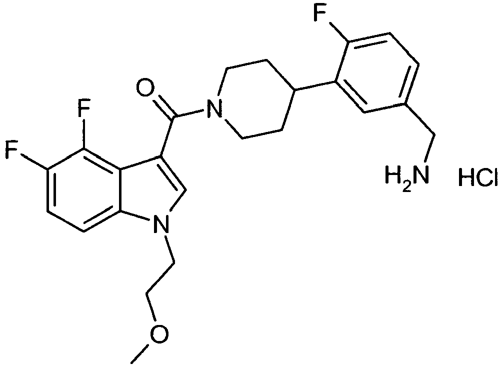

### A.. (3,4-Difluoro-phenyl)-carbamic acid ethyl ester

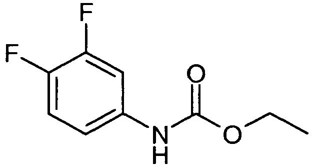

To a solution of 3,4-difluoro-phenylamine (1.0 g, 7.45 mmol) and pyridine (751 µL, 9.29 mmol) in THF (10 mL) is added ethyl chloroformate (888 µL, 9.29 mmol) dropwise over a 30s period. This mixture is stirred at rt for 30 min. The reaction mixture is partitioned between H₂O and EtOAc. The two layers are separated, and the organic layer is washed with HCl (1 M), H₂O, and brine, dried over MgSO₄, filtered, and concentrated *in vacuo* to yield 1.43 g (95%) of the product as a brown solid. ¹H NMR (CDCl₃): δ 7.45-7.35 (m, 1 H), 7.07 (q, *J*= 8.9 Hz,1H), 7.00-6.90 (m, 1 H), 6.55 (br s, 1 H), 4.23 (q, *J* = 7.1 Hz, 2H), 1.31 (t, *J* = 7.1 Hz, 3H); ¹⁹F NMR (CDCl₃): δ -135.33 (m, 1 F), -143.85 (br m, 1 F); MS 243 (M+CH₃CN+1, 100%), 202 (M+1).

### B. (3,4-Difluoro-2-iodo-phenyl)-carbamic acid ethyl ester

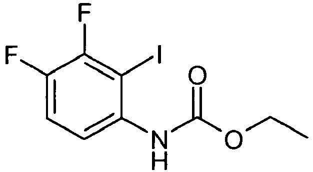

To a solution of (3,4-difluoro-phenyl)-carbamic acid ethyl ester (0.5 g, 2.49 mmol) in THF (7 mL) at -78 °C is added sec-BuLi (4.3 mL, 1.4 M in cyclohexane, 5.98 mol) dropwise over a 10 min period. After 1 h, a solution of I₂ (0.78 g, 2.99 mmol) in THF (5 mL) is added dropwise over a 5 min period. After 30 min, the reaction mixture is quenched with sat. NH₄Cl, and the cooling bath is removed. The mixture is partitioned with H₂O and EtOAc. The two layers are separated, and the organic layer is washed with H₂O and brine, dried over MgSO₄, filtered, and concentrated *in vacuo.* The crude material is purified on silica gel with heptane/EtOAc (100/0 to 70/30) as eluant to yield 0.36 g (44%) of the product as a white solid. ¹H NMR (CDCl₃): δ 7.95-7.75 (m, 1 H), 7.18 (q, *J* = 9.3 Hz, 1 H), 6.88 (br s, 1 H), 4.26 (q, *J* = 7.1 Hz, 2H), 1.34 (t, *J* = 7.1 Hz, 3H); ¹⁹F NMR (CDCl₃): δ -111.17 (m, 1 F), -140.23 (s, 1 F); MS 327 (M+1, 100%).

### C. (3,4-Difluoro-2-trimethylsilanylethynyl-phenyl)-carbamic acid ethyl ester

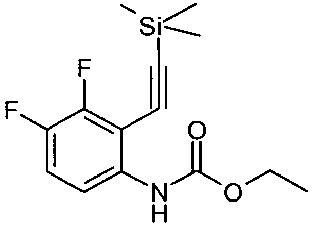

A mixture of (3, 4-difluoro-2-iodo-phenyl)-carbamic acid ethyl ester (309 mg, 0.94 mmol), TMS-aceetylene (401 µL, 2.82 mol), TEA (263 µL, 1.89 mmol), Cul (14 mg, 5% mol), and bistriphenylphosphinepalladium (II) chloride (33 mg, 5% mol) in degassed THF (10 mL) is stirred at rt for 5 h. The mixture is partitioned between H₂O and EtOAc. The two layers are separated, and the organic layer is washed with brine, dried over MgSO₄, filtered, and concentrated *in vacuo.* The crude material was purified on silica gel with heptane/EtOAc (100/0 to 70/30) as eluant to yield 222 mg (79%) of the product as a yellow oil. ¹H NMR (CDCl₃): δ 7.90-7.80 (m, 1 H), 7.26 (br s, 1 H), 7.11 (q, *J* = 9.5 Hz, 1 H), , 4.25 (q, *J* = 7.1 Hz, 2H), 1.33 (t, *J* = 7.1 Hz, 3H), 0.32 (s, 3H); ¹⁹F NMR (CDCl₃): δ -132.73 (m, 1F), -144.40 (m, 1F); MS 298 (M+1, 100%).

### D. 4.5-Difluoro-1H-indole

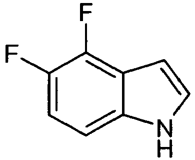

A mixture of (3,4-difluoro-2-trimethylsilanylethynyl-phenyl)-carbamic acid ethyl ester (220 mg, 0.7407 mmol) and KOH (332 mg, 5.927 mmol) in *t*-BuOH (10 mL) is heated at 70 °C overnight. The solvent is removed *in vacuo,* and the residue is partitioned between H₂O and Et₂O. The two layers are separated, and the organic layer is washed with brine, dried over MgSO₄, filtered, and concentrated *in vacuo.* The crude material is purified on silica gel with heptane/EtOAc (100/0 to 50/50) as eluant 71 mg (62%) of the product as a clear brown solid. ¹H NMR (CDCl₃): δ 8.20 (br, s, 1H), 7.30-7.15 (m, 1 H), 7.10-6.95 (m, 2H), 6.70-6.60 (m, 1 H); ¹⁹F NMR (CDCl₃): δ -147.78 (m, 1 F), -155.94 (m, 1 F).

### E. 4,5-Difluoro-1-(2-methoxy-ethyl)-1H-indole

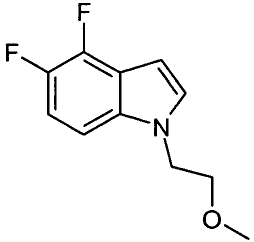

The title compound is prepared in a similar manner as Example 1D using 4, 5-difluoro-1*H*-indole as the starting material. ¹H NMR (CDCl₃, 300 MHz): δ 7.05 (m, 2H), 6.6 (m, H), 6.45 (m, 1 H), 4.4 (t, 2H), 3.6(t, 2H), 3.2(s, 3H). LCMS *m*/*z*: [M+H]⁺=212.

### F. 1-[4,5-Difluoro-1-(2-methoxy-ethyl)-1H-indol-3-yl]-2,2,2-trifluoro-ethanone

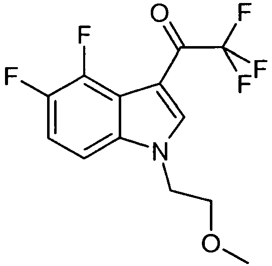

The title compound is prepared in a similar manner as Example 1 E using 4, 5-difluoro-1-(2-methoxy-ethyl)-1*H*-indole as the starting material. ¹H NMR (DMSO-*d₆*, 300 MHz): δ 8.5 (s, 1 H), 7.65 (m, 1 H), 7.1 (m, 1 H), 4.5 (t, 2H), 3.7 (t, 2H), 3.2 (s, 3H). LCMS *m*/*z*: [M+H]⁺=308.

### G. 4,5-Difluoro-1-(2-methoxy-ethyl)-1H-indole-3-carboxylic acid

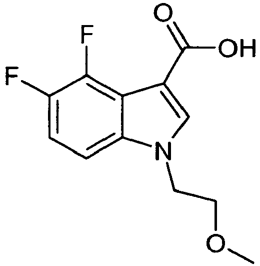

The title compound is prepared in a similar manner as Example 2E using 1-[4, 5-difluoro-1-(2-methoxy-ethyl)-1*H*-indol-3-yl]-2,2,2-trifluoro-ethanone as the starting material. ¹H NMR (CD₃OD, 300 MHz): δ 8.0 (s, 1 H), 7.3 (m, 1 H), 7.1 (m, 1 H), 5.8 (s, 1 H), 4.4 (t, 2H), 3.7 (t, 2H), 3.3 (s, 3H). LCMS *m*/*z*: [M+H]⁺=256.

### H. N-(3-{1-[4,5-Difluoro-1-(2-methoxy-ethyl)-1H-indole-3-carbonyl]-piperidin-4-yl}-4-fluoro-benzyl)-2,2,2-trifluoro-acetamide

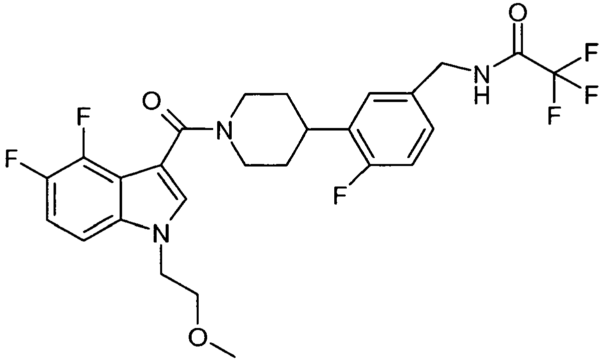

The title compound is prepared in a similar manner as Example 1 G using 4, 5-difluoro-1-(2-methoxy-ethyl)-1*H*-indole-3-carboxylic acid as the starting material. ¹H NMR (DMSO-*d₆*, 300 MHz): δ 7.6 (s, 1 H), 7.3 (m, 1 H), 7.2 (m, 1 H), 7.1 (m, 1 H), 4.5 (m, 4H), 3.8 (m, 1 H), 3.7 (t, 2H), 3.3 (m, 2H), 3.2(s, 3H), 3.0 (m, 2H), 2.9, 1.75(m, 2H), 1.65 (m, 2H). LCMS *m*/*z*: [M+H]⁺=542.

### 1. [4-(5-Aminomethyl-2-fluoro-phenyl)-piperidin-1-yl]-[4,5-difluoro-1-(2-methoxy ethyl)-1H-indol-3-yl]-methanone hydrochloride

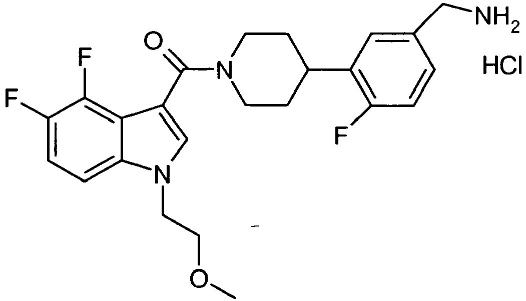

The title compound is prepared in a similar manner as Example 1 H using N-(3-{1-[4, 5-difluoro-1-(2-methoxy-ethyl)-1*H*-indole-3-carbonyl]-piperidin-4-yl}-4-fluoro-benzyl)-2,2,2-trifluoro-acetamide as the starting material. ¹H NMR (DMSO-*d₆*, 300 MHz): δ 7.6 (s, 1 H), 7.35 (m, 1 H), 7.25 (m, 2H), 7.0 (m, 1 H), 6.8 (m, 1 H) 4.5 (m, 3H), 3.3 (m, 2H), 3.25 (m, 3H), 3.3 (s, 3H), 3.2 (m, 2H), 2.9 (m, H), 1.9-1.6 (m, 4H). LCMS *m*/*z*: [M+H]⁺=445.

### EXAMPLE 20

### [4-(5-Aminomethyl-2-fluoro-phenyl)-piperidin-1-yl]-[5,6-dimethoxy-1-(2-methoxyethyl)-1H-indol-3-yl]-methanone hydrochloride

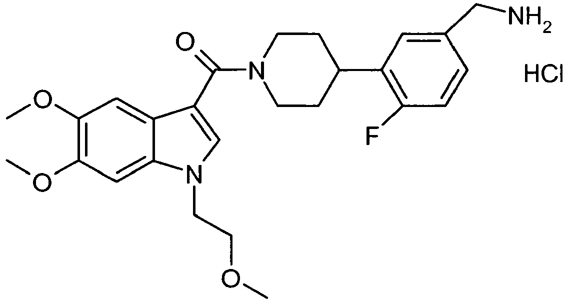

### A. 5,6-Dimethoxy-1-(2-methoxy-ethyl)-1H-indole

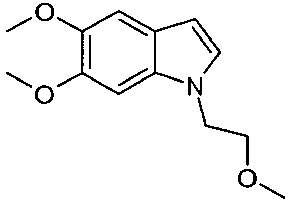

The title compound is prepared in a similar manner as Example 1D using 5, 6-dimethoxy-1*H*-indole as the starting material. ¹H NMR (CDCl₃, 300 MHz): δ 7.1 (s, H), 7.0 (m, 1 H), 6.8 (s, H), 6.4 (m, 1 H), 4.2 (t, 2H), 3.95 (d, 6H), 3.7 (t, 2H), 3.3 (s, 3H). LCMS *m*/*z*: [M+H]⁺=236.

### B. 1-[5,6-Dimethoxy-1-(2-methoxy-ethyl)-1H-indol-3-yl]-2,2,2-trifluoro-ethanone

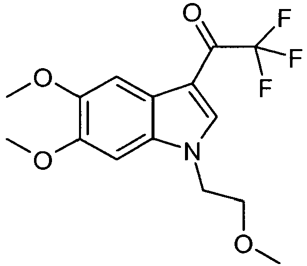

The title compound is prepared in a similar manner as Example 1 E using 5, 6-dimethoxy-1-(2-methoxy-ethyl)-1H-indole as the starting material. ¹H NMR (CDCl₃, 300 MHz): δ 7.9 (m, 2H), 6.9 (s, H), 4.3 (t, 2H), 4.0 (d, 6H), 3.75 (t, 2H), 3.3 (s, 3H). LCMS *m*/*z*: [M+H]⁺=332.

### C. 5,6-Dimethoxy-1-(2-methoxy-ethyl)-1H-indole-3-carboxylic acid

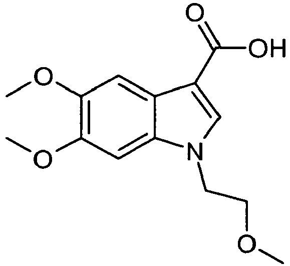

The title compound is prepared in a similar manner as Example 2E using 1-[5,6-dimethoxy-1-(2-methoxy-ethyl)-1H-indol-3-yl]-2,2,2-trifluoro-ethanone as the starting material. ¹H NMR (DMSO-*d₆*, 300 MHz): δ 11.8 (bs, H), 7.8 (s, H), 7.5 (s, 1 H), 7.2 (s, 1 H), 4.4 (t, 2H), 3.8 (d, 6H), 3.6 (t, 2H), 3.3 (s, 3H). LCMS *m*/*z*: [M+H]⁺=280.

### D. N-(3-{1-[5,6-Dimethoxy-1-(2-methoxy-ethyl)-1H-indole-3-carbonyl]-piperidin-4-yl}-4-fluoro-benzyl)-2,2,2-trifluoro-acetamide

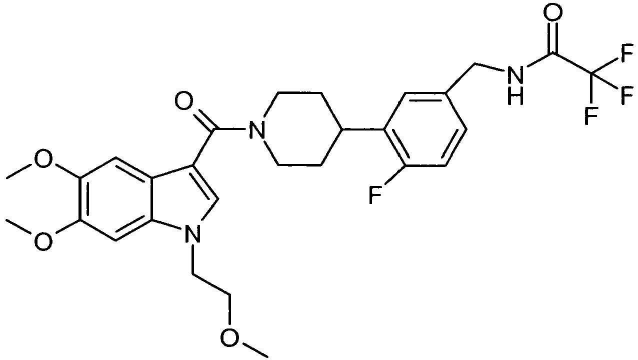

The title compound is prepared in a similar manner as Example 1 G using 5, 6-dimethoxy-1-(2-methoxy-ethyl)-1H-indole-3-carboxylic acid as the starting material. ¹H NMR (CDCl₃, 300 MHz): δ 7.3 (m, 2H), 7.2 (m, 2H), 7.1 (m, H), 6.8 (s, 1 H), 6.7 (bs, H), 4.6 (m, 2H), 4.5 (m, 2H), 4.2 (m, 2H), 3.9 (s, 6H), 3.7 (m, 2H), 3.3(s, 3H), 3.1 (m, 3H), 1.9(m, 2H), 1.8 (m, 2H). LCMS *m*/*z*: [M+H]⁺=566.

### E. [4-(5-Aminomethyl-2-fluoro-phenyl)-piperidin-1-yl]-[5,6-dimethoxy-1-(2-methoxy ethyl)-1H-indol-3-yl]-methanone hydrochloride

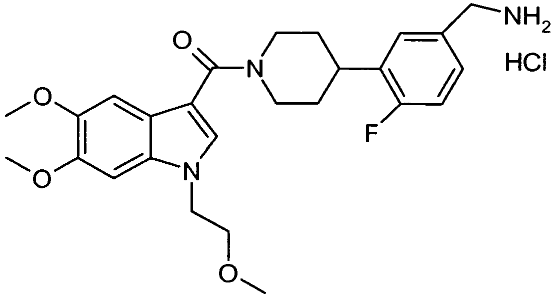

The title compound is prepared in a similar manner as Example 1 H using *N*-(3-{1-[5, 6-dimethoxy-1-(2-methoxy-ethyl)-1*H*-indole-3-carbonyl]-piperidin-4-yl}-4-fluorobenzyl)-2,2,2-trifluoro-acetamide as the starting material. ¹H NMR (DMSO-*d₆*, 300 MHz): δ 8.3 (bs, 2H), 7.6 (m, 2H), 7.4 (m, H), 7.2 (m, 3H), 4.5 (m, 2H), 4.4 (m, 2H), 4.3 (m, 2H), 4.0 (m, 2H), 3.8 (d, 6H), 3.6 (m, 2H), 3.3 (s, 3H), 3.2 (m, 3H), 1.9-1.6 (m, 4H). LCMS *m*/*z*: [M+H]⁺=470.

### Example 21

### [4-(5-Aminomethyl-2-fluoro-phenyl)-piperidin-1-yl]-[7-fluoro-4-trifluoromethoxy-1-(2-trifluoromethoxy-ethyl)-1H-indol-3-yl]-methanone hydrochloride

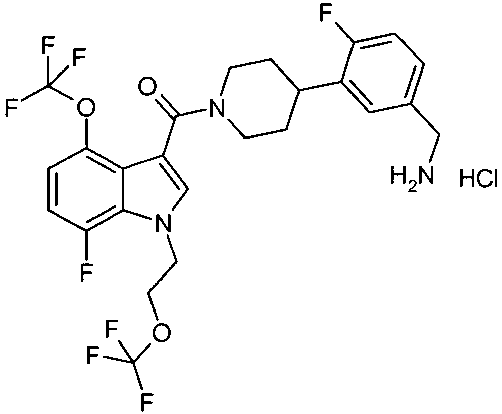

### A. 2,2,2-Trifluoro-1-(7-fluoro-4-trifluoromethoxy-1H-indol-3-yl)-ethanone

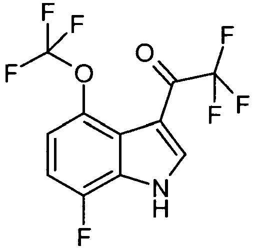

A mixture of 7-fluoro-4-trifluoromethoxy-1*H*-indole (645 mg, 2.94 mmol) and TFAA (1.64 mL, 11.7 mmol) in DMF (10 mL) is stirred at 40 °C overnight. This mixture is partitioned between water and Et₂O. The two layers are separated, and the organic layer is washed with saturated Na₂CO₃, water, and brine, dried over MgSO₄, filtered, and concentrated *in vacuo.* The beige powder is suspended in CH₂Cl₂ and heptane. The suspension is concentrated *in vacuo* and air-dried. The yield of the reaction was 680 mg (73%). ¹H NMR (DMSO-*d₆*): δ 13.69 (bs, 1 H), 8.60 (d, *J* = 1.8 Hz, 1 H), 7.40-7.20 (m, 2H); ¹⁹F NMR (DMSO-*d₆*): δ -56.86 (s, 3F), -70.38 (s, 3F), -131.18 (d, *J* = 8.5 Hz, 1 F); MS 316 (M+1).

### B. 7-Fluoro-4-trifluoromethoxy-1H-indole-3-carboxylic acid

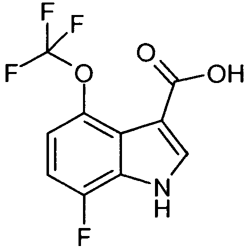

A mixture of 2,2,2-trifluoro-1-(7-fluoro-4-trifluoromethoxy-1*H*-indol-3-yl)-ethanone (200 mg, 0.63 mmol)in MeOH (2 mL) and 5 M NaOH (10 mL) is heated at 140 °C. Water is added occasionally to compensate the lost of solvent. After 8 h, the mixture is cooled to rt. The mixture is partitioned between water and Et₂O. The two layers are separated, and the aqueous layer is cooled in ice bath. 50% acetic acid is added until pH ∼4. The acidified aqueous layer is extracted with EtOAc (2X). The combined organic extracts are washed with water and brine, dried over MgSO₄, filtered, and concentrated *in vacuo* to 87 mg (52%) of the product as a beige powder. ¹H NMR (DMSO-*d₆*): δ 12.75 (bs, 1 H), 12.10 (bs, 1 H), 8.14 (s, 1 H), 7.20-7.00 (m, 2H); ¹⁹F NMR (DMSO-*d₆*): δ -56.62 (s, 3F), -132.83 (d, J = 11.3 Hz, 1 F); MS 264 (M+1).

### C. 7-Fluoro-4-trifluoromethoxy-1H-indole-3-carboxylic acid methyl ester

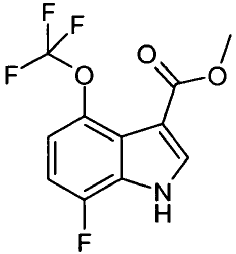

HCl gas is bubbled into a solution of 7-fluoro-4-trifluoromethoxy-1*H*-indole-3-carboxylic acid (80 mg, 0.3 mmol) in MeOH (20 mL) for 30 s. This mixture is stirred at rt overnight. The mixture is concentrated *in vacuo,* and the mixture is suspended in CH₂Cl₂ and heptane. This suspension is concentrated to dryness *in vacuo* to yield 70 mg (84%) of the product as a light yellow powder. ¹H NMR (CDCl₃): δ 8.85 (bs, 1 H), 8.03 (d, *J* = 2.4 Hz, 1H), 7.15-7.05 (m, 1 H), 7.05-6.90 (m, 1 H), 3.91 (s, 3H); ¹⁹F NMR (CDCl₃): δ -57.84 (s, 3F), -135.57 (d, *J* = 8.5 Hz, 1 F); MS 278 (M+1).

### D. 7-Fluoro-4-trifluoromethoxy-1-(2-trifluoromethoxy-ethyl)-1H-indole-3-carboxylic acid methyl ester

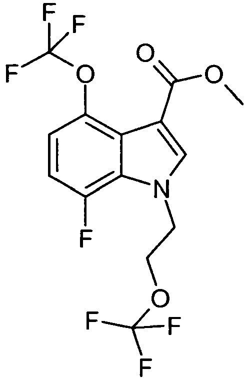

A mixture of 7-fluoro-4-trifluoromethoxy-1*H*-indole-3-carboxylic acid methyl ester (70 mg, 0.25 mmol) and NaH (∼20 mg, 60% oil dispersion) in THF (5 mL) is stirred at rt for 5 min. Trifluoro-methanesulfonic acid 2-trifluoromethoxy-ethyl ester (J. Org. Chem 2001, 66, 1061-1062) (98 µg, 0.38 mmol) is added. This mixture is stirred at rt for 30 min. Saturated NH₄Cl is added, and the mixture is partitioned between water and EtOAc. The two layers are separated, and the organic layer is washed with brine, dried over MgSO₄, filtered, and concentrated *in vacuo.* The crude material is purified on silica gel with heptane/EtOAc (80/20) as eluant to give 84 mg (86%) of the product as a slightly yellow film. ¹H NMR (CDCl₃): δ 7.86 (s, 1H), 7.15-7.05 (m, 1 H), 7.05-6.90 (m, 1 H), 4.60 (t, *J* = 5.1 Hz, 2H), 4.32 (t, *J* = 5.1 Hz, 2H), 3.90 (s, 3H); ¹⁹F NMR (CDCl₃): -57.79 (s, 3F), -60.72 (s, 3F), -136.73 (d, *J* = 8.5 Hz, 1F); MS 390 (M+1).

### E. 7-Fluoro-4-trifluoromethoxy-1-(2-trifluoromethoxy-ethyl)-1H-indole-3-carboxylic acid

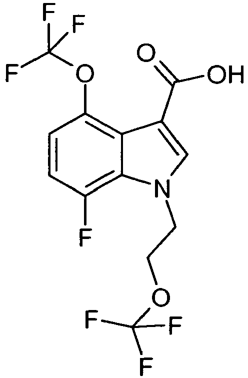

A mixture of 7-fluoro-4-trifluoromethoxy-1-(2-trifluoromethoxy-ethyl)-1*H*-indole-3-carboxylic acid methyl ester (80 mg, 0.21 mmol) in MeOH (5 mL) and aqueous NaOH (1.0 M, 5 mL) is stirred at 30 °C overnight. The mixture is concentrated *in vacuo* to remove the organic solvent. The residue is partitioned between water and EtOAc. The two layers are separated, and the aqueous layer is acidified to pH ∼2 with conc. HCl. The acidified aqueous layer is extracted with EtOAc. The organic extract is washed with water and brine, dried over MgSO₄, filtered, and concentrated *in vacuo* to give 68 mg (86%) of the product as a white powder. ¹H NMR (CDCl₃): δ 7.97 (s, 1H), 7.15-7.05 (m, 1 H), 7.05-6.95 (m, 1 H), 4.63 (t, *J* = 5.1 Hz, 2H), 4.34 (t, *J* = 5.1 Hz, 2H); ¹⁹F NMR (CDCl₃): δ -58.70 (s, 3F), -61.63 (s, 3F), -137.15 (d, *J* = 14.1 Hz, 1 F); MS 376 (M+1).

### D. 2,2,2-Trifluoro-N-(4-fluoro-3-{1-[7-fluoro-4-trifluoromethoxy-1-(2-trifluoromethoxy-ethyl)-1H-indole-3-carbonyl]-piperidin-4-yl}-benzyl)-acetamide

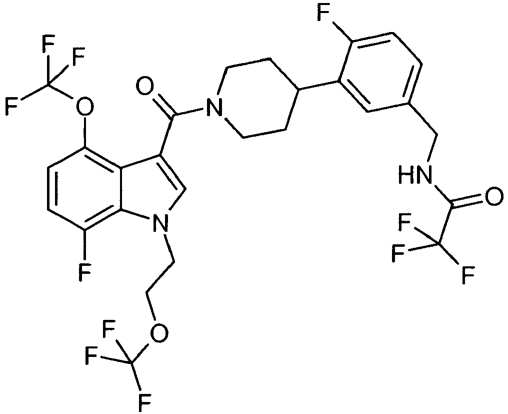

A mixture of 7-fluoro-4-trifluoromethoxy-1-(2-trifluoromethoxy-ethyl)-1*H*-indole-3-carboxylic acid (60 mg, 0.16 mmol), Et₃N (45 µL, 0.32 mmol), 2,2,2-trifluoro-*N*-(4-fluoro-3-piperidin-4-yl-benzyl)-acetamide hydrochloride (658 mg, 0.19 mmol), and EDCI (46 mg, 0.24 mmol) in CH₂Cl₂ (10 mL) is stirred at rt overnight. The mixture is partitioned between H₂O and EtOAc. The two layers are separated, and the organic layer is washed with brine, dried over MgSO₄, filtered, and concentrated *in vacuo.* The crude material is purified on silica gel with heptane/EtOAc (50/50 to 0/100) as eluant to give 80 mg (75%) of the product as a white powder. ¹H NMR (CDCl₃): δ 7.31 (s, 1 H), 7.20-7.10 (m, 2H), 7.10-6.85 (m, 3H), 6.69 (br s, 1 H), 5.15-4.70 (br m, 1 H), 4.65-4.55 (m, 2H), 4.55-4.40 (m, 2H), 4.35-4.25 (m, 2H), 3.95-3.60 (m, 1 H), 3.30-2.70 (m, 3H), 2.10-1.50 (m, 4H); ¹⁹F NMR (CDCl₃): δ -58.45 (s, 3F), -61.56 (s, 3F), -76.32 (s, 3F), -120.03 (s, 1 F), -137.86 (d, *J* = 11.3 Hz, 1 F); MS 662 (M+1, 100%).

### E. [4-(5-Aminomethyl-2-fluoro-phenyl)-piperidin-1-yl]-[7-fluoro-4-trifluoromethoxy-1-(2-trifluoromethoxy-ethyl)-1H-indol-3-yl]-methanone hydrochloride

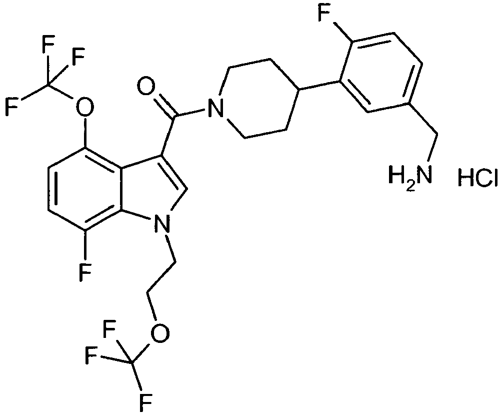

A mixture of 2,2,2-trifluoro-*N*-(4-fluoro-3-{1-[7-fluoro-4-trifluoromethoxy-1-(2-trifluoromethoxy-ethyl)-1*H*-indole-3-carbonyl]-piperidin-4-yl}-benzyl)-acetamide (70 mg, 0.11 mmol) in MeOH (5 mL) is added aqueous K₂CO₃ (700 mg, dissolved in 3 mL H₂O). This mixture is stirred at rt for 7 h. The reaction mixture is concentrated *in vacuo* to remove most of the methanol. The residue is partitioned between H₂O and EtOAc. The two layers are separated, and the organic layer is washed with H₂O and brine, dried over MgSO₄, filtered, and concentrated *in vacuo.* The residue is dissolved in Et₂O, and HCl in Et₂O (1.0 M, 3 mL) is added. The resulting suspension is concentrated *in vacuo,* and then dried *in vacuo* to yield 45 mg (70%) of the product as a white solid. ¹H NMR (DMSO-*d₆*): δ 8.38 (br,s 3H), 7.82 (s, 1 H), 7.55-7.30 (m, 2H), 7.30-7.00 (m, 3H), 4.90-4.60 (m, 3H), 4.55-4.40 (m, 2H), 4.10-3.90 (m, 1 H), 3.90-3.55 (m, 1 H), 3.25-2.60 (m, 3H), 2.00-1.50 (m, 4H); ¹⁹F NMR (DMSO-*d₆*): δ -57.54 (s, 3F), -59.68 (s, 3F), -120.09 (s, 1 F), -135.43 (d, *J* = 8.5 Hz, 1 F); MS 567 (M+1, 100%).

### Example 22

### 4-(5-Aminomethyl-2-fluoro-phenyl)-piperidin-1-yl]-14-fluoro-7-methyl-1-(2-trifluoromethoxy-ethyl)-1H-indol-3-yl]-methanonehydrochloride

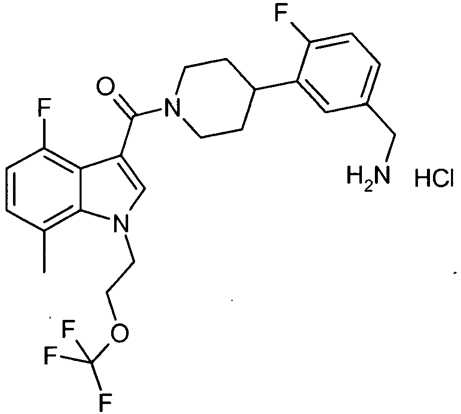

### A. 2,2,2-Trifluoro-1-(4-fluoro-7-methyl-1H-indol-3-yl)-ethanone

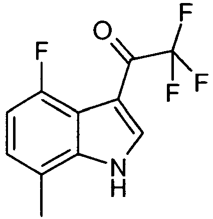

A mixture of 4-fluoro-7-methyl-1*H*-indole (650 mg, 4.35 mmol) and TFAA (1.7 mL, 12.2 mmol) in DMF (20 mL) is stirred at rt for 30 min. This mixture is partitioned between water and Et₂O. The two layers are separated, and the organic layer is washed with saturated Na₂CO₃, water, and brine, dried over MgSO₄, filtered, and concentrated *in vacuo* to yield 690 mg (73%) of the crude product as a beige powder. ¹H NMR (DMSO-*d₆*): δ 12.09 (bs, 1 H), 8.43 (d, *J* = 1.2 Hz, 1 H), 7.20-6.80 (m, 2H), 2.50 (s, 3H); ¹⁹F NMR (DMSO-*d₆*): δ -70.05 (s, 3F), -112.55 (d, *J* = 8.5 Hz, 1 F); MS 246 (M+1).

### B. 4-Fluoro-7-methyl-1H-indole-3-carboxylic acid

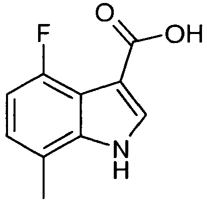

A mixture of 2,2,2-trifluoro-1-(4-fluoro-7-methyl-1H-indol-3-yl)-ethanone (100 mg, 0.41 mmol) in MeOH (2 mL) and 5 M NaOH (10 mL) is heated at 140 °C for 45 min. Water is added occasionally to compensate the lost of solvent. The mixture is cooled to rt. The mixture is partitioned between water and Et_{2O}. The two layers are separated, and the aqueous layer is cooled in ice bath. Glacial acetic acid is added until pH ∼4. The acidified aqueous layer is extracted with EtOAc (2X). The combined organic extracts are washed with water and brine, dried over MgSO₄, filtered, and concentrated *in vacuo* to 55 mg (69%) of the product as slightly pink powder. ¹H NMR (DMSO-*d₆*): δ 12.2 (bs, 1 H), 11.86 (bs, 1 H), 8.00 (d, *J* = 3.1 Hz, 1 H), 7.00-6.85 (m, 1 H), 6.85-6.70 (m, 1 H), 2.44 (s, 3H); ¹⁹ F NMR (DMSO-*d₆*): δ -117.25 (d, *J* = 8.5 Hz, 1 F); MS 194 (M+1).

### C. 4-Fluoro-7-methyl-1H-indole-3-carboxylic acid methyl ester

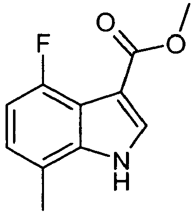

HCl gas is bubbled into a solution of 4-fluoro-7-methyl-1*H*-indole-3-carboxylic acid (50 mg, 0.26 mmol) in MeOH (20 mL) for 30 s. This mixture is stirred at rt overnight. The mixture is concentrated *in vacuo,* and the mixture is suspended in CH₂Cl₂ and heptane. This suspension is concentrated to dryness *in vacuo* to yield 50 mg (92%) of the product as a purple powder. ¹H NMR (CDCl₃): δ 8.51 (bs, 1 H), 7.93 (s, 1 H), 7.05-6.90 (m, 1 H), 6.90-6.85 (m, 1H), 3.91 (s, 3H), 2.47 (s, 3H); ¹⁹F NMR (CDCl₃): δ -116.39 (d, *J* = 11.3 Hz, 1 F); MS 208 (M+1).

### D. 4-Fluoro-7-methyl-1-(2-trifluoromethoxy-ethyl)-1H-indole-3-carboxylic acid methyl ester

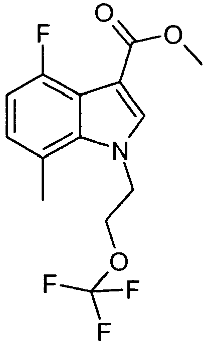

A mixture of 4-fluoro-7-methyl-1*H*-indole-3-carboxylic acid methyl ester (45 mg, 0.22 mmol) and NaH (∼20 mg, 60% oil dispersion) in THF (5 mL) is stirred at rt for 5 min. Trifluoro-methanesulfonic acid 2-trifluoromethoxy-ethyl ester (J. Org. Chem 2001, 66, 1061-1062) (100 g, 0.38 mmol) is added. This mixture is stirred at rt for 30 min. Saturated NH₄Cl is added, and the mixture is partitioned between water and EtOAc. The two layers are separated, and the organic layer is washed with brine, dried over MgSO₄, filtered, and concentrated *in vacuo.* The crude material is purified on silica gel with heptane/EtOAc (80/20 to 50/50) as eluant to give 30 mg (42%) of the product as a beige powder. ¹H NMR (CDCl₃): δ 7.75 (s,1 H), 7.00-6.90 (m, 1 H), 6.90-6.75 (m, 1 H), 4.67 (t, *J* = 5.6 Hz, 2H), 4.26 (t, J = 5.6 Hz, 2H), 3.89 (s, 3H), 2.64 (s, 3H); ¹⁹F NMR (CDCl₃): δ -60.76 (s, 3F), -115.36 (d, *J* = 5.6 Hz, 1 F); MS 320 (M+1).

### E. 4-Fluoro-7-methyl-1-(2-trifluoromethoxy-ethyl)-1H-indole-3-carboxylic acid

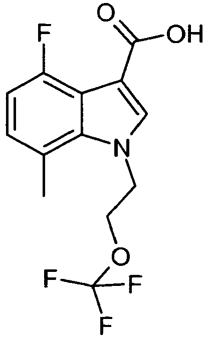

A mixture of 4-fluoro-7-methyl-1-(2-trifluoromethoxy-ethyl)-1*H*-indole-3-carboxylic acid methyl ester (25 mg, 0.078 mmol) in MeOH (5 mL) and aqueous NaOH (3.0 M, 5 mL) is stirred at 40 °C for 4 h. The mixture is concentrated *in vacuo* to remove the organic solvent. The residue is partitioned between water and EtOAc. The two layers are separated, and the aqueous layer is acidified to pH ∼2 with conc. HCl. The acidified aqueous layer is extracted with EtOAc. The organic extract is washed with water and brine, dried over MgSO₄, filtered, and concentrated *in vacuo* to give 21 mg (88%) of the product as a white powder. ¹H NMR (CDCl₃): δ 7.89 (s,1H), 7.05-6.90 (m, 1 H), 6.90-6.80 (m, 1 H), 4.70 (t, *J* = 5.6 Hz, 2H), 4.28 (t, *J* = 5.6 Hz, 2H), 2.66 (s, 3H); ¹⁹F NMR (CDCl₃): δ -60.79 (s, 3F), -115.78 (s, 1 F); MS 306 (M+1).

### D. 2,2,2-Trifluoro-N-(4-fluoro-3-{1-[4-fluoro-7-methyl-1-(2-trifluoromethoxy-ethyl)-1H-indole-3-carbonyl]-piperidin-4-yl}-benzyl)-acetamide

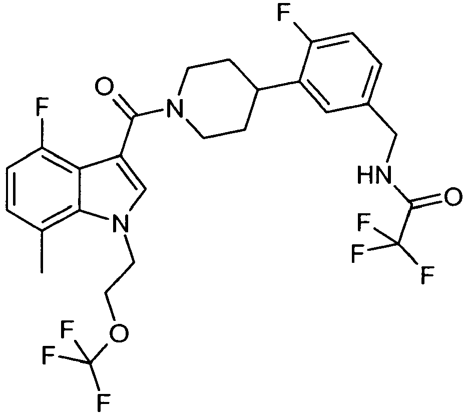

A mixture of 4-fluoro-7-methyl-1-(2-trifluoromethoxy-ethyl)-1*H*-indole-3-carboxylic acid (18 mg, 0.059 mmol), Et₃N (25 µL, 0.17 mmol), 2,2,2-trifluoro-*N*-(4-fluoro-3-piperidin-4-yl-benzyl)-acetamide hydrochloride (24 mg, 0.071 mmol), and EDCI (17 mg, 0.089 mmol) in CH₂Cl₂ (5 mL) is stirred at rt overnight. The reaction mixture is concentrated *in vacuo.* The crude material is purified on silica gel with heptane/EtOAc (50/50 to 0/100) as eluant to give 29 mg (83%) of the product as a white powder. ¹H NMR (CDCl₃): δ 7.26 (s, 1 H), 7.20-7.10 (m, 2H), 7.10-6.95 (m, 1 H), 6.95-6.85 (m, 1 H), 6.80-6.70 (m, 1 H), 6.61 (br s, 1 H), 5.20-4.70 (br m, 1 H), 4.70-4.60 (m, 2H), 4.55-4.40 (m, 2H), 4.30-4.15 (m, 2H), 4.10-3.80 (m, 1 H), 3.30-2.80 (m, 3H), 2.65 (s, 3H), 2.10-1.50 (m, 4H); ¹⁹F NMR (CDCl₃): δ -60.69 (s, 3F), -75.35 (s, 3F), -119.02 (s, 1 F), - 123.63 (s, 1 F); MS 592 (M+1, 100%).

### E. 4-(5-Aminomethvl-2-fluoro-phenyl)-piperidin-1-yl]-[4-fluoro-7-methyl-1-(2-trifluoromethoxy-ethyl)-1H-indol-3-yl]-methanonehydrochloride

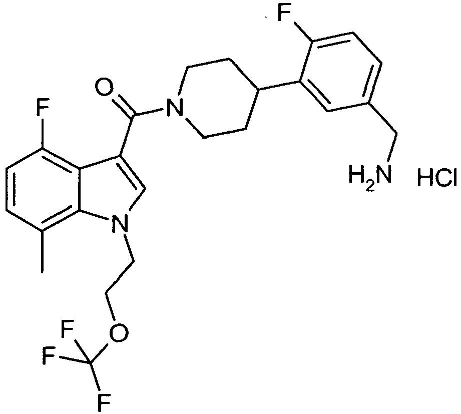

A mixture of 2,2,2-trifluoro-*N*-(4-fluoro-3-{1-[4-fluoro-7-methyl-1-(2-trifluoromethoxy-ethyl)-1*H*-indole-3-carbonyl]-piperidin-4-yl}-benzyl)-acetamide (25 mg, 0..042 mmol) in MeOH (5 mL) is added aqueous K₂CO₃ (29 mg, dissolved in 3 mL H₂O). This mixture is stirred at rt for 4 h and then 40 °c for 1 h. The reaction mixture is concentrated *in vacuo* to remove most of the methanol. The residue is partitioned between H₂O and EtOAc. The two layers are separated, and the organic layer is washed with H₂O and brine, dried over MgSO₄, filtered, and concentrated *in vacuo.* The residue is dissolved in Et₂O, and HCl in Et₂O (1.0 M, 2 mL) is added. The resulting suspension is concentrated *in vacuo,* and then dried *in vacuo* to yield 14 mg (62%) of the product as a white solid. ¹H NMR (DMSO-*d₆*): δ 8.32 (br,s 3H), 7.60 (s, 1 H), 7.55-7.45 (m, 1 H), 7.40-7.30 (m, 1 H), 7.30-7.10 (m, 1 H), 7.00-6.85 (m, 1 H), 6.85-6.70 (m, 1 H), 4.90-4.60 (m, 3H), 4.50-4.30 (m, 2H), 4.20-4.05 (m, 1 H), 4.05-3.90 (m, 2H), 3.30-2.70 (m, 3H), 2.63 (s, 3H), 1.90-1.60 (m, 4H); ¹⁹F NMR (DMSO-*d₆*): δ -59.63 (s, 3F), -120.33 (s, 1F), -125.01 (s, 1 F); MS 496 (M+1, 100%).

### BIOLOGICAL ACTIVITY

The properties of the compound of the present invention are demonstrated by: 1) its β-Tryptase Inhibitory Potency (IC₅₀ and Kᵢ values).

### IN VITRO TEST PROCEDURE

As all the actions of tryptase, as described in the background section, are dependent on its catalytic activity, then compounds that inhibit its catalytic activity will potentially inhibit the actions of tryptase. Inhibition of this catalytic activity may be measured by the in vitro enzyme assay and the cellular assay.

Tryptase inhibition activity is confirmed using either isolated human lung tryptase or recombinant human β tryptase expressed in yeast cells. Essentially equivalent results are obtained using isolated native enzyme or the expressed enzyme. The assay procedure employs a 96 well microplate (Costar 3590) using L-pyroglutamyl-L-prolyl-L-arginine-*para*-nitroanilide (S2366: Quadratech) as substrate (essentially as described by McEuen et. al. Biochem Pharm, 1996, 52, pages 331-340). Assays are performed at room temperature using 0.5 mM substrate (2 x Kₘ) and the microplate is read on a microplate reader (Beckman Biomek Plate reader) at 405 nm wavelength.

### Materials and Methods for Tryptase primary screen (Chromogenic assay)

### Assay buffer

50 mM Tris (pH 8.2), 100 mM NaCl, 0.05% Tween 20, 50 µg/mL heparin.

### Substrate

S2366 (Stock solutions of 2.5 mM).

### Enzyme

Purified recombinant beta Tryptase Stocks of 310 µg/mL.

### Protocol (Single point determination)

- Add 60 µL of diluted substrate (final concentration of 500 µM in assay buffer) to each well
- Add compound in duplicates , final concentration of 20 µM, volume 20 µL
- Add enzyme at a final concentration of 50 ng/mL in a volume of 20 µL
- Total volume for each well is 100 µL
- Agitate briefly to mix and incubate at room temp in the dark for 30 minutes
- Read absorbencies at 405 nM
Each plate has the following controls:

| | |
|---|---|
| Totals : | 60 µL of substrate, 20 µL of buffer (with 0.2% final concentration of DMSO), |
| | 20 µL of enzyme |
| Non-specific: | 60 µL of substrate, 40 µL of buffer (with 0.2% DMSO) |
| Totals: | 60 µL of substrate, 20 µL of buffer (No DMSO), 20 µL of enzyme |
| Non-specific: | 60 µL of substrate, 40 µL of buffer (No DMSO) |

### Protocol (IC₅₀ and Kᵢ determination)

The protocol is essentially the same as above except that the compound is added in duplicates at the following final concentrations: 0.01, 0.03, 0.1, 0.3, 1, 3, 10 µM (All dilutions carried out manually). For every assay, whether single point or IC₅₀ determination, a standard compound is used to derive IC₅₀ for comparison. From the IC₅₀ value, the Kᵢ can be calculated using the following formula: Kᵢ = IC₅₀/(1 + [Substrate]/Kₘ).

Experimental data below is a representation of inhibitory activity as indicated by Ki values for the the Examples which follow. The particular embodiments below are exemplary and do not limit the equivalents pertaining thereto.

| Example Number | Tryptase Ki (nM) |
|---|---|
| 1 | 116 |
| 2 | 45 |
| 3 | 35 |
| 4 | 26 |
| 5 | 322 |
| 6 | 35 |
| 7 | 365 |
| 8 | 134, 74 |
| 9 | 49, 32 |
| 10 | 146, 122 |
| 11 | 208, 186 |
| 12 | 171 |
| 13 | 129 |
| 14 | 48 |
| 15 | 45 |
| 16 | 649 |
| 17 | 25 |
| 18 | 192 |
| 19 | 142 |
| 20 | 90 |
| 21 | 487 |
| 22 | 731 |

The present invention is not to be limited in scope by the specific embodiments describe herein.

## Claims

1. A compound of formula I: 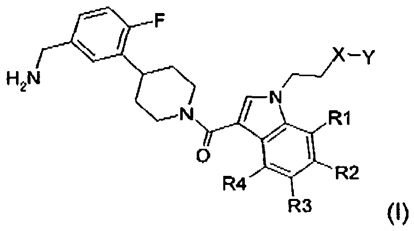 wherein each of substituents R₁, R₂, R₃ and R₄ is independenently for each instance, selected from the group consisting of hydrogen, methyl, fluoro, chloro, trifluoromethyl, methoxy, and trifluoromethoxy such that exactly two of the subtituents are hydrogen;
X is chosen from the group consisting of a bond, CH₂ and O;
Y is chosen from the group consisting of CH₃ and CF₃;
provided that when R₁ is F, R₂ and R₃ are H, R₄ is trifluoromethoxy, and X is O, Y cannot be CH₃;
or a pharmaceutically acceptable salt, or solvate thereof.

2. The compound according to claim 1, wherein:
X is O and Y is CH₃;
X is CH₂ and Y is CH₃;
X is a bond and Y is CH₃; or
X is O and Y is CF₃.

3. The compound according to claim 1, wherein:
R₁ is CH₃, R₂ is H, R₃ is Cl and R₄ is H;
R₁ is CH₃, R₂ is F, R₃ is H and R₄ is H;
R₁ is CH₃, R₂ is H, R₃ is F and R₄ is H;
R₁ is CH₃, R₂ is H, R₃ is H and R₄ is F;
R₁ is OCF₃, R₂ is H, R₃ is H and R₄ is F;
R₁ is CH₃, R₂ is H, R₃ is H and R₄ is Cl;
R₁ is F, R₂ is H, R₃ is H and R₄ is OCF₃;
R₁ and R₄ are Cl, and R₂ and R₃ are H;
R₁ is Cl, R₂ is H, R₃ is H and R₄ is F;
R₁ is Cl, R₂ is H, R₃ is H and R₄ is OCF₃;
R₁ is OCF₃, R₂ is H, R₃ is H and R₄ is Cl;
R₁ is F, R₂ is H, and R₃ is H and R₄ is CF₃;
R₁ is OCH₃, R₂ is H, R₃ is H and R₄ is CF₃;
R₁ is F, R₂ is H, R₃ is H and R₄ is CH₃;
R₁ and R₃ are H, and R₂ and R₄ are Cl;
R₁ and R₄ are F, and R₂ and R₃ are H
R₁ and R₃ are H, and R₂ and R₄ are F;
R₁ and R₂ are H, and R₃ and R₄ are F; or
R₁ and R₄ are H, and R₂ and R₃ are OCH₃.

4. The compound according to claim 2, wherein:
R₁ is CH₃, R₂ is H, R₃ is Cl and R₄ is H;
R₁ is CH₃, R₂ is F, R₃ is H and R₄ is H;
R₁ is CH₃, R₂ is H, R₃ is F and R₄ is H;
R₁ is CH₃, R₂ is H, R₃ is H and R₄ is F;
R₁ is OCF₃, R₂ is H, R₃ is H and R₄ is F;
R₁ is CH₃, R₂ is H, R₃ is H and R₄ is Cl;
R₁ is F, R₂ is H, R₃ is H and R₄ is OCF₃,
R₁ and R₄ are Cl, and R₂ and R₃ are H;
R₁ is Cl, R₂ is H, R₃ is H and R₄ is F;
R₁ is Cl, R₂ is H, R₃ is H and R₄ is OCF₃;
R₁ is OCF₃, R₂ is H, R₃ is H and R₄ is Cl;
R₁ is F, R₂ is H, and R₃ is H and R₄ is CF₃;
R₁ is OCH₃, R₂ is H, R₃ is H and R₄ is CF₃;
R₁ is F, R₂ is H, R₃ is H and R₄ is CH₃;
R₁ and R₃ are H, and R₂ and R₄ are Cl;
R₁ and R₄ are F, and R₂ and R₃ are H
R₁ and R₃ are H, and R₂ and R₄ are F;
R₁ and R₂ are H, and R₃ and R₄ are F; or
R₁ and R₄ are H, and R₂ and R₃ are OCH₃.

5. The compound according to claim 1 which is selected from the group consisting of:
[4-(5-aminomethyl-2-fluoro-phenyl)-piperidin-1-yl]-[5-chloro-1-(2-methoxy-ethyl)-7-methyl-1H-indol-3-yl]-methanone;
[4-(5-aminomethyl-2-fluoro-phenyl)-piperidin-1-yl]-[6-fluoro-1-(2-methoxy-ethyl)-7-methyl-1 H-indol-3-yl]-methanone;
[4-(5-aminomethyl-2-fluoro-phenyl)-piperidin-1-yl]-[5-fluoro-1-(2-methoxy-ethyl)-7-methyl-1 H-indol-3-yl]-methanone;
[4-(5-aminomethyl-4-fluoro-phenyl)-piperidin-I -yl]-[5-fluoro-1 -(2-methoxy-ethyl)-7-methyl-1H-indol-3-yl]-methanone;
[4-(5-aminomethyl-2-fluoro-phenyl)-piperidin-1-yl]-[4-fluoro-1-(2-methoxy-ethyl)-7-trifluoromethoxy-1 H-indol-3-yl]-methanone;
[4-(5-aminomethyi-2-fluoro-phenyl)-piperidin-1-yl]-[4-chloro-1-(2-methoxy-ethyl)-7-methyl-1H-indol-3-yl]-methanone;
[4-(5-aminomethyl-2-fluoro-phenyl)-piperidin-1-yl]-(1-butyl-7-fluoro-4-trifluoromethoxy-1 H-indol-3-yl)-methanone; hydrochloride;
[4-(5-aminomethyl-2-fluoro-phenyl)-piperidin-1-yl]-[4,7-dichloro-1-(2-methoxy-ethyl)-1 H-indol-3-yl]-methanone hydrochloride;
[4-(5-aminomethyl-2-fluoro-phenyl)-piperidin-1-yl]-[7-chloro-4-fluoro-1-(2-methoxyethyl)-1H-indol-3-yl]-methanone hydrochloride;
[4-(5-aminomethyl-2-fluoro-phenyl)-piperidin-1-yl]-[7-chloro-1-(2-methoxy-ethyl)-4-trifluoromethoxy-1H-indol-3-yl]-methanone hydrochloride;
[4-(5-aminomethyl-2-fluoro-phenyl)-piperidin-1-yl]-(1-propyl-7-fluoro-4-trifluoromethoxy-1H-indol-3-yl)-methanone; hydrochloride;
[4-(5-aminomethyl-2-fluoro-phenyl)-piperidin-1-yl]-[4-chloro-1-(2-methoxy-ethyl)-7-trifluoromethoxy-1 H-indol-3-yl]-methanone hydrochloride;
[4-(5-aminomethyl-2-fluoro-phenyl)-piperidin-1-yl]-[7-fluoro-1-(2-methoxy-ethyl)-4-trifluoromethyl-1H-indol-3-yl]-methanone hydrochloride;
[4-(5-aminomethyl-2-fluoro-phenyl)-piperidin-1-yl]-[7-methoxy-1-(2-methoxy-ethyl)-4-trifluoromethyl-1H-indol-3-yl]-methanone hydrochloride;
[4-(5-aminomethyl-2-fluoro-phenyl)-piperidin-1-yl]-[7-fluoro-1-(2-methoxy-ethyl)-4-methyl-1 H-indol-3-yl]-methanone hydrochloride;
[4-(5-aminomethyl-2-fluoro-phenyl)-piperidin-1-yl]-[4,6-dichloro-1-(2-methoxy-ethyl)-1H-indol-3-yl]-methanone hydrochloride;
[4-(5-aminomethyl-2-fluoro-phenyl)-piperidin-1-yl]-[4,7-difluoro-1-(2-methoxy-ethyl)-1 H-indol-3-yl]-methanone;
[4-(5-aminomethyl-2-fluoro-phenyl)-piperidin-1-yl]-[4,6-difluoro-1-(2-methoxy-ethyl)-1H-indol-3-yl]-methanone hydrochloride;
[4-(5-aminomethyl-2-fluoro-phenyl)-piperidin-1-yl]-[4,5-difluoro-1-(2-methoxy-ethyl)-1H-indol-3-yl]-methanone hydrochloride;
[4-(5-aminomethyl-2-fluoro-phenyl)-piperidin-1-yl]-[5,6-dimethoxy-1-(2-methoxyethyl)-1H-indol-3-yl]-methanone hydrochloride;
[4-(5-aminomethyl-2-fluoro-phenyl)-piperidin-1-yl]-[7-fluoro-4-trifluoromethoxy-1-(2-trifluoromethoxy-ethyl)-1H-indol-3-yl]-methanone; and
[4-(5-aminomethyl-2-fluoro-phenyl)-piperidin-1-yl]-[4-fluoro-7-methyl-1-(2-trifluoromethoxy-ethyl)-1H-indol-3-yl]-methanone.

6. A compound according to Claim 1 for use in treatment of a physiological condition in need of amelioration by inhibition of tryptase, wherein the physiological condition is selected from the group consisting of inflammatory disease, a disease of joint cartilage destruction, ocular conjunctivitis, vernal conjunctivitis, inflammatory bowel disease, asthma, allergic rhinitis, interstitial lung disease, fibrosis, chronic obstructive pulmonary disease, sceleroderma, pulmonary fibrosis, liver cirrhosis, myocardial fibrosis, neurofibroma, hypertrophic scar, dermatological condition, condition related to atherosclerotic plaque rupture, periodontal disease, diabetic retinopathy, tumour growth, anaphylaxis, multiple sclerosis, peptic ulcer, and syncytial viral infection.

7. A compound according to Claim 6 for use in treatment of a physiological condition in need of amelioration by inhibition of tryptase, wherein the physiological condition is inflammatory disease.

8. A compound according to Claim 7 for use in treatment of a physiological condition in need of amelioration by inhibition of tryptase, wherein the inflammatory disease is joint inflammation, inflammatory bowel disease, arthritis, rheumatoid arthritis, rheumatoid spondylitis, gouty arthritis, traumatic arthritis, rubella arthritis, psoriatic arthritis, asthma or osteoarthritis.

9. A compound according to Claim 7 for use in treatment of a physiological condition in need of amelioration by inhibition of tryptase, wherein the inflammatory disease is inflammatory bowel disease.

10. A compound according to Claim 6 for use in treatment of a physiological condition in need of amelioration by inhibition of tryptase, wherein the physiological condition is COPD.

11. A compound according to Claim 6 for use in treatment of a physiological condition in need of amelioration by inhibition of tryptase, wherein the physiological condition is asthma.

12. A compound according to Claim 6 for use in treatment of a physiological condition in need of amelioration by inhibition of tryptase, wherein the physiological condition is a dermatological condition.

13. A compound according to Claim 12 for use in treatment of a physiological condition in need of amelioration by inhibition of tryptase, wherein the dermatological condition is selected from atopic dermatitis, psoriasis and eczema.

14. A pharmaceutical composition comprising a therapeutically effective amount of a compound of Claim 1 and a pharmaceutically acceptable carrier thereof.

## Patentansprüche

1. Verbindungen der Formel I: 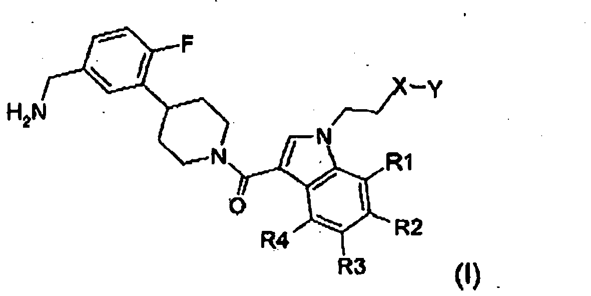 wobei die Substituenten R₁, R₂, R₃ und R₄ jeweils in jedem Fall unabhängig voneinander aus der aus Wasserstoff, Methyl, Fluor, Chlor, Triflüormethyl, Methoxy und Trifluormethoxy bestehenden Gruppe ausgewählt sind, so dass genau zwei der Substituenten für Wasserstoff stehen;
X aus der aus einer Bindung, CH₂ und O bestehenden Gruppe ausgewählt ist;
Y aus der aus CH₃ und CF₃ bestehenden Gruppe ausgewählt ist;
mit der Maßgabe, dass, wenn R₁ für F steht, R₂ und R₃ für H stehen, R₄ für Trifluormethoxy steht und X für O steht, Y nicht für CH₃ stehen kann;
und deren pharmazeutisch unbedenkliche Salze und Solvate.

2. Verbindungen nach Anspruch 1, wobei:
X für O steht und Y für CH₃ steht;
X für CH₂ steht und Y für CH₃ steht;
X für eine Bindung steht und Y für CH₃ steht oder
X für O steht und Y für CF₃ steht.

3. Verbindungen nach Anspruch 1, wobei:
R₁ für CH₃ steht, R₂ für H steht, R₃ für Cl steht und R₄ für H steht;
R₁ für CH₃ steht, R₂ für F steht, R₃ für H steht und R₄ für H steht;
R₁ für CH₃ steht, R₂ für H steht, R₃ für F steht und R₄ für H steht;
R₁ für CH₃ steht, R₂ für H steht, R₃ für H steht und R₄ für F steht;
R₁ für OCF₃ steht, R₂ für H steht, R₃ für H steht und R₄ für F steht;
R₁ für CH₃ steht, R₂ für H steht, R₃ für H steht und R₄ für Cl steht;
R₁ für F steht, R₂ für H steht, R₃ für H steht und R₄ für OCF₃ steht;
R₁ und R₄ für Cl stehen und R₂ und R₃ für H stehen;
R₁ für Cl steht, R₂ für H steht, R₃ für H steht und R₄ für F steht;
R₁ für Cl steht, R₂ für H steht, R₃ für H steht und R₄ für OCF₃ steht;
R₁ für OCF₃ steht, R₂ für H steht, R₃ für H steht und R₄ für Cl steht;
R₁ für F steht, R₂ für H steht, R₃ für H steht und R₄ für CF₃ steht;
R₁ für OCH₃ steht, R₂ für H steht, R₃ für H steht und R₄ für CF₃ steht;
R₁ für F steht, R₂ für H steht, R₃ für H steht und R₄ für CH₃ steht;
R₁ und R₃ für H stehen und R₂ und R₄ für Cl stehen;
R₁ und R₄ für F stehen und R₂ und R₃ für H stehen;
R₁ und R₃ für H stehen und R₂ und R₄ für F stehen;
R₁ und R₂ für H stehen und R₃ und R₄ für F stehen oder
R₁ und R₄ für H stehen und R₂ und R₃ für OCH₃ stehen.

4. Verbindungen nach Anspruch 2, wobei:
R₁ für CH₃ steht, R₂ für H steht, R₃ für Cl steht und R₄ für H steht;
R₁ für CH₃ steht, R₂ für F steht, R₃ für H steht und R₄ für H steht;
R₁ für CH₃ steht, R₂ für H steht, R₃ für. F steht und R₄ für H steht;
R₁ für CH₃ steht, R₂ für H steht, R₃ für H steht und R₄ für F steht;
R₁ für OCF₃ steht, R₂ für H steht, R₃ für H steht und R₄ für F steht;
R₁ für CH₃ steht, R₂ für H steht, R₃ für H steht und R₄ für Cl steht;
R₁ für F steht, R₂ für H steht, R₃ für H steht und R₄ für OCF₃ steht;
R₁ und R₄ für Cl stehen und R₂ und R₃ für H stehen;
R₁ für Cl steht, R₂ für H steht, R₃ für H steht und R₄ für F steht;
R₁ für Cl steht, R₂ für H steht, R₃ für H steht und R₄ für OCF₃ steht;
R₁ für OCF₃ steht, R₂ für H steht, R₃ für H steht und R₄.
für Cl steht;
R₁ für F steht, R₂ für H steht, R₃ für H steht und R₄ für CF₃ steht;
R₁ für OCH₃ steht, R₂ für H steht, R₃ für H steht und R₄ für CF₃ steht;
R₁ für F steht, R₂ für H steht, R₃ für H steht und R₄ für CH₃ steht;
R₁ und R₃ für H stehen und R₂ und R₄ für Cl stehen;
R₁ und R₄ für F stehen und R₂ und R₃ für H stehen;
R₁ und R₃ für H stehen und R₂ und R₄ für F stehen;
R₁ und R₂ für H stehen und R₃ und R₄ für F stehen oder
R₁ und R₄ für H stehen und R₂ und R₃ für OCH₃ stehen.

5. Verbindungen nach Anspruch 1, ausgewählt aus der Gruppe bestehend aus:
[4-(5-Aminomethyl-2-fluorphenyl)piperidin-1-yl]-[5-chlor-1-(2-methoxyethyl)-7-methyl-1H-indol-3-yl]methanon;
[4-(5-Aminomethyl-2-fluorphenyl)piperidin-1-yl]-[6-fluor-1-(2-methoxyethyl)-7-methyl-1H-indol-3-yl]methanon;
[4-(5-Aminomethyl-2-fluorphenyl)piperidin-1-yl]-[5-fluor-1-(2-methoxyethyl)-7-methyl-1H-indol-3-yl]methanon;
[4-(5-Aminomethyl-4-fluorphenyl)piperidin-1-yl]-[5-fluor-1-(2-methoxyethyl)-7-methyl-1H-indol-3-yl]methanon;
[4-(5-Aminomethyl-2-fluorphenyl)piperidin-1-yl]-[4-fluor-1-(2-methoxyethyl)-7-trifluormethoxy-1H-indol-3-yl]methanon;
[4-(5-Aminomethyl-2-fluorphenyl)piperidin-1-yl]-[4-chlor-1-(2-methoxyethyl)-7-methyl-1H-indol-3-yl]methanon;
[4-(5-Aminomethyl-2-fluorphenyl)piperidin-1-yl]-(1-butyl-7-fluor-4-trifluormethoxy-1H-indol-3-yl)methanonhydrochlorid;
[4-(5-Aminomethyl-2-fluorphenyl)piperidin-1-yl]-[4,7-dichlor-1-(2-methoxyethyl)-1H-indol-3-yl]methanonhydrochlorid;
[4-(5-Aminomethyl-2-fluorphenyl)piperidin-1-yl]-[7-chlor-4-fluor-1-(2-methoxyethyl)-1H-indol-3-yl]methanonhydrochlorid;
[4-(5-Aminomethyl-2-fluorphenyl)-piperidin-1-yl]-[7-chlor-1-(2-methoxyethyl)-4-trifluormethoxy-1H-indol-3-yl]methanonhydrochlorid;
[4-(5-Aminomethyl-2-fluorphenyl)piperidin-1-yl]-(1-propyl-7-fluor-4-trifluormethoxy-1H-indol-3-yl)methanonhydrochlorid;
[4-(5-Aminomethyl-2-fluorphenyl)piperidin-1-yl]-[4-chlor-1-(2-methoxyethyl)-7-trifluormethoxy-1H-indol-3-yl]methanonhydrochlorid;
[4-(5-Aminomethyl-2-fluorphenyl)piperidin-1-yl]-[7-fluor-1-(2-methoxyethyl)-4-trifluormethyl-1H-indol-3-yl]methanonhydrochlorid;
[4-(5-Aminomethyl-2-fluorphenyl)piperidin-1-yl]-[7-methoxy-1-(2-methoxyethyl)-4-trifluormethyl-1H-indol-3-yl]methanonhydrochlorid;
[4-(5-Aminomethyl-2-fluorphenyl)piperidin-1-yl]-[7-fluor-1-(2-methoxyethyl)-4-methyl-1H-indol-3-yl]methanonhydrochlorid;
[4-(5-Aminomethyl-2-fluorphenyl)piperidin-1-yl]-[4,6-dichlor-1-(2-methoxyethyl)-1H-indol-3-yl]methanonhydrochlorid;
[4-(5-Aminomethyl-2-fluorphenyl)piperidin-1-yl]-[4,7-difluor-1-(2-methoxyethyl)-1H-indol-3-yl]methanon;
[4-(5-Aminomethyl-2-fluorphenyl)piperidin-1-yl]-[4,6-difluor-1-(2-methoxyethyl)-1H-indol-3-yl]methanonhydrochlorid;
[4-(5-Aminomethyl-2-fluorphenyl)piperidin-1-yl]-[4,5-difluor-1-(2-methoxyethyl)-1H-indol-3-yl]methanonhydrochlorid;
[4-(5-Aminomethyl-2-fluorphenyl)piperidin-1-yl]-[5,6-dimethoxy-1-(2-methoxyethyl)-1H-indol-3-yl]methanonhydrochlorid;
[4-(5-Aminomethyl-2-fluorphenyl)piperidin-1-yl]-[7-fluor-4-trifluormethoxy-1-(2-trifluormethoxyethyl)-1H-indol-3-yl]methanon und
[4-(5-Aminomethyl-2-fluorphenyl)piperidin-1-yl]-[4-fluor-7-methyl-1-(2-trifluormethoxyethyl)-1H-indol-3-yl]methanon.

6. Verbindungen nach Anspruch 1 zur Verwendung bei der Behandlung eines der Linderung bedürftigen physiologischen Leidens durch Inhibieren von Tryptase, wobei das physiologische Leiden aus der aus einer entzündlichen Krankheit, einer mit Gelenkknorpelabbau einhergehenden Krankheit, okularer Konjunktivitis, vernaler Konjunktivitis, entzündlicher Darmkrankheit, Asthma, allergischer Rhinitis, interstitieller Lungenkrankheit, Fibrose, chronischer obstruktiver Atemwegserkrankung, Skleroderm, Lungenfibrose, Leberzirrhose, Herzmuskelfibrose, Neurofibrom, hypertrophischen Narben, dermatologischen Leiden, mit dem Abreißen einer atherosklerotischen Plaque in Zusammenhang stehenden Leiden, Periodontalkrankheit, diabetischer Retinopathie, Tumorwachstum, Anaphylaxe, Multipler Sklerose, Magengeschwür und synzytischer Virusinfektion bestehenden Gruppe ausgewählt ist.

7. Verbindungen nach Anspruch 6 zur Verwendung bei der Behandlung eines der Linderung bedürftigen physiologischen Leidens durch Inhibieren von Tryptase, wobei es sich bei dem physiologischen Leiden um eine entzündliche Krankheit handelt.

8. Verbindungen nach Anspruch 7 zur Verwendung bei der Behandlung eines der Linderung bedürftigen physiologischen Leidens durch Inhibieren von Tryptase, wobei es sich bei der entzündlichen Krankheit um Gelenkentzündung, entzündlicher Darmkrankheit, Arthritis, rheumatoide Arthritis, rheumatoide Spondylitis, gichtartige Arthritis, traumatische Arthritis, Rötelarthritis, Psoriasisarthritis, Asthma oder Osteoarthritis handelt.

9. Verbindungen nach Anspruch 7 zur Verwendung bei der Behandlung eines der Linderung bedürftigen physiologischen Leidens durch Inhibieren von Tryptase, wobei es sich bei der entzündlichen Krankheit um entzündliche Darmkrankheit handelt.

10. Verbindungen nach Anspruch 6 zur Verwendung bei der Behandlung eines der Linderung bedürftigen physiologischen Leidens durch Inhibieren von Tryptase, wobei es sich bei dem physiologischen Leiden um COPD handelt.

11. Verbindungen nach Anspruch 6 zur Verwendung bei der Behandlung eines der Linderung bedürftigen physiologischen Leidens durch Inhibieren von Tryptase, wobei es sich bei dem physiologischen Leiden um Asthma handelt.

12. Verbindungen nach Anspruch 6 zur Verwendung bei der Behandlung eines der Linderung bedürftigen physiologischen Leidens durch Inhibieren von Tryptase, wobei es sich bei dem physiologischen Leiden um ein dermatologisches Leiden handelt.

13. Verbindungen nach Anspruch 12 zur Verwendung bei der Behandlung eines der Linderung bedürftigen physiologischen Leidens durch Inhibieren von Tryptase, wobei das dermatologische Leiden aus atopischer Dermatitis, Psoriasis und Ekzem ausgewählt ist.

14. Pharmazeutische Zusammensetzung, umfassend eine therapeutisch wirksame Menge einer Verbindung nach Anspruch 1 und einen pharmazeutisch unbedenklichen Träger dafür.

## Revendications

1. Composé de formule I : 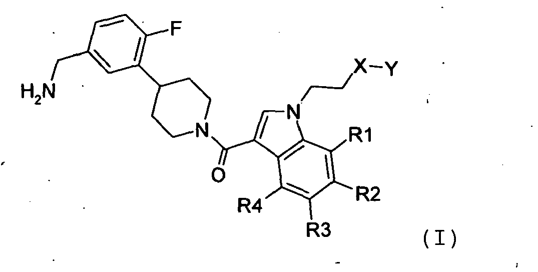 dans laquelle chacun des substituants R₁, R₂, R₃ et R₄ est indépendamment dans chaque cas, choisi dans le groupe constitué d'un hydrogène, un méthyle, un fluoro, un chloro, un trifluorométhyle, un méthoxy, et un trifluorométhoxy de sorte qu'exactement deux des substituants soient un hydrogène ;
X est choisi dans le groupe constitué d'une liaison, CH₂ et O ;
Y est choisi dans le groupe constitué de CH₃ et CF₃ ;
à condition que lorsque R₁ est F, R₂ et R₃ sont H, R₄, est un trifluorométhoxy, et X est O, Y ne puisse pas être CH₃ ;
ou un sel pharmaceutiquement acceptable, ou un solvate de celui-ci.

2. Composé selon la revendication 1, dans lequel :
X est O et Y est CH₃ ;
X est CH₂ et Y est CH₃ ;
X est une liaison et Y est CH₃ ; ou
X est O et Y est CF₃.

3. Composé selon la revendication 1, dans lequel :
R₁ est CH₃, R₂ est H, R₃ est Cl et R₄ est H ;
R₁ est CH₃, R₂ est F, R₃ est H et R₄ est H ;
R₁ est CH₃, R₂ est H, R₃ est F et R₄ est H ;
R₁ est CH₃, R₂ est H, R₃ est H et R₄ est F ;
R₁ est OCF₃, R₂ est H, R₃ est H et R₄ est F ;
R₁ est CH₃, R₂ est H, R₃ est H et R₄ est Cl ;
R₁ est F, R₂ est H, R₃ est H et R₄ est OCF₃ ;
R₁ et R₄ sont Cl, et R₂ et R₃ sont H ;
R₁ est Cl, R₂ est H, R₃ est H et R₄ est F ;
R₁ est Cl, R₂ est H, R₃ est H et R₄ est OCF₃ ;
R₁ est OCF₃, R₂ est H, R₃ est H et R₄ est Cl ;
R₁ est F, R₂ est H, et R₃ est H et R₄ est CF₃ ;
R₁ est OCH₃, R₂ est H, R₃ est H et R₄ est CF₃ ;
R₁ est F, R₂ est H, R₃ est H et R₄ est CH₃ ;
R₁ et R₃ sont H, et R₂ et R₄ sont Cl ;
R₁ et R₄ sont F, et R₂ et R₃ sont H R₁ et R₃ sont H, et R₂ et R₄ sont F ;
R₁ et R₂ sont H, et R₃ et R₄ sont F ; ou
R₁ et R₄ sont H, et R₂ et R₃ sont OCH₃.

4. Composé selon la revendication 2, dans lequel :
R₁ est CH₃, R₂ est H, R₃ est Cl et R₄ est H ;
R₁ est CH₃, R₂ est F, R₃ est H et R₄ est H ;
R₁ est CH₃, R₂ est H, R₃ est F et R₄ est H ;
R₁ est CH₃, R₂ est H, R₃ est H et R₄ est F ;
R₁ est OCF₃, R₂ est H, R₃ est H et R₄ est F ;
R₁ est CH₃, R₂ est H, R₃ est H et R₄ est Cl ;
R₁ est F, R₂ est H, R₃ est H et R₄ est OCF₃ ;
R₁ et R₄ sont Cl, et R₂ et R₃ sont H ;
R₁ est Cl, R₂ est H, R₃ est H et R₄ est F ;
R₁ est Cl, R₂ est H, R₃ est H et R₄ est OCF₃ ;
R₁ est OCF₃, R₂ est H, R₃ est H et R₄ est Cl ;
R₁ est F, R₂ est H, et R₃ est H et R₄ est CF₃ ;
R₁ est OCH₃, R₂ est H, R₃ est, H et R₄ est CF₃ ;
R₁ est F, R₂ est H, R₃ est H et R₄ est CH₃ ;
R₁ et R₃ sont H, et R₂ et R₄ sont Cl ;
R₁ et R₄ sont F, et R₂ et R₃ sont H
R₁ et R₃ sont H, et R₂ et R₄ sont F ;
R₁ et R₂ sont H, et R₃ et R₄ sont F ; ou
R₁ et R₄ sont H, et R₂ et R₃ sont OCH₃.

5. Composé selon la revendication 1 qui est choisi dans le groupe constitué de :
[4-(5-aminométhyl-2-fluoro-phényl)-pipéridin-1-yl]-[5-chloro-1-(2-méthoxy-éthyl)-7-méthyl-1H-indol-3-yl]-méthanone ;
[4-(5-aminométhyl-2-fluoro-phényl)-pipéridin-1-yl]-[6-fluoro-1-(2-méthoxy-éthyl)-7-méthyl-1H-indol-3-yl]-méthanone ;
[4-5-aminométhyl-2-fluoro-phényl)-pipéridin-1-yl]-[5-fluoro-1-(2-méthoxy-éthyl)-7-méthyl-1H-indol-3-yl]-méthanone ;
[4-(5-aminométhyl-4-fluoro-phényl)-pipéridin-1-yl]-[5-fluoro-1-(2-méthoxy-éthyl)-7-méthyl-1H-indol-3-yl]-méthanone ;
[4-(5-aminométhyl-2-fluoro-phényl)-pipéridin-1-yl]-[4-fluoro-1-(2-méthoxy-éthyl)-7-trifluorométhoxy-1H-indol-3-yl]-méthanone ;
[4-(5-aminométhyl-2-fluoro-phényl)-pipéridin-1-yl]-[4-chloro-1-(2-méthoxy-éthyl)-7-méthyl-1H-indol-3-yl]-méthanone ;
chlorhydrate de [4-(5-aminométhyl-2-fluoro-phényl)-pipéridin-1-yl]-(1-butyl-7-fluoro-4-trifluorométhoxy-1H-indol-3-yl)-méthanone ;
chlorhydrate dé [4-(5-aminométhyl-2-fluoro-phényl)-pipéridin-1-yl]-[4,7-dichloro-1-(2-méthoxy-éthyl)-1H-indol-3-yl]-méthanone ;
chlorhydrate de [4-(5-aminométhyl-2-fluoro-phényl)-pipéridin-1-yl]-[7-chloro-4-fluoro-1-(2-méthoxy-éthyl)-1H-indol-3-yl]-méthanone ;
chlorhydrate dé [4-(5-aminométhyl-2-fluoro-phényl)-pipéridin-1-yl]-[7-chloro-1-(2-méthoxy-éthyl)-4-trifluorométhoxy-1H-indol-3-yl]-méthanone ;
chlorhydrate de [4-(5-aminométhyl-2-fluoro-phényl)-pipéridin-1-yl]-(1-propyl-7-fluoro-4-trifluorométhoxy-1H-indol-3-yl)-méthanone ;
chlorhydrate de [4-(5-aminométhyl-2-fluoro-phényl)-pipéridin-1-yl]-[4-chloro-1-(2-méthoxy-éthyl)-7-trifluorométhoxy-1H-indol-3-yl]-méthanone ;
chlorhydrate de [4-(5-aminométhyl-2-fluoro-phényl)-pipéridin-1-yl]-[7-fluoro-1-(2-méthoxy-éthyl)-4-trifluorométhyl-1H-indol-3-yl]-méthanone ;
chlorhydrate de [4-(5-aminométhyl-2-fluoro-phényl)-pipéridin-1-yl]-[7-méthoxy-1-(2-méthoxy-éthyl)-4-trifluorométhyl-1H-indol-3-yl]-méthanone ;
chlorhydrate de [4-(5-aminométhyl-2-fluoro-phényl)-pipéridin-1-yl]-[7-fluoro-1-(2-méthoxy-éthyl)-4-méthyl-1H-indol-3-yl]-méthanone ;
chlorhydrate de [4-(5-aminométhyl-2-fluoro-phényl)-pipéridin-1-yl]-[4,6-dichloro-1-(2-méthoxy-éthyl)-1H-indol-3-yl]-méthanone ;
[4-(5-aminométhyl-2-fluoro-phényl)-pipéridin-1-yl]-[4,7-difluoro-1-(2-méthoxy-éthyl)-1H-indol-3-yl]-méthanone ;
chlorhydrate de [4-(5-aminométhyl-2-fluoro-phényl)-pipéridin-1-yl]-[4,6-difluoro-1-(2-méthoxy-éthyl)-1H-indol-3-yl]-méthanone ;
chlorhydrate de [4-(5-aminométhyl-2-fluoro-phényl)-pipéridin-1-yl]-[4,5-difluoro-1-(2-méthoxy-éthyl)-1H-indol-3-yl]-méthanone ;
chlorhydrate de [4-(5-aminométhyl-2-fluoro-phényl)-pipéridin-1-yl]-[5,'6-diméthoxy-1-(2-méthoxy-éthyl)-1H-indol-3-yl]-méthanone ;
[4-(5-aminométhyl-2-fluoro-phényl)-pipéridin-1-yl]-[7-fluoro-4-trifluorométhoxy-1-(2-trifluorométhoxy-éthyl)-1H-indol-3-yl]-méthanone ; et
[4-(5-aminométhyl-2-fluoro-phényl)-pipéridin-1-yl]-[4-fluoro-7-méthyl-1-(2-trifluorométhoxy-éthyl)-1H-indol-3-yl]-méthanone.

6. Composé selon la revendication 1 destiné a être utilisé dans le traitement d'un état physiologique nécessitant une amélioration par inhibition de tryptase, dans lequel l'état physiologique est choisi dans le groupe constitué d'une maladie inflammatoire, une maladie de destruction du cartilage articulaire, la conjonctivite oculaire, la conjonctivite printanière, l'affection abdominale inflammatoire, l'asthme, la rhinite allergique, une maladie pulmonaire interstitielle, la fibrose, la bronchopneumopathie chronique obstructive (COPD), la sclérodermie, la fibrose pulmonaire, la cirrhose du foie, la fibrose myocardique, un neurofibrome, une cicatrice hypertropbique, une affection dermatologique, une affection liée à une rupture de plaque athéroscléreuse, une maladie parodontale, la rétinopathie diabétique, la croissance de tumeur, l'anaphylaxie, la sclérose en plaques, un ulcère gastro-duodénal, et une infection virale syncytiale.

7. Composé selon la revendication 6, destiné a être utilisé dans le traitement d'un état physiologique nécessitant une amélioration par inhibition de tryptase, dans lequel l'état physiologique est une maladie inflammatoire.

8. Composé selon la revendication 7 destiné a être utilisé dans le traitement d'un état physiologique nécessitant une amélioration par inhibition de tryptase, dans lequel la maladie inflammatoire est une inflammation articulaire, l'affection abdominale inflammatoire, l'arthrite, la polyarthrite rhumatoïde, la spondylarthrite ankylosante, l'arthrite goutteuse, l'arthrite traumatique, l'arthrite rubéoleuse, le rhumatisme psoriasique, l'asthme ou l'arthrose.

9. Composé selon la revendication 7 destiné a être utilisé dans le traitement d'un état physiologique nécessitant une amélioration par inhibition de tryptase, dans lequel la maladie inflammatoire est l'affection abdominale inflammatoire.

10. Composé selon la revendication 6 destiné a être utilisé dans le traitement d'un état physiologique nécessitant une amélioration par inhibition de tryptase, dans lequel l'état physiologique est COPD.

11. Composé selon la revendication 6 destiné a être utilisé dans le traitement d'un état physiologique nécessitant une amélioration par inhibition de tryptase, dans lequel l'état physiologique est l'asthme.

12. Composé selon la revendication 6 destiné a être utilisé dans le traitement d'un état physiologique nécessitant une amélioration par inhibition de tryptase, dans lequel l'état physiologique est une affection dermatologique.

13. Composé selon la revendication 12 destiné a être utilisé dans le traitement d'un état physiologique nécessitant une amélioration par inhibition de tryptase, dans lequel l'affection dermatologique est choisie parmi la dermite atopique, le psoriasis et l'eczéma.

14. Composition pharmaceutique comprenant une quantité thérapeutiquement efficace d'un composé de la revendication 1 et un véhicule pharmaceutiquement acceptable de celui-ci.
